# EUROPEAN PATENT SPECIFICATION

(11) **EP 2 885 306 B1**
(45) Date of publication and mention of the grant of the patent: **01.06.2016**
(21) Application number: 13745668.7
(22) Date of filing: 06.08.2013
(51) Int. Cl.: C07D 513/04, A61K 31/429, A61P 25/28

(54) **IMIDAZO[2,1]THIAZOL-3-ONE DERIVATIVES USEFUL AS DIAGNOSTIC AGENTS FOR ALZHEIMER'S DISEASE**
IMIDAZO[2,1]THIAZOL-3-ON-DERIVATE ALS DIAGNOSTISCHE MITTEL FÜR MORBUS ALZHEIMER
DÉRIVÉS IMIDAZO[2,1]THIAZOL-3-ONE UTILES COMME AGENTS DE DIAGNOSTIC DE LA MALADIE D'ALZHEIMER

(30) Priority: 14.08.2012 EP 12180367
(43) Date of publication of application: 24.06.2015
(73) Proprietor: F.Hoffmann-La Roche AG, 4070 Basel (CH)
(72) Inventor: GOBBI, Luca, 4132 Muttenz (CH); KNUST, Henner, 79618 Rheinfelden (DE); KOBLET, Andreas, 4103 Bottmingen (CH)
(74) Representative: Poppe, Regina
(86) International application number: PCT/EP2013/066447
(87) International publication number: WO 2014/026881

(56) References cited:
- WO-A1-2010/034982

## Description

The invention relates to imidazo[2,1-b]thiazol-3-one derivatives of formula 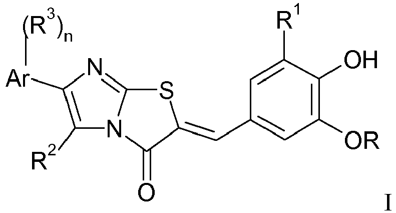 wherein
- Ar: is phenyl, pyridinyl, 2,3-dihydro-benzo[1,4]dioxinyl, 1,3-dihydro-indol-2-one, pyrazinyl, isoxazol-3-yl, imidazolyl, thiophenyl or pyrimidinyl;
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
- R³: is hydrogen, halogen, lower alkyl, lower alkyl substituted by halogen, lower alkoxy, lower alkoxy substituted by halogen, O(CH₂)ₘO(CH₂)ₘO-lower alkyl substituted by halogen, cyano, lower alkoxy substituted by hydroxy, lower alkenyloxy, C(O)OH, heterocycloalkyl selected from morpholinyl, pyrrolidinyl or pyrrolidin-2-one, or is heteroaryl selected from imidazolyl substituted by lower alkyl, or is NR'R" and R'/R" are independently from each other hydrogen or lower alkyl or -C(O)-lower alkyl; or is -C(O)NR⁴R⁵ and R⁴ is hydrogen or lower alkyl and R⁵ is hydrogen, lower alkyl, lower alkenyl, -(CH₂)ₘO-lower alkyl substituted by halogen, lower alkyl substituted by halogen, -(CH₂)ₙ-phenyl optionally substituted by halogen, -CH₂)ₘNHC(O)-lower alkyl, or -(CH₂)ₘNH₂, or R⁴ and R⁵ may form together with the N-atom to which they are attached a piperidine or azetidine ring, which may be substituted by halogen; or is -C(O)O-lower alkyl substituted by halogen;
- n: is 1 or 2;
- m: is 1, 2 or 3;
or to a pharmaceutically acceptable acid addition salt, to a racemic mixture or to its corresponding enantiomer and/or optical isomers thereof.

The most similar compound imidazo[2,1-b]thiazol-3(2H)-one, 2-[(4-hydroxy-3-methoxyphenyl)methylene]-5-phenyl- is specifically disclosed in Journal of the Indian Chemical Society (1981), 58(11), 1117-18.

WO2010/034982 discloses imidazo[2,1-b]thiazole derivatives useful for diagnosing and treating diseases such as Alzheimer's Disease. These compounds have different peripheral substituents than the presently claimed ones.

It has been shown that the present compounds may be used for binding and imaging tau aggregates and related b-sheet aggregates including besides others beta-amyloid aggregates or alpha-synuclein aggregates, especially for use in binding and imaging tau aggregates in Alzheimer patients.

Alzheimer's disease (AD) is a progressive neurodegenerative disorder characterized by cognitive decline, irreversible memory loss, disorientation and language impairment (Arch. Neurol. 1985, 42(11), 1097-1105). Postmortem examination of AD brain sections reveals abundant senile plaques (SPs), composed of beta amyloid (Aβ) peptides, and numerous neurofibrillary tangles (NFTs) formed by filaments of hyperphosphorylated tau protein.

Tau belongs to the family of microtubule-associated proteins and is mainly expressed in neurons where it plays an important role in the assembly of tubulin monomers into microtubules to constitute the neuronal microtubule network as tracks for axonal transport (Brain Res. Rev. 2000, 33(1), 95-130). Tau is translated from a single gene located on chromosome 17 and the expression is developmentally regulated by an alternative splicing mechanism generating six different isoforms in the human adult brain that can be distinguished by their number of binding domains. The underlying mechanisms leading to tau hyperphosphorylation, misfolding and aggregation are not well understood, but the deposition of tau aggregates follows a stereotyped spatiotemporal pathway both at the intracellular levels as well as on the level of brain topography.

The recent discovery of tau gene mutations leading to frontotemporal dementia (FTD) with parkinsonism linked to chromosome 17 has reinforced the predominant role attributed to tau in the pathogenesis of neurodegenerative disorders and underlined the fact that distinct sets of tau isoforms expressed in different neuronal populations could lead to different pathologies (Biochim. Biophys. Acta 2005, 1739(2) 240-250). Neurodegenerative diseases characterized by pathological tau accumulation are termed 'tauopathies' (Ann. Rev. Neurosci. 2001, 24, 1121-1159). Besides AD and FTD, other tauopathies include progressive supranuclear palsy (PSP), tangle-predominant dementia, Pick's disease, frontotemporal lobar degeneration (FTLD), Down's syndrome and others.

A direct correlation has been established between the progressive involvement of neocortical areas and the increasing severity of dementia, suggesting that pathological tau aggregates such as NFTs are a reliable marker of the neurodegenerative process. The degree of NFT involvement in AD is defined by Braak stages (Acta Neuropathol. 1991, 82, 239-259). Braak stages I and II are defined when NFT involvement is confined mainly to the transentorhinal region of the brain, stages III and IV are diagnosed when limbic regions such as the hippocampus are involved, and stages V and VI when extensive neocortical involvement is found.

Presently, detection of tau aggregates is only possible by histological analysis of biopsy or autopsy materials. *In vivo* imaging of tau pathology would provide novel insights into deposition of tau aggregates in the human brain and allow to non-invasively examine the degree of tau pathology, quantify changes in tau deposition over time, assess its correlation with cognition and analyze the efficacy of an anti-tau therapy. Potential ligands for detecting tau aggregates in the living brain must cross the blood-brain barrier and possess high affinity and specificity for tau aggregates. To this end, successful neuroimaging radiotracers must have appropriate lipophilicity (logD 1-3) and low molecular weight (<450), show rapid clearance from blood and low non-specific binding.

Therefore, the object of the present application is to find an imaging tool which will improve diagnosis by identifying potential patients with excess of tau aggregates in the brain, which may be likely to develop Alzheimer's disease. It will also be useful to monitor the progression of the disease. When an anti-tau aggregate drug become available, imaging tau tangles in the brain may provide a essential tool for monitoring treatment.

A further object of the present invention is a method of imaging tau aggregate deposits, comprising
- introducing into a mammal a detectable quantity of a composition
- allowing sufficient time for the compound of formula I to be associated with tau aggregate deposits, and
- detecting the compound associated with one or more tau aggregate deposits.
A further object of the present invention is a pharmaceutical composition, containing compounds of formula I and pharmaceutical acceptable carriers, which may be used for identifying potential patients.

The following definitions of the general terms used in the present description apply irrespective of whether the terms in question appear alone or in combination.

As used herein, the term "lower alkyl" denotes a saturated, i.e. aliphatic hydrocarbon group including a straight or branched carbon chain with 1 - 7 carbon atoms. Examples for "alkyl" are methyl, ethyl, n-propyl, and isopropyl.

The term "alkoxy" denotes a group -O-R' wherein R' is lower alkyl as defined above.

The term "halogen" denotes chlorine, bromine, fluorine or iodine.

The term "lower alkyl substituted by halogen" denotes an alkyl group as defined above, wherein at least one hydrogen atom is replaced by a halogen atom.

The term "lower alkoxy substituted by halogen" denotes an alkoxy group as defined above, wherein at least one hydrogen atom is replaced by a halogen atom.

The term "lower alkoxy substituted by hydroxy" denotes an alkoxy group as defined above, wherein at least one hydrogen atom is replaced by a hydroxy group.

The term "lower alkenyl" denotes an unsaturated hydrocarbon group, containing from 2 to 7 carbon atoms.

The term "lower alkenyloxy" denotes the group -O-R" wherein R" is lower alkenyl as defined above.

The term "pharmaceutically acceptable salt" or "pharmaceutically acceptable acid addition salt" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

It has been found that the compounds of formula I may be used for binding and imaging tau aggregates and related b-sheet aggregates including besides others beta-amyloid aggregates or alpha-synuclein aggregates.
One object of the present invention are compounds of formula IA 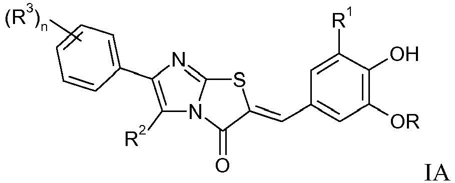 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
- R³: is hydrogen, halogen, lower alkyl, lower alkyl substituted by halogen, lower alkoxy, lower alkoxy substituted by halogen, O(CH₂)ₘO(CH₂)ₘO-lower alkyl substituted by halogen, cyano, lower alkoxy substituted by hydroxy, lower alkenyloxy, C(O)OH, heterocycloalkyl selected from morpholinyl, pyrrolidinyl or pyrrolidin-2-one, or is heteroaryl selected from imidazolyl substituted by lower alkyl, or is NR'R" and R'/R" are independently from each other hydrogen or lower alkyl or -C(O)-lower alkyl; or is -C(O)NR⁴R⁵ and R⁴ is hydrogen or lower alkyl and R⁵ is hydrogen, lower alkyl, lower alkenyl, -(CH₂)ₘO-lower alkyl substituted by halogen, lower alkyl substituted by halogen, -(CH₂)ₙ-phenyl optionally substituted by halogen, -CH₂)ₘNHC(O)-lower alkyl, or -(CH₂)ₘNH₂, or R⁴ and R⁵ may form together with the N-atom to which they are attached a piperidine or azetidine ring, which may be substituted by halogen; or is -C(O)O-lower alkyl substituted by halogen;
- n: is 1 or 2;
- m: is 1, 2 or 3;
or a pharmaceutically acceptable acid addition salt, a racemic mixture or its corresponding enantiomer and/or optical isomers thereof,
for example the following compounds:
6-(4-Chloro-phenyl)-2-[1-(4-hydroxy-3,5-dimethoxy-phenyl)-meth-(Z)-ylidene]-imidazo[2,1-b]thiazol-3-one
6-(4-Chloro-phenyl)-2-[1-(4-hydroxy-3-methoxy-phenyl)-meth-(Z)-ylidene]-imidazo[2,1-b]thiazol-3-one
6-(4-Chloro-phenyl)-2-[1-(3-fluoro-4-hydroxy-5-methoxy-phenyl)-meth-(Z)-ylidene]-imidazo[2,1-b]thiazol-3-one
6-(4-Chloro-phenyl)-2-[1-(3-ethoxy-4-hydroxy-phenyl)-meth-(Z)-ylidene]-imidazo[2,1-b]thiazol-3-one
6-(4-Chloro-phenyl)-2-[1-(3-chloro-4-hydroxy-5-methoxy-phenyl)-meth-(Z)-ylidene]-imidazo[2,1-b]thiazol-3-one
6-(4-Chloro-phenyl)-2-[1-(3-bromo-4-hydroxy-5-methoxy-phenyl)-meth-(Z)-ylidene]-imidazo[2,1-b]thiazol-3-one
6-(4-Chloro-phenyl)-2-[1-(3,4-dihydroxy-5-methoxy-phenyl)-meth-(Z)-ylidene]-imidazo[2,1-b]thiazol-3-one
2-[1-(3-Fluoro-4-hydroxy-5-methoxy-phenyl)-meth-(Z)-ylidene]-6-o-tolyl-imidazo[2,1-b]thiazol-3-one
2-[1-(3-Chloro-4-hydroxy-5-methoxy-phenyl)-meth-(Z)-ylidene]-6-o-tolyl-imidazo[2,1-b]thiazol-3-one
2-[1-(4-hydroxy-3-methoxy-phenyl)-meth-(Z)-ylidene]-6-o-tolyl-imidazo[2,1-b]thiazo1-3-one
2-[1-(3-Chloro-4-hydroxy-5-methoxy-phenyl)-meth-(Z)-ylidene]-6-(4-methoxy-phenyl)-5-methyl-imidazo[2,1-b]thiazol-3-one
2-[1-(3-Fluoro-4-hydroxy-5-methoxy-phenyl)-meth-(Z)-ylidene]-6-(4-methoxy-phenyl)-5-methyl-imidazo[2,1-b]thiazol-3-one
2-[1-(4-Hydroxy-3,5-dimethoxy-phenyl)-meth-(Z)-ylidene]-6-(3-trifluoromethoxy-phenyl)-imidazo[2,1-b]thiazol-3-one
2-[1-(4-Hydroxy-3,5-dimethoxy-phenyl)-meth-(Z)-ylidene]-6-(3-trifluoromethyl-phenyl)-imidazo[2,1-b]thiazol-3-one
2-[1-(4-Hydroxy-3,5-dimethoxy-phenyl)-meth-(Z)-ylidene]-6-m-tolyl-imidazo[2,1-b]thiazo1-3-one
6-(3-Chloro-phenyl)-2-[1-(4-hydroxy-3,5-dimethoxy-phenyl)-meth-(Z)-ylidene]-imidazo[2,1-b]thiazol-3-one
2-[1-(3-Chloro-4-hydroxy-5-methoxy-phenyl)-meth-(Z)-ylidene]-6-(3-trifluoromethoxy-phenyl)-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide
2-[1-(3-Chloro-4-hydroxy-5-methoxy-phenyl)-meth-(Z)-ylidene]-6-(3-trifluoromethyl-phenyl)-imidazo[2,1-b]thiazo1-3-one
6-(3-Chloro-phenyl)-2-[1-(3-chloro-4-hydroxy-5-methoxy-phenyl)-meth-(Z)-ylidene]-imidazo[2,1-b]thiazol-3-one
6-(4-Chloro-phenyl)-2-[1-(3-ethoxy-4-hydroxy-5-methoxy-phenyl)-meth-(Z)-ylidene]-imidazo[2,1-b]thiazol-3-one
6-(4-Chloro-phenyl)-2-[1-(3-(2-fluoro-ethoxy)-4-hydroxy-5-methoxy-phenyl)-meth-(Z)-ylidene]-imidazo[2,1-b]thiazol-3-one
2-[1-(3-Chloro-4-hydroxy-5-methoxy-phenyl)-meth-(Z)-ylidene]-6-(3-fluoro-4-trifluoromethylphenyl)-imidazo[2,1-b]thiazol-3-one
2-[1-(3-Hydroxy-3,5-dimethoxy-phenyl)-meth-(Z)-ylidene]-6-(3-fluoro-4-trifluoromethyl-phenyl)-imidazo[2,1-b]thiazol-3-one
6-(4-Chloro-phenyl)-2-[1-(4-hydroxy-3-isopropoxy-5-methoxy-phenyl)-meth-(Z)-ylidene]-imidazo[2,1-b]thiazol-3-one
6-(4-Chloro-phenyl)-2-[1-(4-hydroxy-3-methoxy-5-propoxy-phenyl)-meth-(Z)-ylidene]-imidazo[2,1-b]thiazol-3-one
6-(4-Chloro-phenyl)-2-[1-(4-hydroxy-3-methoxy-5-methyl-phenyl)-meth-(Z)-ylidene]-imidazo[2,1-b]thiazol-3-one
2-[1-(4-Hydroxy-3-methoxy-5-propoxy-phenyl)-meth-(Z)-ylidene]-6-(3-trifluoromethoxy-phenyl)-imidazo[2,1-b]thiazol-3-one
2-[1-(4-Hydroxy-3-methoxy-5-isopropoxy-phenyl)-meth-(Z)-ylidene]-6-(3-trifluoromethoxy-phenyl)-imidazo[2,1-b]thiazol-3-one
2-[1-(3-Fluoro-4-hydroxy-5-methoxy-phenyl)-meth-(Z)-ylidene]-6-(3-trifluoromethoxy-phenyl)-imidazo[2,1-b]thiazo1-3-one
2-[1-(4-Hydroxy-3-methoxy-phenyl)-meth-(Z)-ylidene]-6-(3-trifluoromethoxy-phenyl)-imidazo[2,1-b]thiazol-3-one
2-[1-(3-Bromo-4-hydroxy-5-methoxy-phenyl)-meth-(Z)-ylidene]-6-(3-trifluoromethoxy-phenyl)-imidazo[2,1-b]thiazol-3-one
3-{2-[-(3-Fluoro-4-hydroxy-5-methoxy-phenyl)-meth-(Z)-ylidene]-3-oxo-2,3-dihydro-imidazo[2,1-b]thiazol-6-yl}-benzonitrile
2-[1-(3-fluoro-4-hydroxy-5-methoxy-phenyl)-meth-(Z)-ylidene]-6-(3-chloro-phenyl)-imidazo[2,1-b]thiazol-3-one
2-[1-(3-Ethyl-4-hydroxy-5-methoxy-phenyl)-meth-(Z)-ylidene]-6-(3-trifluoromethoxy-phenyl)-imidazo[2,1-b]thiazol-3-one
2-[-(3-fluoro-4-hydroxy-5-methoxy-phenyl)-meth-(Z)-ylidene]-6-(3-(trifluoromethyl)-phenyl)-imidazo[2,1-b]thiazol-3-one
2-[1-(3-fluoro-4-hydroxy-5-methoxy-phenyl)-meth-(Z)-ylidene]-6-(4-fluoro-3-(trifluoromethyl)-phenyl)-imidazo[2,1-b]thiazol-3-one
2-[1-(3-fluoro-4-hydroxy-5-methoxy-phenyl)-meth-(Z)-ylidene]-6-phenyl-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile
3-{2-[1-(3-Bromo-4-hydroxy-5-methoxy-phenyl)-meth-(Z)-ylidene]-3-oxo-2,3-dihydro-imidazo[2,1-b]thiazol-6-yl}-benzonitrile
2-[1-(3-bromo-4-hydroxy-5-methoxy-phenyl)-meth-(Z)-ylidene]-6-(4-fluoro-3-(trifluoromethyl)-phenyl)-imidazo[2,1-b]thiazol-3-one
2-[1-(3-Fluoro-4-hydroxy-5-methoxy-phenyl)-meth-(Z)-ylidene]-6-(3-methoxy-phenyl)-imidazo[2,1-b]thiazol-3-one
2-[1-(3-Chloro-4-hydroxy-5-methoxy-phenyl)-meth-(Z)-ylidene]-6-(3,5-difluoro-phenyl)-imidazo[2,1-b]thiazol-3-one
3-{2-[1-(4-Hydroxy-3,5-dimethoxy-phenyl)-meth-(Z)-ylidene]-3-oxo-2,3-dihydro-imidazo[2,1-b]thiazol-6-yl}-benzonitrile
3-{2-[1-(4-Hydroxy-3-methoxy-5-isopropoxyphenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-m-tolyl-imidazo[2,1-b]thiazol-3-one
2-[1-(3-Chloro-4-hydroxy-5-methoxy-phenyl)-meth-(Z)-ylidene]-6-(2-fluoro-phenyl)-imidazo[2,1-b]thiazol-3-one
2-[1-(3-Chloro-4-hydroxy-5-methoxy-phenyl)-meth-(Z)-ylidene]-6-(3-ethyl-phenyl)-imidazo[2,1-b]thiazol-3-one
2-[1-(3-Chloro-4-hydroxy-5-methoxy-phenyl)-meth-(Z)-ylidene]-6-(2-methoxy-phenyl)-imidazo[2,1-b]thiazol-3-one
2-[1-(3-Chloro-4-hydroxy-5-methoxy-phenyl)-meth-(Z)-ylidene]-6-(2,5-difluoro-phenyl)-imidazo[2,1-b]thiazol-3-one
2-[1-(3-Chloro-4-hydroxy-5-methoxy-phenyl)-meth-(Z)-ylidene]-6-(3-fluoro-phenyl)-imidazo[2,1-b]thiazol-3-one
2-[1-(3-Fluoro-4-hydroxy-5-methoxy-phenyl)-meth-(Z)-ylidene]-6-(3-fluoro-phenyl)-imidazo[2,1-b]thiazol-3-one
2-[1-(4-Hydroxy-3,5-dimethoxy-phenyl)-meth-(Z)-ylidene]-6-(3-fluoro-phenyl)-imidazo[2,1-b]thiazol-3-one
2-[1-(3-Chloro-4-hydroxy-5-methoxy-phenyl)-meth-(Z)-ylidene]-6-phenyl-imidazo[2,1-b]thiazol-3-one
2-[1-(3-Chloro-4-hydroxy-5-methoxy-phenyl)-meth-(Z)-ylidene]-6-(3-methoxy-phenyl)-imidazo[2,1-b]thiazol-3-one
2-[1-(3-Chloro-4-hydroxy-5-methoxy-phenyl)-meth-(Z)-ylidene]-6-(3-(difluoromethoxy)-phenyl)-imidazo[2,1-b]thiazol-3-one
(Z)-3-(2-(3-fluoro-4-hydroxy-5-methoxybenzylidene)-3-oxo-2,3-dihydroimidazo[2,1-b]thiazol-5-yl)benzonitrile
2-[1-(3-Chloro-4-hydroxy-5-methoxy-phenyl)-meth-(Z)-ylidene]-6-(3-(chloromethyl)phenyl)-imidazo[2,1-b]thiazol-3-one
3-{2-[1(3-Fluoro-4-hydroxy-5-methoxy-phenyl)-meth-(Z)-ylidene]-3-oxo-2,3-dihydro-imidazo[2,1-b]thiazol-6-yl} -benzonitrile
2-[1-(3-Chloro-4-hydroxy-5-methoxy-phenyl)-meth-(Z)-ylidene]-6-(3-(2-fluoroethoxy)phenyl)-imidazo[2,1-b]thiazol-3-one
2-[1-(3-Chloro-4-hydroxy-5-methoxy-phenyl)-meth-(Z)-ylidene]-6-(4-(2-fluoroethoxy)phenyl)-imidazo[2,1-b]thiazol-3-one
2-[1-(3-Chloro-4-hydroxy-5-methoxy-phenyl)-meth-(Z)-ylidene]-6-(2-(2-fluoroethoxy)phenyl)-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-(4-(difluoromethoxy)phenyl)-imidazo[2,1-b]thiazo1-3-one
2-[1-(3-Chloro-4-hydroxy-5-methoxy-phenyl)-meth-(Z)-ylidene]-6-(3-morpholinophenyl)-imidazo[2,1-b]thiazol-3-one
2-[1-(3-Chloro-4-hydroxy-5-methoxy-phenyl)-meth-(Z)-ylidene]-6-(4-(pyrrolidin-1-yl)phenyl)-imidazo[2,1-b]thiazol-3-one
2-[1-(3-Chloro-4-hydroxy-5-methoxy-phenyl)-meth-(Z)-ylidene]-6-(4-(diethylamino)phenyl)-imidazo[2,1-b]thiazol-3-one
2-[1-(3-Chloro-4-hydroxy-5-methoxy-phenyl)-meth-(Z)-ylidene]-6-(4-morpholinophenyl)-imidazo[2,1-b]thiazol-3-one
2-[1-(3-Chloro-4-hydroxy-5-methoxy-phenyl)-meth-(Z)-ylidene]-6-(4-(dimethylamino)phenyl)-imidazo[2,1-b]thiazol-3-one
2-[1-(3-Chloro-4-hydroxy-5-methoxy-phenyl)-meth-(Z)-ylidene]-6-[3-(2-oxo-pyrrolidin-1-yl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid
2-[1-(3-Chloro-4-hydroxy-5-methoxy-phenyl)-meth-(Z)-ylidene]-6-(3,4-dimethoxy-phenyl)-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide
N-(4-{2-[1-(3-Chloro-4-hydroxy-5-methoxy-phenyl)-meth-(Z)-ylidene]-3-oxo-2,3-dihydro-imidazo[2,1-b]thiazol-6-yl} -phenyl)-acetamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid
2-[1-(3-Chloro-4-hydroxy-5-methoxy-phenyl)-meth-(Z)-ylidene]-6-(2,6-difluoro-phenyl)-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide
3-{2-[1(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide
N-Allyl-3-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-methyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide
4-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-(3-fluoro-propyl)-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(3-fluoro-phenyl)-ethyl]-N-methyl-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-(2-fluoro-ethyl)-N-methyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N,N-dimethyl-benzamide
2-[1-(3-Chloro-4-hydroxy-5-methoxy-phenyl)-meth-(Z)-ylidene]-6-[4-(4-fluoro-piperidine-1-carbonyl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-methyl-N-propyl-benzamide
4-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-(3-fluoropropoxy)-benzamide
2-[1-(3-Chloro-4-hydroxy-5-methoxy-phenyl)-meth-(Z)-ylidene]-6-[4-(3-fluoro-azetidine-1-carbonyl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzoic acid
6-[4-(3-Fluoro-azetidine-1-carbonyl)-phenyl]-2-[1-(3-fluoro-4-hydroxy-5-methoxy-phenyl)-meth-(Z)-ylidene]-imidazo[2,1-b]thiazol-3-one
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-benzamide
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide
2-[1-(3-Chloro-4-hydroxy-5-methoxy-phenyl)-meth-(Z)-ylidene]-6-[3-(3-fluoro-azetidine-1-carbonyl)-phenyl]-imidazo[2,1-b]thiazol-3-one
2-[1-(3-Chloro-4-hydroxy-5-methoxy-phenyl)-meth-(Z)-ylidene]-6-[3-(4-fluoro-piperidine-1-carbon-phenyl]-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazo1-6-yl}-N-[2-(2-fluoro-ethoxy)-ethyl]-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazo1-6-yl}-N-(2-fluoroethoxy)-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoropropoxy)-N-methyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 2-fluoro-ethyl ester
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 3-fluoro-propyl ester
[2-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoylamino)-ethyl]-carbamic acid tert-butyl ester
N-(2-Amino-ethyl)-4-{2-[1-(3-chloro-4-hydroxy-5-methoxy-phenyl)-meth-(Z)-ylidene]-3-oxo-2,3-dihydro-imidazo[2,1-b]thiazo1-6-yl}-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzenesulfonyl fluoride
3-{2-[1-[3-Chloro-5-(3-fluoro-propoxy)-4-hydroxy-phenyl]-meth-(Z)-ylidene]-3-oxo-2,3-dihydro-imidazo[2,1-b]thiazol-6-yl}-benzonitrile
3-Bromo-5-{2-[1-(3-chloro-4-hydroxy-5-methoxy-phenyl)-meth-(Z)-ylidene]-3-oxo-2,3-dihydro-imidazo[2,1-b]thiazo1-6-yl}-benzonitrile
2-[1-(3-Chloro-4-hydroxy-5-methoxy-phenyl)-meth-(Z)-ylidene]-6-(3-{2-[2-(2-fluoro-ethoxy)-ethoxy]-ethoxy}-phenyl)-imidazo[2,1-b]thiazol-3-one or
2-[1-(3-Chloro-4-hydroxy-5-methoxy-phenyl)-meth-(Z)-ylidene]-6-(2-{2-[2-(2-fluoro-ethoxy)-ethoxy]-ethoxy}-phenyl)-imidazo[2,1-b]thiazo1-3-one.

One further embodiment of the invention are compounds of formula IB 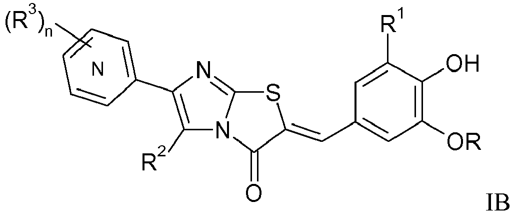 wherein the N-atom of the pyridinyl group may be in different positions, and wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
- R³: is hydrogen, halogen, lower alkyl, lower alkyl substituted by halogen, lower alkoxy, lower alkoxy substituted by halogen, O(CH₂)ₘO(CH₂)ₘO-lower alkyl substituted by halogen, cyano, lower alkoxy substituted by hydroxy, lower alkenyloxy, C(O)OH, heterocycloalkyl selected from morpholinyl, pyrrolidinyl or pyrrolidin-2-one, or is heteroaryl selected from imidazolyl substituted by lower alkyl, or is NR'R" and R'/R" are independently from each other hydrogen or lower alkyl or -C(O)-lower alkyl; or is -C(O)NR⁴R⁵ and R⁴ is hydrogen or lower alkyl and R⁵ is hydrogen, lower alkyl, lower alkenyl, -(CH₂)ₘO-lower alkyl substituted by halogen, lower alkyl substituted by halogen, -(CH₂)ₙ-phenyl optionally substituted by halogen, -CH₂)ₘNHC(O)-lower alkyl, or -(CH₂)ₘNH₂, or R⁴ and R⁵ may form together with the N-atom to which they are attached a piperidine or azetidine ring, which may be substituted by halogen; or is -C(O)O-lower alkyl substituted by halogen;
- n: is 1 or 2;
- m: is 1, 2 or 3;
or a pharmaceutically acceptable acid addition salt, a racemic mixture or its corresponding enantiomer and/or optical isomers thereof, for example
2-[1-(4-Hydroxy-3,5-dimethoxy-phenyl)-meth-(Z)-ylidene]-6-pyridin-4-yl-imidazo[2,1-b]thiazol-3-one
2-[1-(4-Hydroxy-3-methoxy-phenyl)-meth-(Z)-ylidene]-6-(2-methyl-pyridin-4-yl)-imidazo[2,1-b]thiazol-3-one
2-[1-(4-Hydroxy-3-methoxy-phenyl)-meth-(Z)-ylidene]-6-(2,6-dimethyl-pyridin-4-yl)-imidazo[2,1-b]thiazol-3-one
2-[1-(4-Hydroxy-3,5-dimethoxy-phenyl)-meth-(Z)-ylidene]-6-(2-methyl-pyridin-4-yl)-imidazo[2,1-b]thiazol-3-one
2-[1-(4-Hydroxy-3,5-dimethoxy-phenyl)-meth-(Z)-ylidene]-6-(2,6-dimethyl-pyridin-4-yl)-imidazo[2,1-b]thiazol-3-one
2-[1-(3-Fluoro-4-hydroxy-5-methoxy-phenyl)-meth-(Z)-ylidene]-6-(6-methoxy-pyridin-2-yl)-imidazo[2,1-b]thiazol-3-one
2-[1-(3-Chloro-4-hydroxy-5-methoxy-phenyl)-meth-(Z)-ylidene]-6-(2,6-dimethyl-pyridin-4-yl)-imidazo[2,1-b]thiazol-3-one
2-[1-(3-Fluoro-4-hydroxy-5-methoxy-phenyl)-meth-(Z)-ylidene]-6-(2,6-dimethyl-pyridin-4-yl)-imidazo[2,1-b]thiazol-3-one
2-[1-(3-Bromo-4-hydroxy-5-methoxy-phenyl)-meth-(Z)-ylidene]-6-(2,6-dimethyl-pyridin-4-yl)-imidazo[2,1-b]thiazol-3-one
2-[1-(3-Chloro-4-hydroxy-5-methoxy-phenyl)-meth-(Z)-ylidene]-6-(pyridin-4-yl)-imidazo[2,1-b]thiazol-3-one
2-[1-(3-Fluoro-4-hydroxy-5-methoxy-phenyl)-meth-(Z)-ylidene]-6-(pyridin-4-yl)-imidazo[2,1-b]thiazol-3-one
2-[1-(3-Bromo-4-hydroxy-5-methoxy-phenyl)-meth-(Z)-ylidene]-6-(pyridin-4-yl)-imidazo[2,1-b]thiazol-3-one
2-[1-(3-Fluoro-4-hydroxy-5-methoxy-phenyl)-meth-(Z)-ylidene]-6-(pyridin-3-yl)-imidazo[2,1-b]thiazol-3-one
2-[1-(3-Bromo-4-hydroxy-5-methoxy-phenyl)-meth-(Z)-ylidene]-6-(pyridin-3-yl)-imidazo[2,1-b]thiazol-3-one
2-[1-(3-Chloro-4-hydroxy-5-methoxy-phenyl)-meth-(Z)-ylidene]-6-(2-methylpyridin-4-yl)-imidazo[2,1-b]thiazo1-3-one
2-[1-(3-Fluoro-4-hydroxy-5-methoxy-phenyl)-meth-(Z)-ylidene]-6-(2-methylpyridin-4-yl)-imidazo[2,1-b]thiazol-3-one
2-[1-(3-Bromo-4-hydroxy-5-methoxy-phenyl)-meth-(Z)-ylidene]-6-(2-methylpyridin-4-yl)-imidazo[2,1-b]thiazol-3-one
2-[1-(3-Chloro-4-hydroxy-5-methoxy-phenyl)-meth-(Z)-ylidene]-6-(pyridin-3-yl)-imidazo[2,1-b]thiazol-3-one
2-[1-(3-Chloro-4-hydroxy-5-methoxy-phenyl)-meth-(Z)-ylidene]-6-[6-(4-methyl-3H-imidazol-1-yl)-pyridin-3-yl]-imidazo[2,1-b]thiazol-3-one
2-[1-(3-Chloro-4-hydroxy-5-propoxy-phenyl)-meth-(Z)-ylidene]-6-(4-methyl-pyridin-3-yl)-imidazo[2,1-b]thiazo1-3-one
2-[1-(3-Chloro-4-hydroxy-5-propoxy-phenyl)-meth-(Z)-ylidene]-6-pyridin-3-yl-imidazo[2,1-b]thiazol-3-one
2-[1-(3-Chloro-4-hydroxy-5-propoxy-phenyl)-meth-(Z)-ylidene]-6-pyridin-4-yl-imidazo[2,1-b]thiazol-3-one
2-[1-(3-Chloro-4-hydroxy-5-propoxy-phenyl)-meth-(Z)-ylidene]-6-[6-(4-methyl-imidazol-1-yl)-pyridin-3-yl]-imidazo[2,1-b]thiazol-3-one
2-[1-(3-Fluoro-4-hydroxy-5-propoxy-phenyl)-meth-(Z)-ylidene]-6-pyridin-4-yl-imidazo[2,1-b]thiazol-3-one
2-[1-(3-Chloro-5-(3-fluoropropoxy)-4-hydroxy-phenyl)-meth-(Z)-ylidene]-6-pyridin-4-yl-imidazo[2,1-b]thiazo1-3-one
2-[1-(3-Fluoro-5-(3-fluoropropoxy)-4-hydroxy-phenyl)-meth-(Z)-ylidene]-6-pyridin-4-yl-imidazo[2,1-b]thiazol-3-one
2-[1-(3-Chloro-4-hydroxy-5-propoxy-phenyl)-meth-(Z)-ylidene]-6-pyridin-2-yl-imidazo[2,1-b]thiazol-3-one
2-[1-(3-Chloro-4-hydroxy-5-methoxy-phenyl)-meth-(Z)-ylidene]-6-(2-chloro-pyridin-4-yl)-imidazo[2,1-b]thiazol-3-one or
2-[1-[3-Chloro-5-(3-fluoro-propoxy)-4-hydroxy-phenyl]-meth-(Z)-ylidene]-6-(2,6-dimethyl-pyridin-4-yl)-imidazo[2,1-b]thiazol-3-one.

One further embodiment of the invention are compounds of formula IC 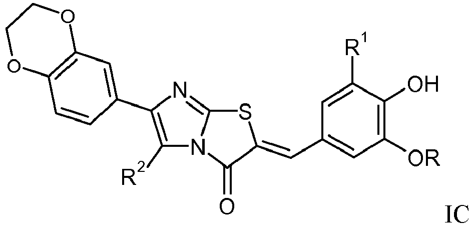
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof,
for example
2-[1-(3-Chloro-4-hydroxy-5-methoxy-phenyl)-meth-(Z)-ylidene]-6-(2,3-dihydro-benzo[1,4]dioxin-6-yl)-imidazo[2,1-b]thiazol-3-one.

One further embodiment of the invention are compounds of formula ID, 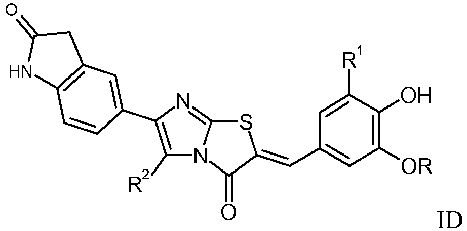 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
and pharmaceutically acceptable salts thereof,
for example
5-{2-[1-(3-Chloro-4-hydroxy-5-methoxy-phenyl)-meth-(Z)-ylidene]-3-oxo-2,3-dihydro-imidazo[2,1-b]thiazol-6-yl}-1,3-dihydro-indol-2-one.

One further embodiment of the invention are compounds of formula IE, 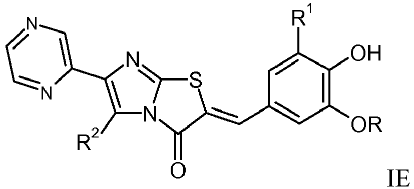 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof,.
for example
2-[1-(3-Chloro-4-hydroxy-5-propoxy-phenyl)-meth-(Z)-ylidene]-6-pyrazin-2-yl-imidazo [2,1-b]thiazol-3-one.

One further embodiment of the invention are compounds of formula IF 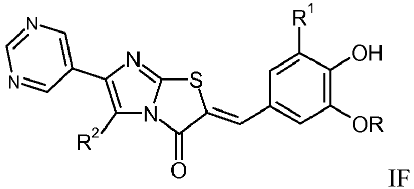 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof,
for example
2-[1-(3-Chloro-4-hydroxy-5-propoxy-phenyl)-meth-(Z)-ylidene]-6-pyrimidin-5-yl-imidazo[2,1-b]thiazol-3-one.

One further embodiment of the invention are compounds of formula IG, 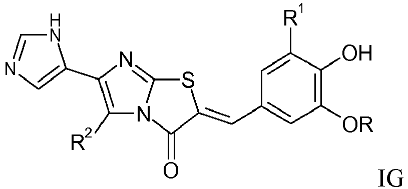 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof, for example
2-[1-(3-Chloro-4-hydroxy-5-propoxy-phenyl)-meth-(Z)-ylidene]-6-(3H-imidazol-4-yl)-imidazo[2,1-b]thiazol-3-one

One further embodiment of the invention are compounds of formula IH, 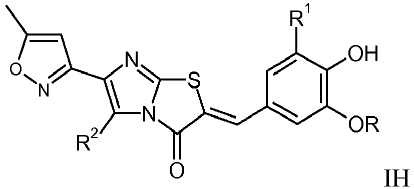 wherein
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof,
for example
2-[1-(3-Chloro-4-hydroxy-5-methoxy-phenyl)-meth-(Z)-ylidene]-6-(5-methyl-isoxazo1-3-yl)-imidazo[2,1-b]thiazol-3-one.

One further embodiment of the invention are compounds of formula IJ, 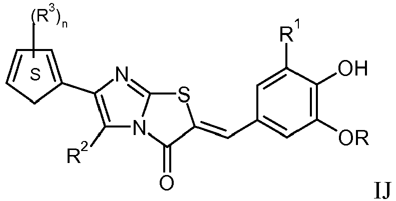 wherein
- R³: is hydrogen or lower alkyl
- n: is 1 or 2
- R: is lower alkyl or lower alkyl substituted by halogen;
- R¹: is hydrogen, halogen, hydroxy, lower alkoxy, lower alkyl and lower alkoxy substituted by halogen;
- R²: is hydrogen, lower alkyl;
or a pharmaceutically acceptable acid addition salt, a racemic mixture or its corresponding enantiomer and/or optical isomers thereof,
for example
2-[1-(3-Chloro-4-hydroxy-5-propoxy-phenyl)-meth-(Z)-ylidene]-6-thiophen-3-yl-imidazo[2,1-b]thiazol-3-one
2-[1-(3-Chloro-4-hydroxy-5-propoxy-phenyl)-meth-(Z)-ylidene]-6-thiophen-2-yl-imidazo[2,1-b]thiazol-3-one or
2-[1-(3-Chloro-4-hydroxy-5-propoxy-phenyl)-meth-(Z)-ylidene]-6-(2,4-dimethylthiophen-3-yl)-imidazo[2,1-b]thiazol-3-one.

The compounds of formula I may be used in binding and imaging tau aggregates, beta-amyloid aggregates, alpha-synuclein aggregates or huntingtin aggregates.

The preferred use of compounds of formula I is the use in binding and imaging tau aggregates in Alzheimer patients.

Furthermore, the compounds of formula I may be used in a tau-binding study.

The compounds of formula I are suitable for diagnostic imaging of tau-aggregates in the brain of a mammal.

In addition, the present invention comprises a pharmaceutical composition containing a compound of formula I and a pharmaceutical acceptable carrier.

Furthermore, the invention comprises a method of imaging tau-aggregate deposits, including
- introducing into a mammal a detectable quantity of a composition;
- allowing sufficient time for the compound of formula I to be associated with tau-aggregate deposit, and
- detecting the compound associated with one or more tau-aggregate deposits.

One object of the invention is also the use of a compound of formula I for diagnostic imaging of tau-aggregate deposits in the brain of a mammal.

The present compounds of formulas 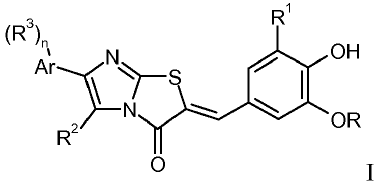 and their pharmaceutically acceptable salts can be prepared by processes described below, which process comprises
a) coupling a compound of formula Ia 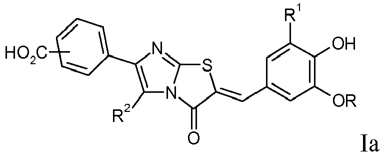 with an suitable amine HNR'R" to afford a compound of formula I 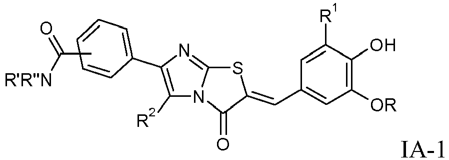 wherein the substituents R, R¹, R², R' and R" are as defined above,
   and if desired, converting the compounds obtained into pharmaceutically acceptable acid addition salts; or
b) coupling a compound with formula III 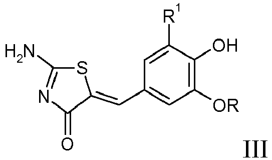 with a corresponding α-activated ketone of formula IVa 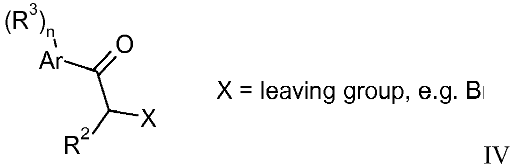 to afford a compound of formula I 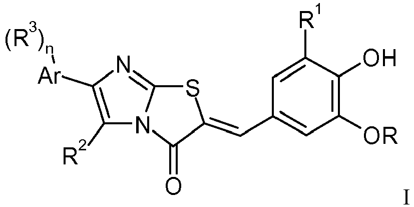 wherein the substituents R, R¹, R² and R³ are as defined above
   and if desired, converting the compounds obtained into pharmaceutically acceptable acid addition salts.
   or
c) coupling a compound with formula V 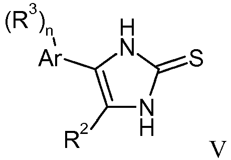 with a suitable acetic acid derivative and a corresponding aldehyde of formula VI 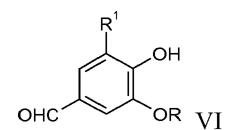 to afford a compound of formula I 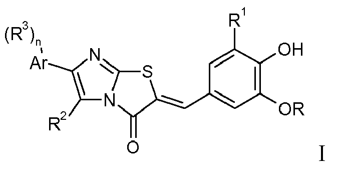 wherein the substituents R, R¹, R² and R³ are as defined above
   and if desired, converting the compounds obtained into pharmaceutically acceptable acid addition salts.

The preparation of compounds of formula **I** of the present invention may be carried out in sequential or convergent synthetic routes. Syntheses of the compounds of the invention are shown in the following schemes 1 to 3. The skills required for carrying out the reaction and purification of the resulting products are known to those skilled in the art. The substituents and indices used in the following description of the processes have the significance given herein before unless indicated to the contrary.

In more detail, the compounds of formula **I** can be manufactured by the methods given below, by the methods given in the examples or by analogous methods. Appropriate reaction conditions for the individual reaction steps are known to a person skilled in the art. The reaction sequence is not limited to the one displayed in schemes 1 to 3, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art. 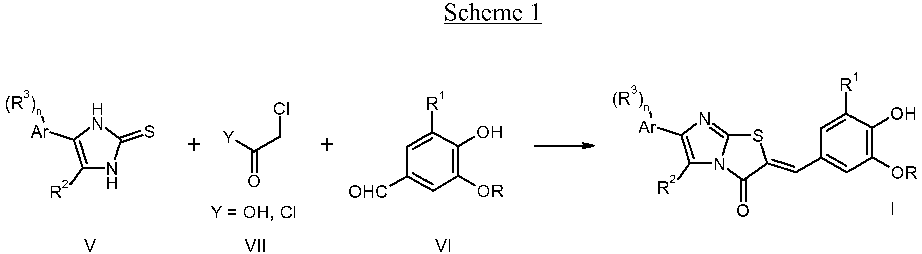 wherein the substituents R, R¹, R² and R³ are as defined above

According to scheme 1, derivatives of imidazothiazolone **I** are prepared *via* a condensation reaction of substituted 1,3-dihydro-imidazole-2-thiones **V,** an activated acetic acid derivative like chloroacetic acid or chloro-acetyl chloride in presence of a base, e.g. ethyldiisopropylamine or sodium acetate, and substituted benzaldehydes **VI** in a suitable solvent, e.g. acetic acid or dioxane, at elevated temperature. 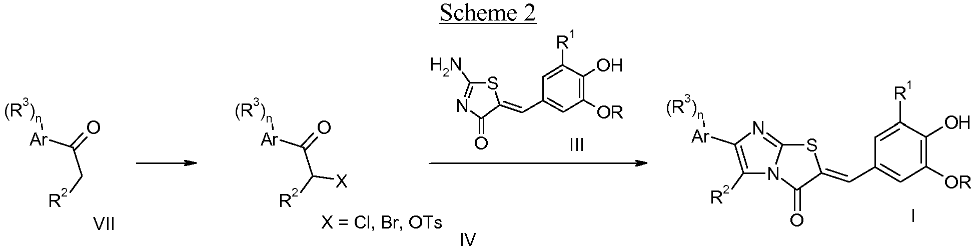 wherein the substituents R, R¹, R² and R³ are as defined above

According to scheme 2, an activated ketone **IV** with e.g. X = BR, is reacted with amino-thiazolone **III** in a suitable solvent, e.g. isopropanol, at elevated temperature in an oilbath or in a microwave to afford derivatives of compound **I.** An activated ketone **IV** can be synthesized *in-situ* by reacting ketone **VII** with an oxidation agent like [hydroxy(tosyloxy)iodo]benzene in a suitable solvent like acetonitrile affording the corresponding activated ketone **IV** with X = O-tosyl which can then react with aminothiazole **III** at elevated temperature yielding derivatives of imidazothiazolones **I.** 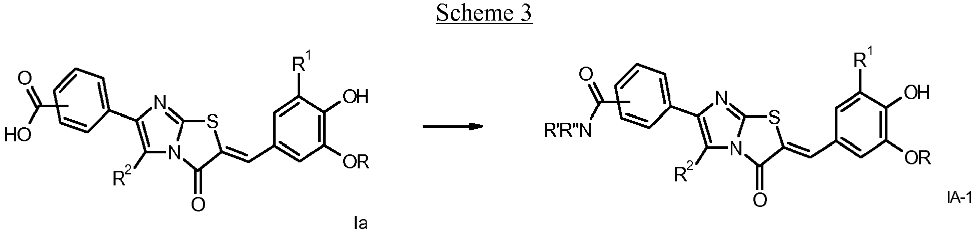 wherein the substituents R, R¹, R², R'and R" are as defined above

According to scheme 3, further derivatives of imidazothiazolones **I** are synthesized by coupling a corresponding carboxylic acid of formula Ia with a corresponding amine HNR'R" by using a suitable amide bond coupling reagent, e.g. 2-(1H-benzo[d][1,2,3]triazol-1-yl)-1,1,3,3-tetramethylisouronium tetrafluoroborate, in a suitable solvent, e.g. N-methyl-2-pyrrolidinone, at ambient or elevated temperature.

### Isolation and purification of the compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the preparations and examples herein below. However, other equivalent separation or isolation procedures could, of course, also be used. Racemic mixtures of chiral compounds of formula IA or IB can be separated using chiral HPLC.

### Salts of compounds of formula I

The compounds of formula I are basic and may be converted to a corresponding acid addition salt. The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained between 0 °C and 50 °C. The resulting salt precipitates spontaneously or may be brought out of solution with a less polar solvent.

The acid addition salts of the basic compounds of formula I may be converted to the corresponding free bases by treatment with at least a stoichiometric equivalent of a suitable base such as sodium or potassium hydroxide, potassium carbonate, sodium bicarbonate, ammonia, and the like.
The compounds were investigated in accordance with the test given hereinafter.

### Assay for determination of IC₁₅₀ for Tau and A-beta

Recombinant human-microtubule associated protein Tau purified from E.coli is aggregated at a concentration of 5 µM with Arachidonic Acid (100 µM) in Tris 10 mM pH8, 24h at 37 °C. Synthetic Aβ40 is aggregated. at a concentration of 50 µM with Arachidonic Acid (100 µM) in Tris 10 mM pH8, for three days at 37°C, under shaking at 150 rpm.

Human recombinant-Alpha-synuclein-purified from E.coli is aggregated at a concentration of 70 µM with Arachidonic Acid (100 µM) in Tris 10 mM pH 8, for 5 days at 37°C, under shaking at 150 rpm.

A saturation analysis of Thiazin-red R to the aggregated proteins is done to determine the affinity (Kd) of the Thiazin-red R to the aggregated protein. Table 1 shows the affinity constants of Thiazin-red R for aggregated tau, Abeta and alpha-synuclein. The results show that there are two binding sites with different affinity on each aggregated protein for Thiazin-red R.

Thiazin-red R will be added at the concentration corresponding to the Kd to the respective aggregated protein binding site, to induce a fluorescent signal that can be inhibited by the addition of a displacer compound.

To determine the affinity of a displacer compound to the Thiazin -red R binding sites of the aggregated proteins, the compound is added at different concentrations in the assay ranging from 0.3 nM to 10000 nM.

In parallel, auto fluorescence of the compound is measured together with the aggregated proteins, but without Thiazin-red R. As negative control, ligand and aggregated protein is used and as positive control, Thiazin-red R, reference compound with known activity and aggregated protein is used.

Assay is performed in Perkin Elmer OptiPlate 384, black, 45 ul assay volume, assay buffer is DPBS no CaCl₂ no MgCl₂ (GIBCO N. 14020). Tested compounds are diluted in DMSO and 2 µl is added to the assay (5% DMSO final). Assay is started by the addition of the aggregated protein (competitive condition). Plates are shortly shacked (1 min with Sterico variomag teleshake) and incubated for 30 min at room temperature. Measurement are done with En:Vision (Perkin Elmer), at Excitation 531 nm / Emission 595 nm.

**Table**

| | | | | |
|---|---|---|---|---|
| 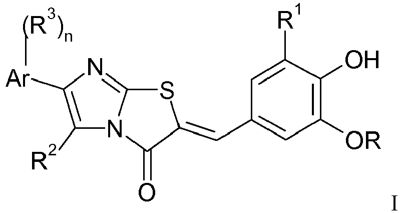 | | | | |

| **Structure** | **Name** | **IC₅₀ Tau** | **IC₅₀ A-beta** | **Expl.** |
|---|---|---|---|---|
| 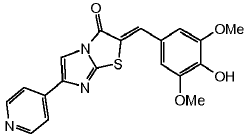 | 2-[1-(4-Hydroxy-3,5-dimethoxy-phenyl)-meth-(Z)-ylidene]-6-pyridin-4-yl-imidazo[2,1-b]thiazol-3-one | 0.005 | 0.093 | 1 |
| 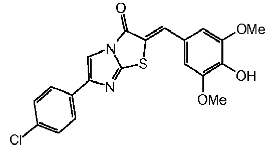 | 6-(4-Chloro-phenyl)-2-[1-(4-hydroxy-3,5-dimethoxy-phenyl)-meth-(Z)-ylidene]-imidazo[2,1-b]thiazol-3-one | 0.002 | 0.007 | 2 |
| 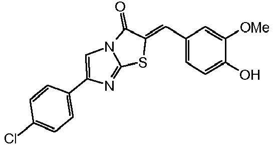 | 6-(4-Chloro-phenyl)-2-[1-(4-hydroxy-3-methoxy-phenyl)-meth-(Z)-ylidene]-imidazo[2,1-b]thiazol-3-one | 0.049 | 0.215 | 3 |
| 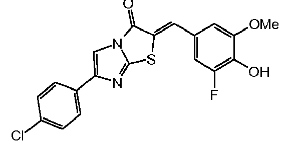 | 6-(4-Chloro-phenyl)-2-[1-(3-fluoro-4-hydroxy-5-methoxyphenyl)-meth-(Z)-ylidene]-imidazo[2,1-b]thiazol-3-one | 0.011 | 0.393 | 4 |
| 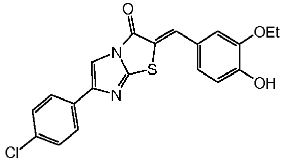 | 6-(4-Chloro-phenyl)-2-[1-(3-ethoxy-4-hydroxy-phenyl)-meth-(Z)-ylidene]-imidazo[2,1-b]thiazol-3-one | 0.178 | 1.156 | 5 |
| 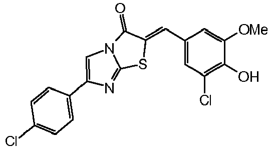 | 6-(4-Chloro-phenyl)-2-[1-(3-chloro-4-hydroxy-5-methoxy-phenyl)-meth-(Z)-ylidene]-imidazo[2,1-b]thiazol-3-one | 0.002 | 0.032 | 6 |
| 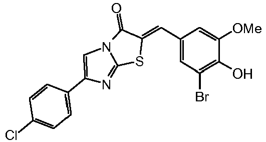 | 6-(4-Chloro-phenyl)-2-[1-(3-bromo-4-hydroxy-5-methoxy-phenyl)-meth-(Z)-ylidene]-imidazo[2,1-b]thiazol-3-one | 0.002 | 0.018 | 7 |
| 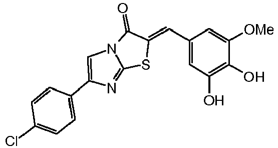 | 6-(4-Chloro-phenyl)-2-[1-(3,4-dihydroxy-5-methoxy-phenyl)-meth-(Z)-ylidene]-imidazo[2,1-b]thiazol-3-one | 0.005 | 0.018 | 8 |
| 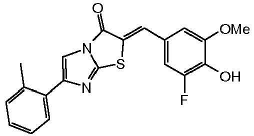 | 2-[1-(3-Fluoro-4-hydroxy-5-methoxy-phenyl)-meth-(Z)-ylidene]-6-o-tolyl-imidazo[2,1-b]thiazol-3-one | 0.105 | 0.706 | 9 |
| 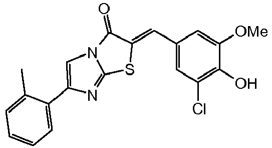 | 2-[1-(3-Chloro-4-hydroxy-5-methoxy-phenyl)-meth-(Z)-ylidene]-6-o-tolyl-imidazo[2,1-b]thiazol-3-one | 0.028 | 1.217 | 10 |
| 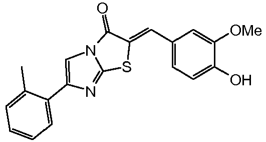 | 2-[1-(4-hydroxy-3-methoxy-phenyl)-meth-(Z)-ylidene]-6-o-tolyl-imidazo[2,1-b]thiazol-3-one | 0.837 | 1.283 | 11 |
| 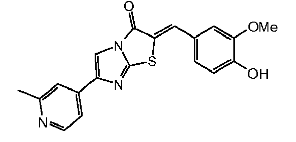 | 2-[1-(4-Hydroxy-3-methoxy-phenyl)-meth-(Z)-ylidene]-6-(2-methyl-pyridin-4-yl)-imidazo [2,1-b]thiazol-3-one | 0.079 | >10 | 12 |
| 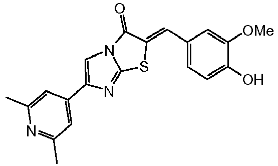 | 2-[1-(4-Hydroxy-3-methoxy-phenyl)-meth-(Z)-ylidene]-6-(2,6-dimethyl-pyridin-4-yl)-imidazo [2,1-b]thiazol-3-one | 0.091 | >10 | 13 |
| 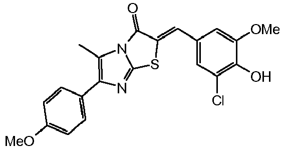 | 2-[1-(3-Chloro-4-hydroxy-5-methoxy-phenyl)-meth-(Z)-ylidene]-6-(4-methoxyphenyl)-5-methyl-imidazo[2,1-b]thiazol-3-one | 0.037 | 0.466 | 14 |
| 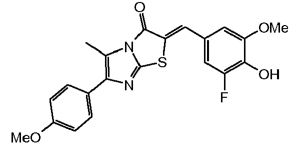 | 2-[1-(3-Fluoro-4-hydroxy-5-methoxy-phenyl)-meth-(Z)-ylidene]-6-(4-methoxyphenyl)-5-methyl-imidazo[2,1-b]thiazol-3-one | 0.053 | 0.437 | 15 |
| 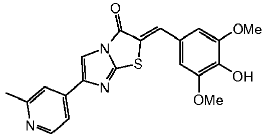 | 2-[1-(4-Hydroxy-3,5-dimethoxy-phenyl)-meth-(Z)-ylidene]-6-(2-methyl-pyridin-4-yl)-imidazo [2,1-b]thiazol-3-one | 0.008 | 0.032 | 16 |
| 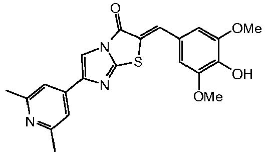 | 2-[1-(4-Hydroxy-3,5-dimethoxy-phenyl)-meth-(Z)-ylidene]-6-(2,6-dimethyl-pyridin-4-yl)-imidazo[2,1-b]thiazol-3-one | 0.005 | 0.068 | 17 |
| 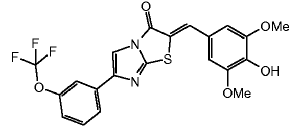 | 2-[1-(4-Hydroxy-3,5-dimethoxy-phenyl)-meth-(Z)-ylidene]-6-(3-trifluoromethoxy-phenyl)-imidazo[2,1-b]thiazol-3-one | 0.004 | 0.114 | 18 |
| 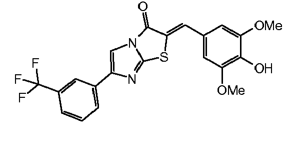 | 2-[1-(4-Hydroxy-3,5-dimethoxy-phenyl)-meth-(Z)-ylidene]-6-(3-trifluoromethyl-phenyl)-imidazo[2,1-b]thiazo1-3-one | 0.001 | 0.115 | 19 |
| 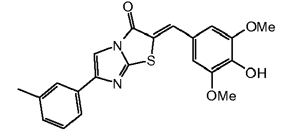 | 2-[1-(4-Hydroxy-3,5-dimethoxy-phenyl)-meth-(Z)-ylidene]-6-m-tolyl-imidazo[2,1-b]thiazo1-3-one | 0.003 | 0.020 | 20 |
| 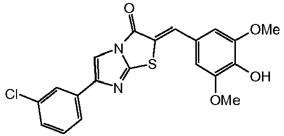 | 6-(3-Chloro-phenyl)-2-[1-(4-hydroxy-3,5-dimethoxy-phenyl)-meth-(Z)-ylidene]-imidazo[2,1-b]thiazo1-3-one | 0.001 | 0.006 | 21 |
| 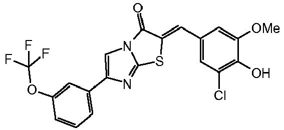 | 2-[1-(3-Chloro-4-hydroxy-5-methoxy-phenyl)-meth-(Z)-ylidene]-6-(3-trifluoromethoxy-phenyl)-imidazo[2,1-b]thiazo1-3-one | 0.003 | 0.198 | 22 |
| 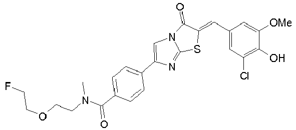 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide | 0.026 | 0.328 | 23 |
| 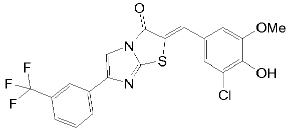 | 2-[1-(3-Chloro-4-hydroxy-5-methoxy-phenyl)-meth-(Z)-ylidene]-6-(3-trifluoromethyl-phenyl)-imidazo[2,1-b]thiazo1-3-one | 0.003 | 0.123 | 24 |
| 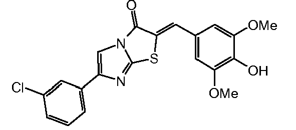 | 6-(3-Chloro-phenyl)-2-[1-(3-chloro-4-hydroxy-5-methoxy-phenyl)-meth-(Z)-ylidene]-imidazo[2,1-b]thiazol-3-one | 0.0005 | 0.253 | 25 |
| 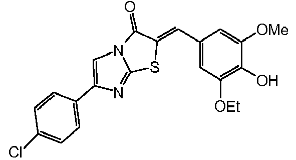 | 6-(4-Chloro-phenyl)-2-[1-(3-ethoxy-4-hydroxy-5-methoxy-phenyl)-meth-(Z)-ylidene]-imidazo[2,1-b]thiazol-3-one | 0.002 | 0.004 | 26 |
| 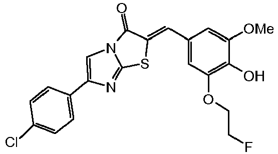 | 6-(4-Chloro-phenyl)-2-[1-(3-(2-fluoro-ethoxy)-4-hydroxy-5-methoxy-phenyl)-meth-(Z)-ylidene]-imidazo[2,1-b]thiazol-3-one | 0.001 | 0.007 | 27 |
| 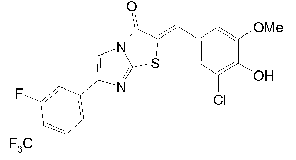 | 2-[1-(3-Chloro-4-hydroxy-5-methoxy-phenyl)-meth-(Z)-ylidene]-6-(3-fluoro-4-trifluoromethyl-phenyl)-imidazo[2,1-b]thiazol-3-one | 0.023 | 1.056 | 28 |
| 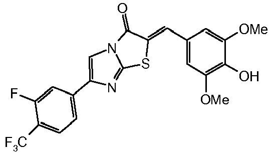 | 2-[1-(3-Hydroxy-3,5-dimethoxy-phenyl)-meth-(Z)-ylidene]-6-(3-fluoro-4-trifluoromethyl-phenyl)-imidazo[2,1-b]thiazo1-3-one | 0.011 | 0.043 | 29 |
| 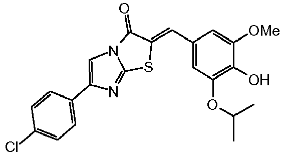 | 6-(4-Chloro-phenyl)-2-[1-(4-hydroxy-3-isopropoxy-5-methoxy-phenyl)-meth-(Z)-ylidene]-imidazo [2,1-b]thiazol-3-one | 0.006 | 0.016 | 30 |
| 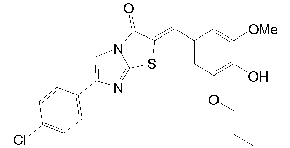 | 6-(4-Chloro-phenyl)-2-[1-(4-hydroxy-3-methoxy-5-propoxy-phenyl)-meth-(Z)-ylidene]-imidazo[2,1-b]thiazol-3-one | 0.003 | 0.012 | 31 |
| 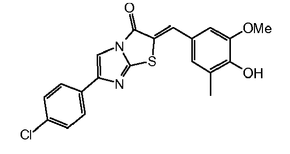 | 6-(4-Chloro-phenyl)-2-[1-(4-hydroxy-3-methoxy-5-methyl-phenyl)-meth-(Z)-ylidene]-imidazo[2,1-b]thiazol-3-one | 0.007 | 0.039 | 32 |
| 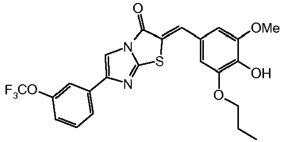 | 2-[1-(4-Hydroxy-3-methoxy-5-propoxy-phenyl)-meth-(Z)-ylidene]-6-(3-trifluoromethoxyphenyl)-imidazo[2,1-b]thiazol-3-one | 0.025 | 0.256 | 33 |
| 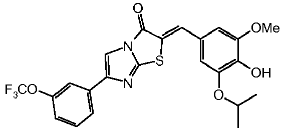 | 2-[1-(4-Hydroxy-3-methoxy-5-isopropoxy-phenyl)-meth-(Z)-ylidene]-6-(3-trifluoromethoxyphenyl)-imidazo[2,1-b]thiazol-3-one | 0.026 | 0.437 | 34 |
| 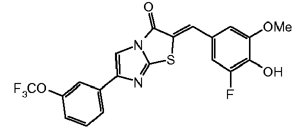 | 2-[ 1-(3-Fluoro-4-hydroxy-5-methoxy-phenyl)-meth-(Z)-ylidene]-6-(3-trifluoromethoxy-phenyl)-imidazo[2,1-b]thiazo1-3-one | 0.013 | >10 | 35 |
| 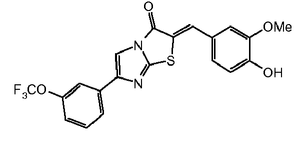 | 2-[1-(4-Hydroxy-3-methoxy-phenyl)-meth-(Z)-ylidene]-6-(3-trifluoromethoxy-phenyl)-imidazo[2,1-b]thiazo1-3-one | 0.401 | >10 | 36 |
| 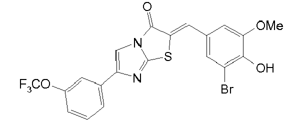 | 2-[1-(3-Bromo-4-hydroxy-5-methoxy-phenyl)-meth-(Z)-ylidene]-6-(3-trifluoromethoxy-phenyl)-imidazo[2,1-b]thiazo1-3-one | 0.006 | 0.552 | 37 |
| 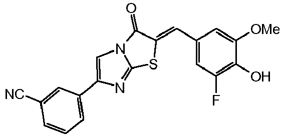 | 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile | 0.021 | >10 | 38 |
| 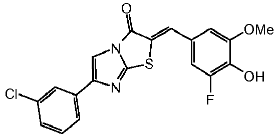 | 2-[ 1-(3-fluoro-4-hydroxy-5-methoxy-phenyl)-meth-(Z)-ylidene]-6-(3-chlorophenyl)-imidazo[2,1-b]thiazol-3-one | 0.011 | 0.412 | 39 |
| 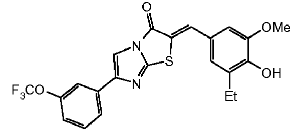 | 2-[1-(3-Ethyl-4-hydroxy-5-methoxy-phenyl)-meth-(Z)-ylidene]-6-(3-trifluoromethoxy-phenyl)-imidazo[2,1-b]thiazol-3-one | 0.701 | >10 | 40 |
| 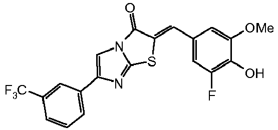 | 2-[1-(3-fluoro-4-hydroxy-5-methoxy-phenyl)-meth-(Z)-ylidene]-6-(3-(trifluoromethyl)-phenyl)-imidazo[2,1-b]thiazol-3-one | 0.016 | 1.810 | 41 |
| 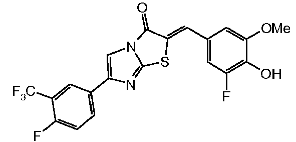 | 2-[1-(3-fluoro-4-hydroxy-5-methoxy-phenyl)-meth-(Z)-ylidene]-6-(4-fluoro-3-(trifluoromethyl)-phenyl)-imidazo[2,1-b]thiazol-3-one | 0.024 | 0.198 | 42 |
| 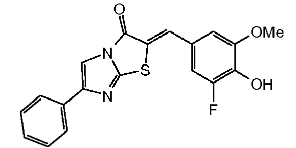 | 2-[1-(3-fluoro-4-hydroxy-5-methoxy-phenyl)-meth-(Z)-ylidene]-6-phenyl-imidazo[2,1-b]thiazol-3-one | 0.051 | 2.320 | 43 |
| 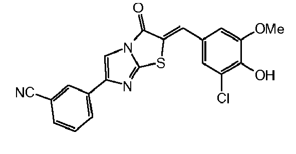 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile | 0.006 | 24.015 | 44 |
| 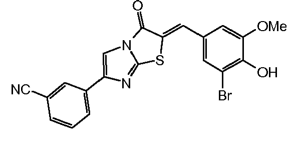 | 3-{2-[1-(3-Bromo-4-hydroxy-5-methoxy-phenyl)-meth-(Z)-ylidene]-3-oxo-2,3-dihydro-imidazo[2,1-b]thiazol-6-yl}-benzonitrile | 0.025 | >10 | 45 |
| 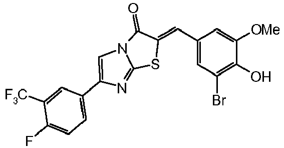 | 2-[1-(3-bromo-4-hydroxy-5-methoxy-phenyl)-meth-(Z)-ylidene]-6-(4-fluoro-3-(trifluoromethyl)-phenyl)-imidazo[2,1-b]thiazo1-3-one | 0.028 | 5.9105 | 46 |
| 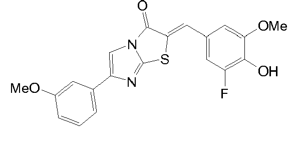 | 2-[1-(3-Fluoro-4-hydroxy-5-methoxy-phenyl)-meth-(Z)-ylidene]-6-(3-methoxyphenyl)-imidazo[2,1-b]thiazol-3-one | 0.017 | 0.540 | 47 |
| 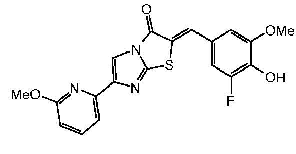 | 2-[ 1-(3-Fluoro-4-hydroxy-5-methoxy-phenyl)-meth-(Z)-ylidene]-6-(6-methoxy-pyridin-2-yl)-imidazo[2,1-b]thiazol-3-one | 0.020 | 1.790 | 48 |
| 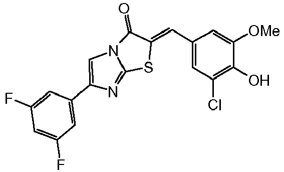 | 2-[1-(3-Chloro-4-hydroxy-5-methoxy-phenyl)-meth-(Z)-ylidene]-6-(3,5-difluoro-phenyl)-imidazo[2,1-b]thiazo1-3-one | 0.016 | 1.371 | 49 |
| 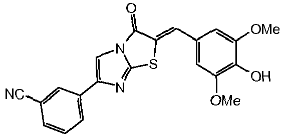 | 3-{2-[1-(4-Hydroxy-3,5-dimethoxy-phenyl)-meth-(Z)-ylidene]-3-oxo-2,3-dihydro-imidazo[2,1-b]thiazol-6-yl}-benzonitrile | 0.007 | | 50 |
| 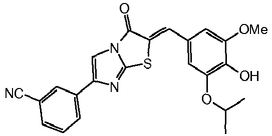 | 3-{2-[1-(4-Hydroxy-3-methoxy-5-isopropoxyphenyl)-meth-(Z)-ylidene]-3-oxo-2,3-dihydro-imidazo [2,1-b]thiazol-6-yl}-benzonitrile | 0.275 | >10 | 51 |
| 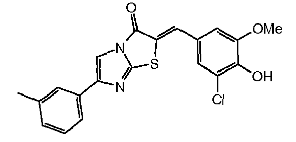 | 2-[1-(3-Chloro-4-hydroxy-5-methoxy-phenyl)-meth-(Z)-ylidene]-6-m-tolyl-imidazo[2,1-b]thiazo1-3-one | 0.007 | 1.023 | 52 |
| 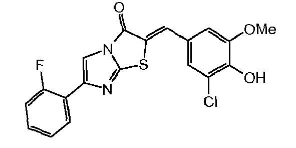 | 2-[1-(3-Chloro-4-hydroxy-5-methoxy-phenyl)-meth-(Z)-ylidene]-6-(2-fluoro-phenyl)-imidazo[2,1-b]thiazol-3-one | 0.016 | 5.649 | 53 |
| 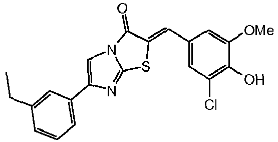 | 2-[1-(3-Chloro-4-hydroxy-5-methoxy-phenyl)-meth-(Z)-ylidene]-6-(3-ethylphenyl)-imidazo[2,1-b]thiazol-3-one | 0.025 | 0.653 | 54 |
| 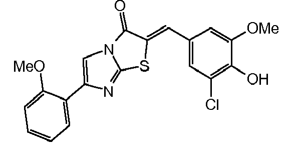 | 2-[1-(3-Chloro-4-hydroxy-5-methoxy-phenyl)-meth-(Z)-ylidene]-6-(2-methoxyphenyl)-imidazo[2,1-b]thiazol-3-one | 0.103 | | 55 |
| 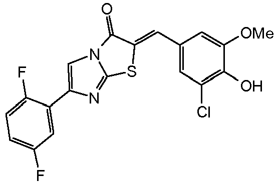 | 2-[1-(3-Chloro-4-hydroxy-5-methoxy-phenyl)-meth-(Z)-ylidene]-6-(2,5-difluoro-phenyl)-imidazo[2,1-b]thiazol-3-one | 0.027 | | 56 |
| 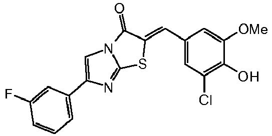 | 2-[1-(3-Chloro-4-hydroxy-5-methoxy-phenyl)-meth-(Z)-ylidene]-6-(3-fluorophenyl)-imidazo[2,1-b]thiazol-3-one | 0.006 | 3.323 | 57 |
| 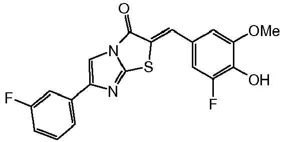 | 2-[1-(3-Fluoro-4-hydroxy-5-methoxy-phenyl)-meth-(Z)-ylidene]-6-(3-fluorophenyl)-imidazo[2,1-b]thiazol-3-one | 0.019 | 0.752 | 58 |
| 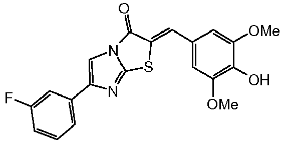 | 2-[1-(4-Hydroxy-3,5-dimethoxy-phenyl)-meth-(Z)-ylidene]-6-(3-fluorophenyl)-imidazo[2,1-b]thiazol-3-one | 0.002 | 0.05 | 59 |
| 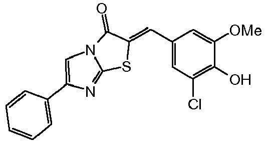 | 2-[1-(3-Chloro-4-hydroxy-5-methoxy-phenyl)-meth-(Z)-ylidene]-6-phenyl-imidazo[2,1-b]thiazol-3-one | 0.025 | 0.851 | 60 |
| 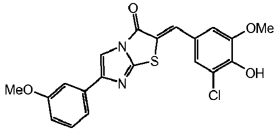 | 2-[1-(3-Chloro-4-hydroxy-5-methoxy-phenyl)-meth-(Z)-ylidene]-6-(3-methoxy-phenyl)-imidazo[2,1-b]thiazol-3-one | 0.004 | 0.395 | 61 |
| 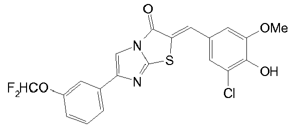 | 2-[1-(3-Chloro-4-hydroxy-5-methoxy-phenyl)-meth-(Z)-ylidene]-6-(3-(difluoromethoxy)-phenyl)-imidazo[2,1-b]thiazo1-3-one | 0.006 | 4.161 | 62 |
| 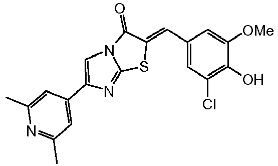 | 2-[1-(3-Chloro-4-hydroxy-5-methoxy-phenyl)-meth-(Z)-ylidene]-6-(2,6-dimethyl-pyridin-4-yl)-imidazo[2,1-b]thiazol-3-one | 0.013 | 0.418 | 63 |
| 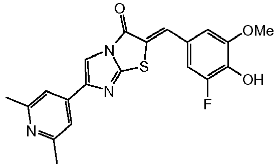 | 2-[1-(3-Fluoro-4-hydroxy-5-methoxy-phenyl)-meth-(Z)-ylidene]-6-(2,6-dimethyl-pyridin-4-yl)-imidazo[2,1-b]thiazol-3-one | 0.032 | 0.406 | 64 |
| 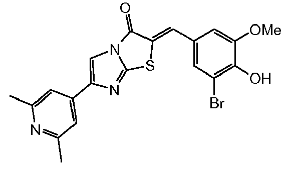 | 2-[1-(3-Bromo-4-hydroxy-5-methoxy-phenyl)-meth-(Z)-ylidene]-6-(2,6-dimethyl-pyridin-4-yl)-imidazo[2,1-b]thiazol-3-one | 0.007 | 0.304 | 65 |
| 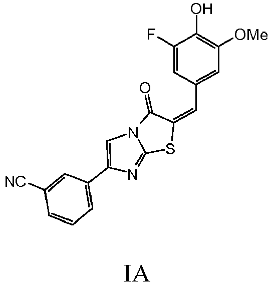 | (Z)-3-(2-(3-fluoro-4-hydroxy-5-methoxybenzylidene)-3-oxo-2,3-dihydroimidazo [2,1-b]thiazol-5-yl)benzonitrile | 0.004 | 0.008 | 66 |
| 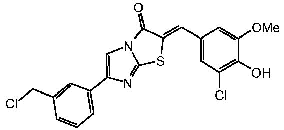 | 2-[ 1-(3-Chloro-4-hydroxy-5-methoxy-phenyl)-meth-(Z)-ylidene]-6-(3-(chloromethyl)phenyl)-imidazo[2,1-b]thiazol-3-one | 0.0008 | 0.009 | 67 |
| 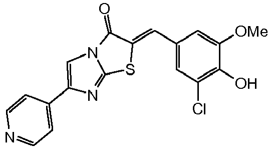 | 2-[1-(3-Chloro-4-hydroxy-5-methoxy-phenyl)-meth-(Z)-ylidene]-6-(pyridin-4-yl)-imidazo[2,1-b]thiazol-3-one | 0.003 | 0.300 | 68 |
| 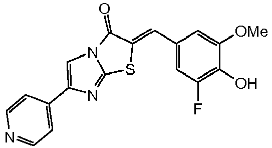 | 2-[1-(3-Fluoro-4-hydroxy-5-methoxy-phenyl)-meth-(Z)-ylidene]-6-(pyridin-4-yl)-imidazo[2,1-b]thiazol-3-one | 0.020 | 0.62 | 69 |
| 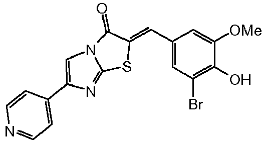 | 2-[1-(3-Bromo-4-hydroxy-5-methoxy-phenyl)-meth-(Z)-ylidene]-6-(pyridin-4-yl)-imidazo[2,1-b]thiazol-3-one | 0.0013 | 0.103 | 70 |
| 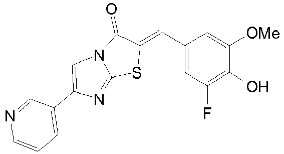 | 2-[1-(3-Fluoro-4-hydroxy-5-methoxy-phenyl)-meth-(Z)-ylidene]-6-(pyridin-3-yl)-imidazo[2,1-b]thiazol-3-one | 0.020 | 0.238 | 71 |
| 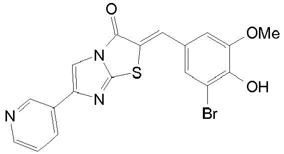 | 2-[1-(3-Bromo-4-hydroxy-5-methoxy-phenyl)-meth-(Z)-ylidene]-6-(pyridin-3-yl)-imidazo[2,1-b]thiazol-3-one | 0.003 | 0.229 | 72 |
| 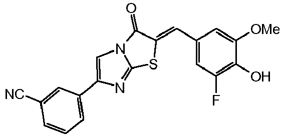 | 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile | 0.003 | 0.013 | 73 |
| 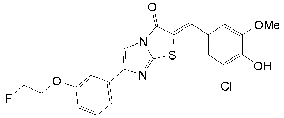 | 2-[1-(3-Chloro-4-hydroxy-5-methoxy-phenyl)-meth-(Z)-ylidene]-6-(3-(2-fluoroethoxy)phenyl)-imidazo[2,1-b]thiazo1-3-one | 0.005 | 0.055 | 74 |
| 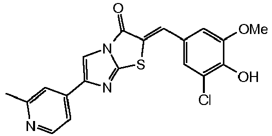 | 2-[1-(3-Chloro-4-hydroxy-5-methoxy-phenyl)-meth-(Z)-ylidene]-6-(2-methylpyridin-4-yl)-imidazo[2,1-b]thiazo1-3-one | 0.0013 | 0.122 | 75 |
| 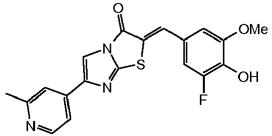 | 2-[1-(3-Fluoro-4-hydroxy-5-methoxy-phenyl)-meth-(Z)-ylidene]-6-(2-methylpyridin-4-yl)-imidazo[2,1-b]thiazo1-3-one | 0.006 | 0.260 | 76 |
| 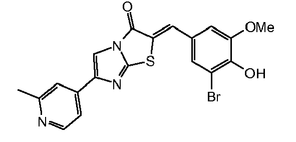 | 2-[1-(3-Bromo-4-hydroxy-5-methoxy-phenyl)-meth-(Z)-ylidene]-6-(2-methylpyridin-4-yl)-imidazo[2,1-b]thiazo1-3-one | 0.001 | 0.076 | 77 |
| 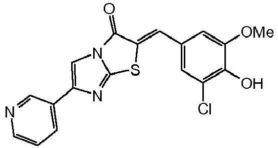 | 2-[1-(3-Chloro-4-hydroxy-5-methoxy-phenyl)-meth-(Z)-ylidene]-6-(pyridin-3-yl)-imidazo[2,1-b]thiazol-3-one | 0.0104 | 0.700 | 78 |
| 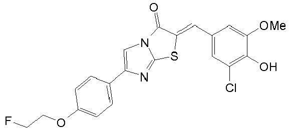 | 2-[1-(3-Chloro-4-hydroxy-5-methoxy-phenyl)-meth-(Z)-ylidene]-6-(4-(2-fluoroethoxy)phenyl)-imidazo[2,1-b]thiazol-3-one | 0.006 | 0.025 | 79 |
| 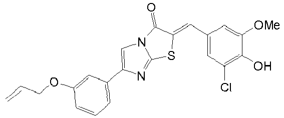 | 2-[1-(3-Chloro-4-hydroxy-5-methoxy-phenyl)-meth-(Z)-ylidene]-6-(3-(allyloxy)phenyl)-imidazo[2,1-b]thiazol-3-one | 0.006 | 0.020 | 80 |
| 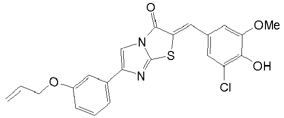 | 2-[1-(3-Chloro-4-hydroxy-5-methoxy-phenyl)-meth-(Z)-ylidene]-6-(3-propoxyphenyl)-imidazo[2,1-b]thiazol-3-one | 0.011 | 1.498 | 81 |
| 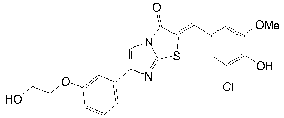 | 2-[1-(3-Chloro-4-hydroxy-5-methoxy-phenyl)-meth-(Z)-ylidene]-6-(2-(hydroxyethoxy)phenyl)-imidazo[2,1-b]thiazol-3-one | 0.003 | 0.174 | 82 |
| 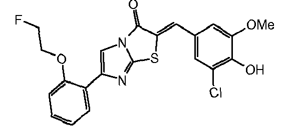 | 2-[1-(3-Chloro-4-hydroxy-5-methoxy-phenyl)-meth-(Z)-ylidene]-6-(2-(2-fluoroethoxy)phenyl)-imidazo[2,1-b]thiazol-3-one | 0.173 | | 83 |
| 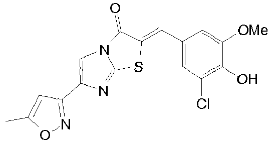 | 2-[1-(3-Chloro-4-hydroxy-5-methoxy-phenyl)-meth-(Z)-ylidene]-6-(5-methyl-isoxazol-3-yl)-imidazo[2,1-b]thiazol-3-one | 0.018 | 0.990 | 84 |
| 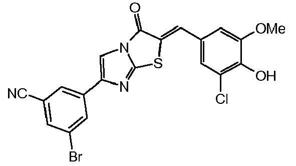 | 3-Bromo-5-{2-[1-(3-chloro-4-hydroxy-5-methoxy-phenyl)-meth-(Z)-ylidene]-3-oxo-2,3-dihydro-imidazo [2,1-b]thiazol-6-yl}-benzonitrile | 0.0008 | 1.730 | 85 |
| 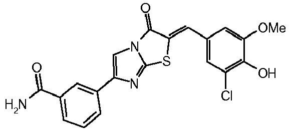 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide | 0.001 | 1.242 | 86 |
| 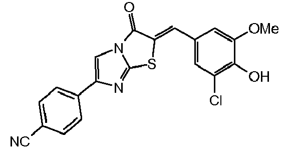 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile | 0.0003 | 0.006 | 87 |
| 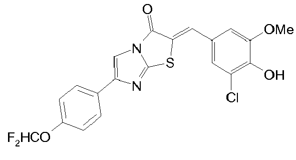 | 2-[1-(3-Chloro-4-hydroxy-5-methoxy-phenyl)-meth-(Z)-ylidene]-6-(4-(difluoromethoxy)phenyl)-imidazo[2,1-b]thiazol-3-one | 0.0006 | 0.014 | 88 |
| 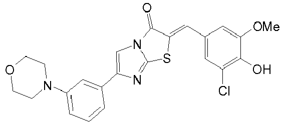 | 2-[1-(3-Chloro-4-hydroxy-5-methoxy-phenyl)-meth-(Z)-ylidene]-6-(3-morpholinophenyl)-imidazo[2,1-b]thiazol-3-one | 0.024 | 0.356 | 89 |
| 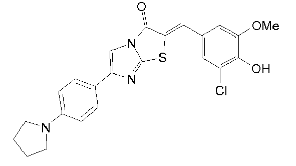 | 2-[1-(3-Chloro-4-hydroxy-5-methoxy-phenyl)-meth-(Z)-ylidene]-6-(4-(pyrrolidin-1-yl)phenyl)-imidazo[2,1-b]thiazol-3-one | 0.037 | 0.016 | 90 |
| 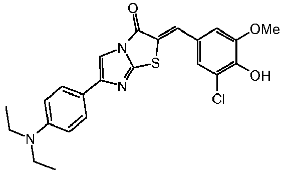 | 2-[1-(3-Chloro-4-hydroxy-5-methoxy-phenyl)-meth-(Z)-ylidene]-6-(4-(diethylamino)phenyl)-imidazo[2,1-b]thiazol-3-one | 0.020 | 0.184 | 91 |
| 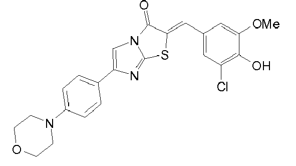 | 2-[1-(3-Chloro-4-hydroxy-5-methoxy-phenyl)-meth-(Z)-ylidene]-6-(4-morpholinophenyl)-imidazo[2,1-b]thiazol-3-one | 0.039 | 0.482 | 92 |
| 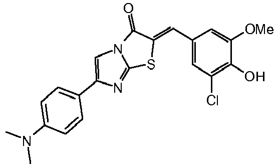 | 2-[1-(3-Chloro-4-hydroxy-5-methoxy-phenyl)-meth-(Z)-ylidene]-6-(4-(dimethylamino)phenyl)-imidazo[2,1-b]thiazol-3-one | 0.030 | 0.029 | 93 |
| 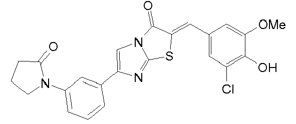 | 2-[1-(3-Chloro-4-hydroxy-5-methoxy-phenyl)-meth-(Z)-ylidene]-6-[3-(2-oxo-pyrro lidin-1-yl)-phenyl]-imidazo[2,1-b]thiazol-3-one | 0.042 | 3.892 | 94 |
| 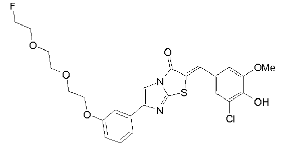 | 2-[1-(3-Chloro-4-hydroxy-5-methoxy-phenyl)-meth-(Z)-ylidene]-6-(3-{2-[2-(2-fluoro-ethoxy)-ethoxy]-ethoxy}-phenyl)-imidazo[2,1-b]thiazol-3-one | 0.028 | 2.147 | 95 |
| 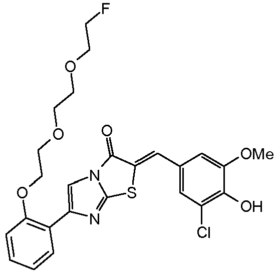 | 2-[1-(3-Chloro-4-hydroxy-5-methoxy-phenyl)-meth-(Z)-ylidene]-6-(2-{2-[2-(2-fluoro-ethoxy)-ethoxy]-ethoxy}-phenyl)-imidazo[2,1-b]thiazol-3-one | 0.102 | | 96 |
| 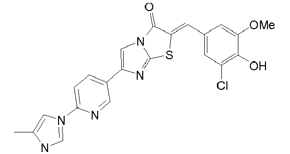 | 2-[1-(3-Chloro-4-hydroxy-5-methoxy-phenyl)-meth-(Z)-ylidene]-6-[6-(4-methyl-3H-imidazol-1-yl)-pyridin-3-yl]-imidazo[2,1-b]thiazol-3-one | 0.0017 | 0.185 | 97 |
| 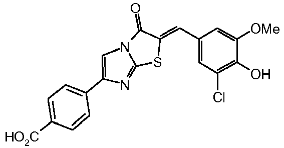 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid | 0.021 | 1.846 | 98 |
| 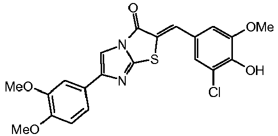 | 2-[1-(3-Chloro-4-hydroxy-5-methoxy-phenyl)-meth-(Z)-ylidene]-6-(3,4-dimethoxy-phenyl)-imidazo[2,1-b]thiazol-3-one | 0.014 | 0.325 | 99 |
| 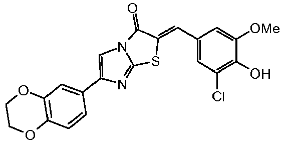 | 2-[1-(3-Chloro-4-hydroxy-5-methoxy-phenyl)-meth-(Z)-ylidene]-6-(2,3-dihydro-benzo[1,4]dioxin-6-yl)-imidazo[2,1-b]thiazol-3-one | 0.009 | 1.061 | 100 |
| 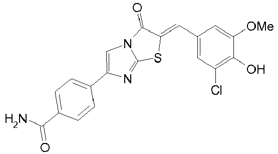 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide | 0.002 | 0.792 | 101 |
| 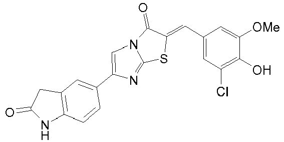 | 5-{2-[1-(3-Chloro-4-hydroxy-5-methoxy-phenyl)-meth-(Z)-ylidene]-3-oxo-2,3-dihydro-imidazo[2,1-b]thiazol-6-yl}-1,3-dihydro-indol-2-one | 0.012 | 0.226 | 102 |
| 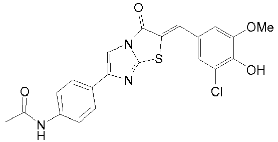 | N-(4-{2-[1-(3-Chloro-4-hydroxy-5-methoxy-phenyl)-meth-(Z)-ylidene]-3-oxo-2,3-dihydro-imidazo[2,1-b]thiazol-6-yl} -phenyl)-acetamide | 0.024 | 1.165 | 103 |
| 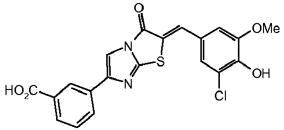 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid | 0.019 | >10 | 104 |
| 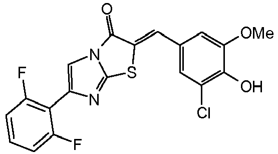 | 2-[1-(3-Chloro-4-hydroxy-5-methoxy-phenyl)-meth-(Z)-ylidene]-6-(2,6-difluoro-phenyl)-imidazo[2,1-b]thiazol-3-one | 0.013 | 5.551 | 105 |
| 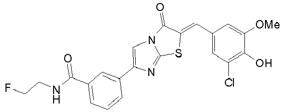 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide | 0.008 | 1.080 | 106 |
| 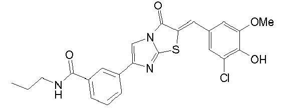 | 3-{2-[-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide | 0.003 | 2.630 | 107 |
| 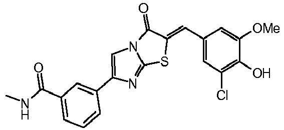 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide | 0.004 | 0.395 | 108 |
| 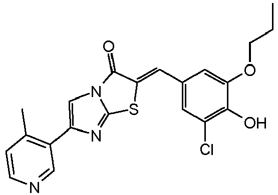 | 2-[1-(3-Chloro-4-hydroxy-5-propoxy-phenyl)-meth-(Z)-ylidene]-6-(4-methyl-pyridin-3-yl)-imidazo[2,1-b]thiazol-3-one | 0.033 | 4.917 | 109 |
| 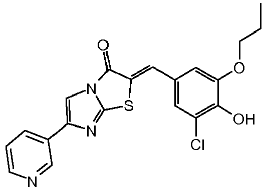 | 2-[1-(3-Chloro-4-hydroxy-5-propoxy-phenyl)-meth-(Z)-ylidene]-6-pyridin-3-yl-imidazo[2,1-b]thiazol-3-one | 0.017 | 0.425 | 110 |
| 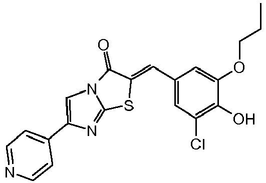 | 2-[1-(3-Chloro-4-hydroxy-5-propoxy-phenyl)-meth-(Z)-ylidene]-6-pyridin-4-yl-imidazo[2,1-b]thiazol-3-one | 0.017 | 1.449 | 111 |
| 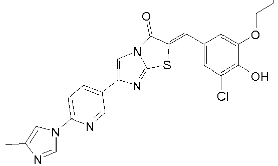 | 2-[1-(3-Chloro-4-hydroxy-5-propoxy-phenyl)-meth-(Z)-ylidene]-6-[6-(4-methyl-imidazol-1-yl)-pyridin-3-yl]-imidazo[2,1-b]thiazol-3-one | 0.008 | 0.493 | 112 |
| 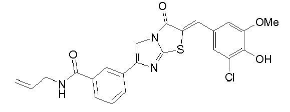 | N-Allyl-3-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide | 0.016 | 1.164 | 113 |
| 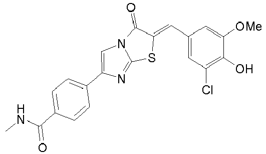 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide | 0.006 | 0.352 | 114 |
| 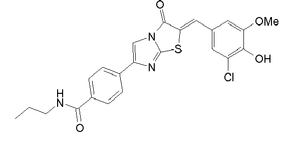 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide | 0.002 | 0.356 | 115 |
| 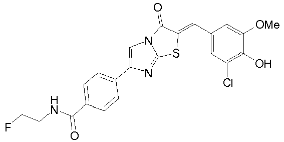 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide | 0.003 | 0.820 | 116 |
| 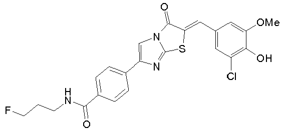 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoro-propyl)-benzamide | 0.005 | 0.643 | 117 |
| 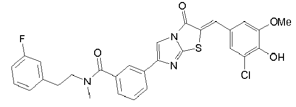 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(3-fluoro-phenyl)-ethyl]-N-methyl-benzamide | 0.0129 | >10 | 118 |
| 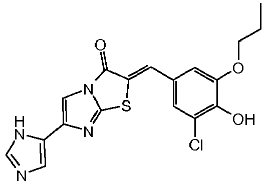 | 2-[1-(3-Chloro-4-hydroxy-5-propoxy-phenyl)-meth-(Z)-ylidene]-6-(3H-imidazol-4-yl)-imidazo[2,1-b]thiazo1-3-one | 0.060 | 3.117 | 119 |
| 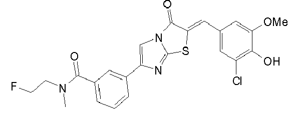 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide | 0.049 | | 120 |
| 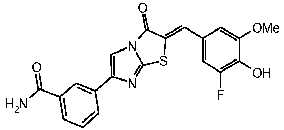 | 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzamide | 0.019 | 0.591 | 121 |
| 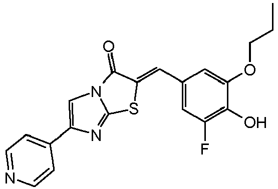 | 2-[1-(3-Fluoro-4-hydroxy-5-propoxy-phenyl)-meth-(Z)-ylidene]-6-pyridin-4-yl-imidazo[2,1-b]thiazol-3-one | 0.0235 | 2.673 | 122 |
| 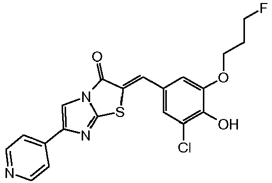 | 2-[1-(3-Chloro-5-(3-fluoropropoxy)-4-hydroxyphenyl)-meth-(Z)-ylidene]-6-pyridin-4-yl-imidazo[2,1-b]thiazol-3-one | 0.012 | 1.061 | 123 |
| 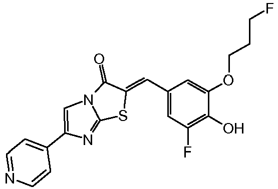 | 2-[1-(3-Fluoro-5-(3-fluoropropoxy)-4-hydroxyphenyl)-meth-(Z)-ylidene]-6-pyridin-4-yl-imidazo[2,1-b]thiazol-3-one | 0.017 | 1.225 | 124 |
| 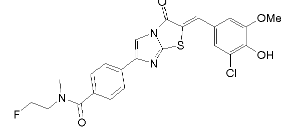 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide | 0.017 | 0.621 | 125 |
| 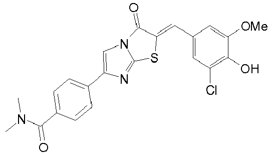 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N,N-dimethyl-benzamide | 0.028 | 0.550 | 126 |
| 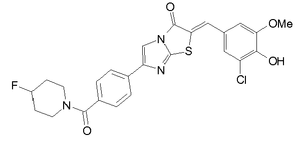 | 2-[1-(3-Chloro-4-hydroxy-5-methoxy-phenyl)-meth-(Z)-ylidene]-6-[4-(4-fluoro-piperidine-1-carbonyl)-phenyl]-imidazo[2,1-b]thiazol-3-one | 0.006 | 0.118 | 127 |
| 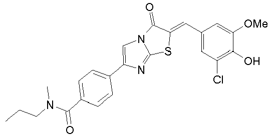 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-N-propyl-benzamide | 0.009 | 0.218 | 128 |
| 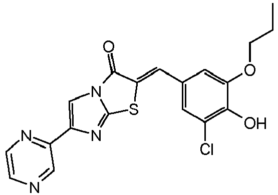 | 2-[1-(3-Chloro-4-hydroxy-5-propoxy-phenyl)-meth-(Z)-ylidene]-6-pyrazin-2-yl-imidazo[2,1-b]thiazol-3-one | 0.064 | 2.216 | 129 |
| 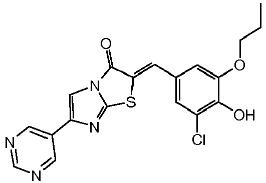 | 2-[1-(3-Chloro-4-hydroxy-5-propoxy-phenyl)-meth-(Z)-ylidene]-6-pyrimidin-5-yl-imidazo[2,1-b]thiazol-3-one | 0.067 | 1.945 | 130 |
| 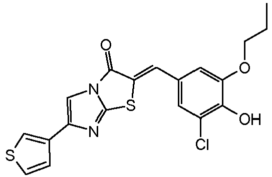 | 2-[1-(3-Chloro-4-hydroxy-5-propoxy-phenyl)-meth-(Z)-ylidene]-6-thiophen-3-yl-imidazo[2,1-b]thiazol-3-one | 0.026 | 0.979 | 131 |
| 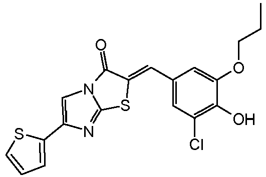 | 2-[1-(3-Chloro-4-hydroxy-5-propoxy-phenyl)-meth-(Z)-ylidene]-6-thiophen-2-yl-imidazo[2,1-b]thiazol-3-one | 0.041 | 0.718 | 132 |
| 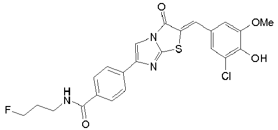 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-benzamide | 0.002 | 0.086 | 133 |
| 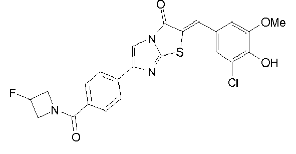 | 2-[1-(3-Chloro-4-hydroxy-5-methoxy-phenyl)-meth-(Z)-ylidene]-6-[4-(3-fluoro-azetidine-1-carbonyl)-phenyl]-imidazo[2,1-b]thiazol-3-one | 0.002 | 0.133 | 134 |
| 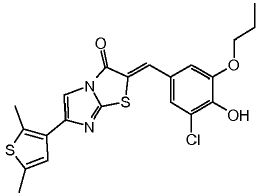 | 2-[1-(3-Chloro-4-hydroxy-5-propoxy-phenyl)-meth-(Z)-ylidene]-6-(2,4-dimethylthiophen-3-yl)-imidazo[2,1-b]thiazol-3-one | 0.043 | 1.058 | 135 |
| 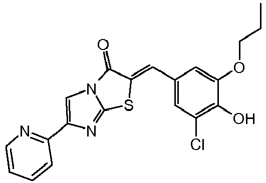 | 2-[1-(3-Chloro-4-hydroxy-5-propoxy-phenyl)-meth-(Z)-ylidene]-6-pyridin-2-yl-imidazo[2,1-b]thiazol-3-one | 0.030 | 0.293 | 136 |
| 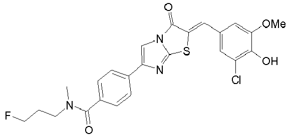 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide | 0.007 | 0.332 | 137 |
| 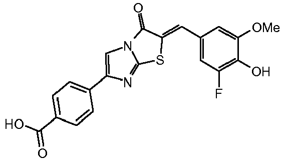 | 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzoic acid | 0.014 | 0.898 | 138 |
| 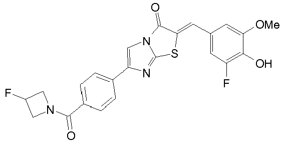 | 6-[4-(3-Fluoro-azetidine-1-carbonyl)-phenyl]-2-[1-(3-fluoro-4-hydroxy-5-methoxy-phenyl)-meth-(Z)-ylidene]-imidazo[2,1-b]thiazol-3-one | | | 139 |
| 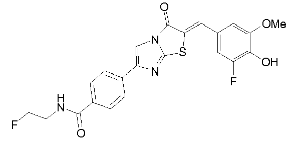 | 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-benzamide | | | 140 |
| 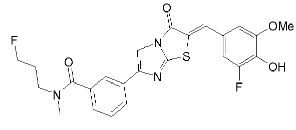 | 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide | | | 141 |
| 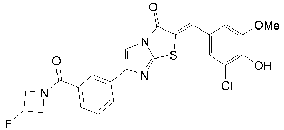 | 2-[1-(3-Chloro-4-hydroxy-5-methoxy-phenyl)-meth-(Z)-ylidene]-6-[3-(3-fluoro-azetidine-1-carbonyl)-phenyl]-imidazo[2,1-b]thiazol-3-one | | | 142 |
| 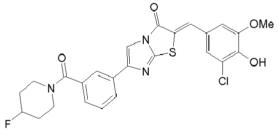 | 2-[1-(3-Chloro-4-hydroxy-5-methoxy-phenyl)-meth-(Z)-ylidene]-6-[3-(4-fluoro-piperidine-1-carbon-phenyl]-imidazo[2,1-b]thiazol-3-one | | | 143 |
| 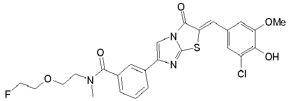 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide | | | 144 |
| 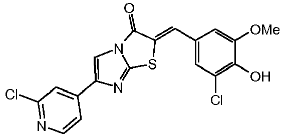 | 2-[1-(3-Chloro-4-hydroxy-5-methoxy-phenyl)-meth-(Z)-ylidene]-6-(2-chloro-pyridin-4-yl)-imidazo [2,1-b]thiazol-3-one | | | 145 |
| 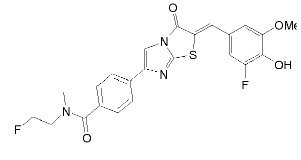 | 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-N-methyl-benzamide | 0.048 | 1.010 | 146 |
| 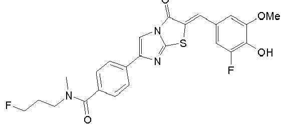 | 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoropropoxy)-N-methyl-benzamide | 0.041 | 0.448 | 147 |
| 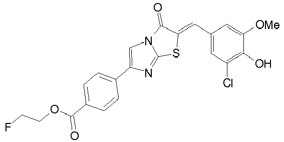 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 2-fluoroethyl ester | 0.004 | 0.038 | 148 |
| 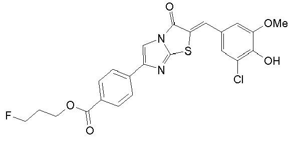 | 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 3-fluoropropyl ester | 0.002 | 0.168 | 149 |
| 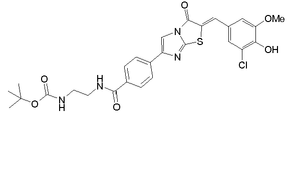 | [2-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoylamino)-ethyl]-carbamic acid tert-butyl ester | 0.0018 | 0.118 | 150 |
| 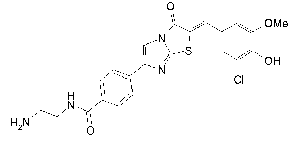 | N-(2-Amino-ethyl)-4- {2-[1-(3-chloro-4-hydroxy-5-methoxy-phenyl)-meth-(Z)-ylidene]-3-oxo-2,3-dihydro-imidazo [2,1-b]thiazol-6-yl}-benzamide | 0.012 | 0.024 | 151 |
| 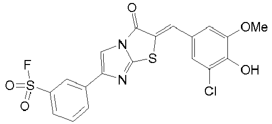 | 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzenesulfonyl fluoride | 0.007 | 0.019 | 152 |
| 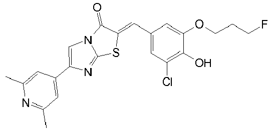 | 2-[1-[3-Chloro-5-(3-fluoro-propoxy)-4-hydroxy-phenyl]-meth-(Z)-ylidene]-6-(2,6-dimethyl-pyridin-4-yl)-imidazo[2,1-b]thiazol-3-one | 0.046 | 0.605 | 153 |
| 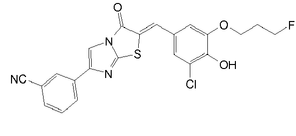 | 3-{2-[1-[3-Chloro-5-(3-fluoro-propoxy)-4-hydroxy-phenyl]-meth-(Z)-ylidene]-3-oxo-2,3-dihydro-imidazo[2,1-b]thiazol-6-yl}-benzonitrile | | | 154 |

### Example 1

### 2-[1-(4-Hydroxy-3,5-dimethoxy-phenyl)-meth-(Z)-ylidene]-6-pyridin-4-yl-imidazo[2,1-b]thiazol-3-one

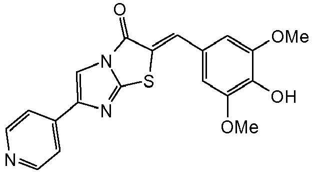

To a mixture of 5-(pyridin-4-yl)-1H-imidazole-2-thiol (70 mg, 395 µmol), 4-hydroxy-3,5-dimethoxybenzaldehyde (72.0 mg, 395 µmol), chloroacetic acid (37.3 mg, 26.6 µl, 395 µmol) and sodium acetate (64.8 mg, 790 µmol) was added acetic acid (1.5 mL). The resulting suspension was warmed to 130 °C and stirred overnight at this temperature in a sealed tube. After cooling to ambient temperature water (2 mL) was added and the reaction mixture was centrifuged. The supernatant was removed and the residue washed with water (2 mL). Suspension in boiling ethanol (2 mL) was followed by filtering. Washing of the remaining solid with ethanol (2 mL) afforded the title compound (20 mg, 13 %) as a yellow solid. MS m/e: 382.1 [M+H]⁺

### Example 2

### 6-(4-Chloro-phenyl)-2-[1-(4-hydroxy-3,5-dimethoxy-phenyl)-meth-(Z)-ylidene]-imidazo[2,1-b]thiazol-3-one

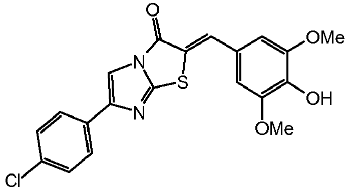

In analogy to the experimental procedure of example 1) 5-(4-chlorophenyl)-1H-imidazole-2-thiol instead of 5-(pyridin-4-yl)-1H-imidazole-2-thiol was converted into the title compound (23 mg,17 %) which was obtained as a yellow solid. MS m/e: 415.1 [M+H]⁺

### Example 3

### 6-(4-Chloro-phenyl)-2-[1-(4-hydroxy-3-methoxy-phenyl)-meth-(Z)-ylidene]-imidazo[2,1-b]thiazol-3-one

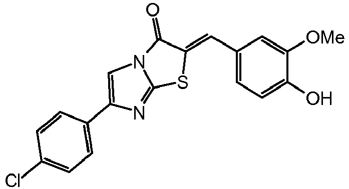

In analogy to the experimental procedure of example 1) 5-(4-chlorophenyl)-1H-imidazole-2-thiol was converted using 4-hydroxy-3-methoxybenzaldehyde instead of 4-hydroxy-3,5-dimethoxybenzaldehyde into the title compound (82 mg, 45 %) which was obtained as a light yellow solid. MS m/e: 385.0 [M+H]⁺

### Example 4

### 6-(4-Chloro-phenyl)-2-[1-(3-fluoro-4-hydroxy-5-methoxy-phenyl)-meth-(Z)-ylidene]-imidazo[2,1-b]thiazol-3-one

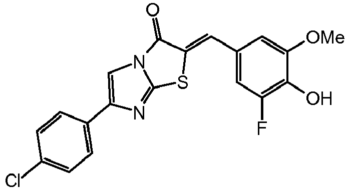

In analogy to the experimental procedure of example 1) 5-(4-chlorophenyl)-1H-imidazole-2-thiol was converted using 3-fluoro-4-hydroxy-5-methoxybenzaldehyde instead of 4-hydroxy-3,5-dimethoxybenzaldehyde into the title compound (64 mg, 34 %) which was obtained as a light yellow solid. MS m/e: 403.0 [M+H]⁺

### Example 5

### 6-(4-Chloro-phenyl)-2-[1-(3-ethoxy-4-hydroxy-phenyl)-meth-(Z)-ylidene]-imidazo[2,1-b]thiazol-3-one

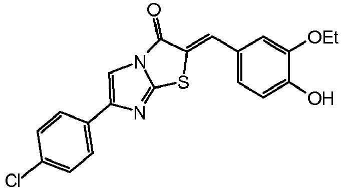

In analogy to the experimental procedure of example 1) 5-(4-chlorophenyl)-1H-imidazole-2-thiol was converted using 3-ethoxy-4-hydroxy-benzaldehyde instead of 4-hydroxy-3,5-dimethoxybenzaldehyde into the title compound (58 mg, 31 %) which was obtained as a light yellow solid. MS m/e: 399.1 [M+H]⁺

### Example 6

### 6-(4-Chloro-phenyl)-2-[1-(3-chloro-4-hydroxy-5-methoxy-phenyl)-meth-(Z)-ylidene]-imidazo[2,1-b]thiazol-3-one

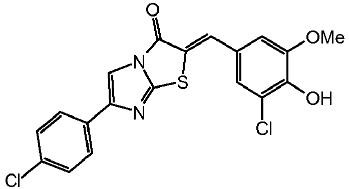

In analogy to the experimental procedure of example 1) 5-(4-chlorophenyl)-1H-imidazole-2-thiol was converted using 3-chloro-4-hydroxy-5-methoxybenzaldehyde instead of 4-hydroxy-3,5-dimethoxybenzaldehyde into the title compound (75 mg, 38 %) which was obtained as a light brown solid. MS m/e: 419.0 [M+H]⁺

### Example 7

### 6-(4-Chloro-phenyl)-2-[1-(3-bromo-4-hydroxy-5-methoxy-phenyl)-meth-(Z)-ylidene]-imidazo[2,1-b]thiazol-3-one

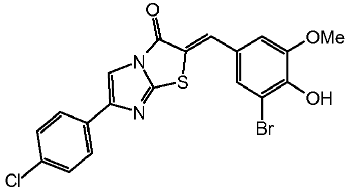

In analogy to the experimental procedure of example 1) 5-(4-chlorophenyl)-1H-imidazole-2-thiol was converted using 3-bromo-4-hydroxy-5-methoxybenzaldehyde instead of 4-hydroxy-3,5-dimethoxybenzaldehyde into the title compound (75 mg, 34 %) which was obtained as a light brown solid. MS m/e: 465.1 [M+H]⁺

### Example 8

### 6-(4-Chloro-phenyl)-2-[1-(3,4-dihydroxy-5-methoxy-phenyl)-meth-(Z)-ylidene]-imidazo[2,1-b]thiazol-3-one

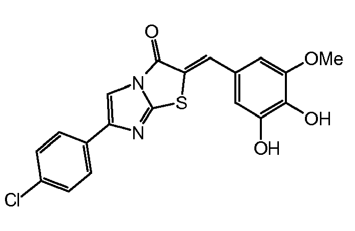

In analogy to the experimental procedure of example 1) 5-(4-chlorophenyl)-1H-imidazole-2-thiol was converted using 3,4-dihydroxy-5-methoxybenzaldehyde instead of 4-hydroxy-3,5-dimethoxybenzaldehyde into the title compound (135 mg, 71 %) which was obtained as a light brown solid. MS m/e: 401.0 [M+H]⁺

### Example 9

### 2-[1-(3-Fluoro-4-hydroxy-5-methoxy-phenyl)-meth-(Z)-ylidene]-6-o-tolyl-imidazo[2,1-b]thiazol-3-one

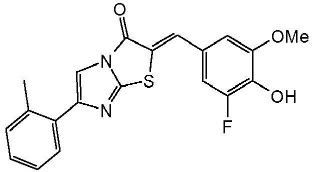

To a suspension of 4-o-tolyl-1H-imidazole-2(3H)-thione (100 mg, 526 µmol) and N,N-diisopropylethylamine (102 mg, 138 µl, 788 µmol) in dioxane (1.5 mL) was added at 10 °C dropwise chloroacetyl chloride (71.2 mg, 50.5 µl, 631 µmol). After stirring for 2 min 3-fluoro-4-hydroxy-5-methoxybenzaldehyde (93.9 mg, 552 µmol) was added and the reaction mixture stirred for 18 h at 110 °C After cooling to ambient temperature water (3 mL) was added and stirred for 15 min. The suspension was centrifuged and the upper layer was pipetted off and the solid was washed with water (2 mL). After the addition of ethanol (3 mL) it was stirred for 1 h at 80 °C. It was centrifuged and the upper layer was pipetted off. The residue was washed twice with ethanol (2 mL) affording the title compound (65 mg, 32 %) as a light brown solid. MS m/e: 383.2 [M+H]⁺

### Example 10

### 2-[1-(3-Chloro-4-hydroxy-5-methoxy-phenyl)-meth-(Z)-ylidene]-6-o-tolyl-imidazo[2,1-b]thiazol-3-one

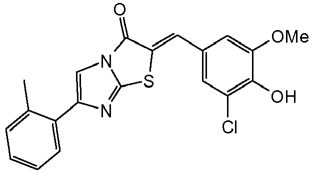

In analogy to the experimental procedure of example 9) 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (86 mg, 41 %) which was obtained as a light brown solid. MS m/e: 399.1 [M+H]⁺

### Example 11

### 2-[1-(4-hydroxy-3-methoxy-phenyl)-meth-(Z)-ylidene]-6-o-tolyl-imidazo[2,1-b]thiazol-3-one

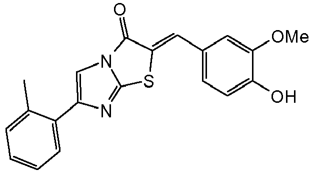

In analogy to the experimental procedure of example 9) 4-o-tolyl-1H-imidazole-2(3H)-thione was converted using 4-hydroxy-3-methoxybenzaldehyde instead of 3-fluoro-4-hydroxy-5-methoxybenzaldehyde into the title compound (94 mg, 49 %) which was obtained as a yellow solid. MS m/e: 365.1 [M+H]⁺

### Example 12

### 2-[1-(4-Hydroxy-3-methoxy-phenyl)-meth-(Z)-ylidene]-6-(2-methyl-pyridin-4-yl)-imidazo[2,1-b]thiazol-3-one

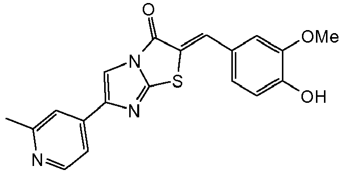

In analogy to the experimental procedure of example 9) 4-(2-methylpyridin-4-yl)-1H-imidazole-2(3H)-thione instead of 4-o-tolyl-1H-imidazole-2(3H)-thione was converted using 4-hydroxy-3-methoxybenzaldehyde instead of 3-fluoro-4-hydroxy-5-methoxybenzaldehyde into the title compound (22 mg, 8 %) which was obtained as a light brown solid. MS m/e: 364.1 [M+H]⁺

### Example 13

### 2-[1-(4-Hydroxy-3-methoxy-phenyl)-meth-(Z)-ylidene]-6-(2,6-dimethyl-pyridin-4-yl)-imidazo[2,1-b]thiazol-3-one

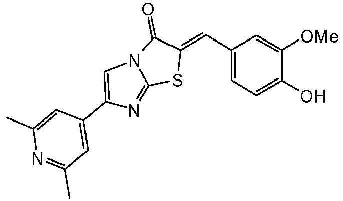

In analogy to the experimental procedure of example 9) 4-(2,6-dimethylpyridin-4-yl)-1H-imidazole-2(3H)-thione instead of 4-o-tolyl-1H-imidazole-2(3H)-thione was converted using 4-hydroxy-3-methoxybenzaldehyde instead of 3-fluoro-4-hydroxy-5-methoxybenzaldehyde into the title compound (5 mg, 3 %) which was obtained as a brown solid. MS m/e: 380.1 [M+H]⁺

### Example 14

### 2-[1-(3-Chloro-4-hydroxy-5-methoxy-phenyl)-meth-(Z)-ylidene]-6-(4-methoxy-phenyl)-5-methyl-imidazo[2,1-b]thiazol-3-one

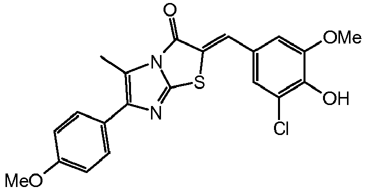

In analogy to the experimental procedure of example 9) 4-(4-methoxyphenyl)-5-methyl-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (46 mg, 24 %) which was obtained as a brown solid. MS m/e: 429.1 [M+H]⁺

### Example 15

### 2-[1-(3-Fluoro-4-hydroxy-5-methoxy-phenyl)-meth-(Z)-ylidene]-6-(4-methoxy-phenyl)-5-methyl-imidazo[2,1-b]thiazol-3-one

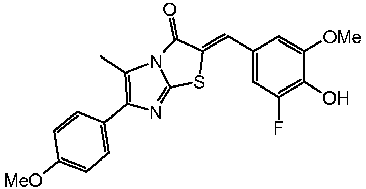

In analogy to the experimental procedure of example 9) 4-(4-methoxyphenyl)-5-methyl-1H-imidazole-2(3H)-thione instead of 4-o-tolyl-1H-imidazole-2(3H)-thione was converted into the title compound (35 mg, 19 %) which was obtained as a brown solid. MS m/e: 413.1 [M+H]⁺

### Example 16

### 2-[1-(4-Hydroxy-3,5-dimethoxy-phenyl)-meth-(Z)-ylidene]-6-(2-methyl-pyridin-4-yl)-imidazo[2,1-b]thiazol-3-one

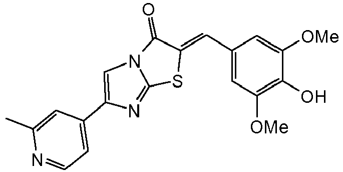

In analogy to the experimental procedure of example 9) 4-(2-methylpyridin-4-yl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (25 mg, 17 %) which was obtained as an orange solid. MS m/e: 396.1 [M+H]⁺

### Example 17

### 2-[1-(4-Hydroxy-3,5-dimethoxy-phenyl)-meth-(Z)-ylidene]-6-(2,6-dimethyl-pyridin-4-yl)-imidazo[2,1-b]thiazol-3-one

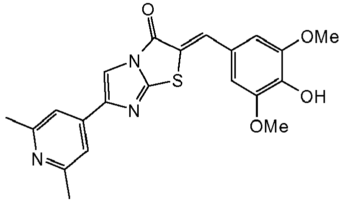

In analogy to the experimental procedure of example 9) 4-(2,6-dimethylpyridin-4-yl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (14 mg, 7 %) which was obtained as a red solid. MS m/e: 410.1 [M+H]⁺

### Example 18

### 2-[1-(4-Hydroxy-3,5-dimethoxy-phenyl)-meth-(Z)-ylidene]-6-(3-trifluoromethoxy-phenyl)-imidazo[2,1-b]thiazol-3-one

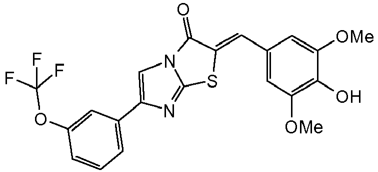

In analogy to the experimental procedure of example 9) 4-(3-(trifluoromethoxy)phenyl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (35 mg, 20 %) which was obtained as a yellow solid. MS m/e: 465.1 [M+H]⁺

### Example 19

### 2-[1-(4-Hydroxy-3,5-dimethoxy-phenyl)-meth-(Z)-ylidene]-6-(3-trifluoromethyl-phenyl)-imidazo[2,1-b]thiazol-3-one

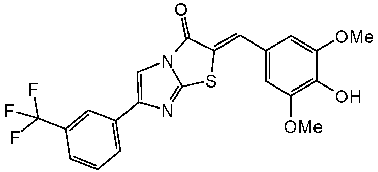

In analogy to the experimental procedure of example 9) 4-(3-(trifluoromethyl)phenyl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (16 mg, 9 %) which was obtained as an orange solid. MS m/e: 449.1 [M+H]⁺

### Example 20

### 2-[1-(4-Hydroxy-3,5-dimethoxy-phenyl)-meth-(Z)-ylidene]-6-m-tolyl-imidazo[2,1-b]thiazol-3-one

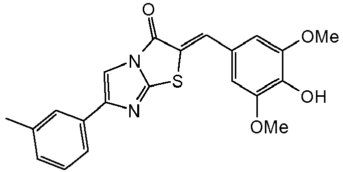

In analogy to the experimental procedure of example 9) 4-m-tolyl-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (50 mg, 28 %) which was obtained as a yellow solid. MS m/e: 395.1 [M+H]⁺

### Example 21

### 6-(3-Chloro-phenyl)-2-[1-(4-hydroxy-3,5-dimethoxy-phenyl)-meth-(Z)-ylidene]-imidazo[2,1-b]thiazol-3-one

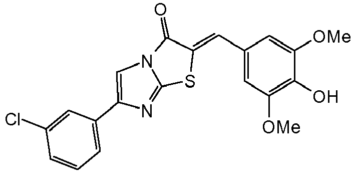

In analogy to the experimental procedure of example 9) 4-(3-chlorophenyl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (60 mg, 31 %) which was obtained as a yellow solid. MS m/e: 415.1 [M+H]⁺

### Example 22

### 2-[1-(3-Chloro-4-hydroxy-5-methoxy-phenyl)-meth-(Z)-ylidene]-6-(3-trifluoromethoxy-phenyl)-imidazo[2,1-b]thiazol-3-one

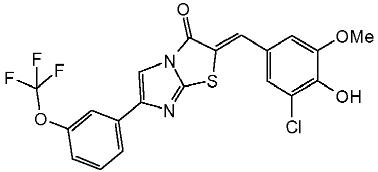

In analogy to the experimental procedure of example 9) 4-(3-(trifluoromethoxy)phenyl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (19 mg, 11 %) which was obtained as a orange solid. MS m/e: 469.1 [M+H]⁺

### Example 23

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide

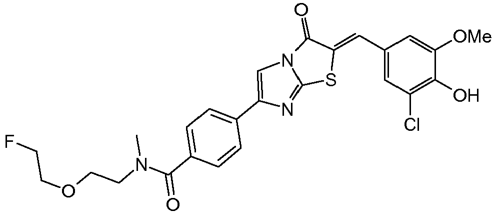

In analogy to the experimental procedure of example 105) of (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 2-(2-fluoroethoxy)-N-methylethanamine hydrochloride hydrochloride instead of 2-fluoroethylamine hydrochloride into the title compound (33 mg, 24 %) which was obtained as a yellow solid. MS m/e: 532.1 [M+H]⁺

### Example 24

### 2-[1-(3-Chloro-4-hydroxy-5-methoxy-phenyl)-meth-(Z)-ylidene]-6-(3-trifluoromethyl-phenyl)-imidazo[2,1-b]thiazol-3-one

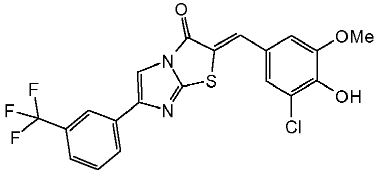

In analogy to the experimental procedure of example 9) 4-(3-(trifluoromethyl)phenyl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (29 mg, 16 %) which was obtained as a yellow solid. MS m/e: 453.1 [M+H]⁺

### Example 25

### 6-(3-Chloro-phenyl)-2-[1-(3-chloro-4-hydroxy-5-methoxy-phenyl)-meth-(Z)-ylidene]-imidazo[2,1-b]thiazol-3-one

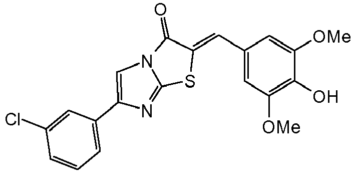

In analogy to the experimental procedure of example 9) 4-(3-chlorophenyl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (61 mg, 31 %) which was obtained as a yellow solid. MS m/e: 419.1 [M+H]⁺

### Example 26

### 6-(4-Chloro-phenyl)-2-[1-(3-ethoxy-4-hydroxy-5-methoxy-phenyl)-meth-(Z)-ylidene]-imidazo[2,1-b]thiazol-3-one

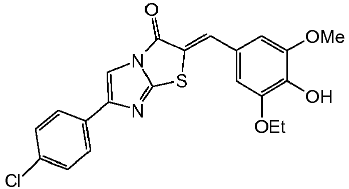

In analogy to the experimental procedure of example 9) 4-(4-chlorophenyl)-1H-imidazole-2(3H)-thione instead of 4-o-tolyl-1H-imidazole-2(3H)-thione was converted using 3-ethoxy-4-hydroxy-5-methoxybenzaldehyde instead of 3-fluoro-4-hydroxy-5-methoxybenzaldehyde into the title compound (58 mg, 29 %) which was obtained as a yellow solid. MS m/e: 429.1 [M+H]⁺

### Example 27

### 6-(4-Chloro-phenyl)-2-[1-(3-(2-fluoro-ethoxy)-4-hydroxy-5-methoxy-phenyl)-meth-(Z)-ylidene]-imidazo[2,1-b]thiazol-3-one

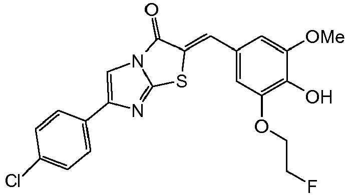

In analogy to the experimental procedure of example 9) 4-(4-chlorophenyl)-1H-imidazole-2(3H)-thione instead of 4-o-tolyl-1H-imidazole-2(3H)-thione was converted using 3-(2-fluoro-ethoxy)-4-hydroxy-5-methoxybenzaldehyde instead of 3-fluoro-4-hydroxy-5-methoxybenzaldehyde into the title compound (71 mg, 34 %) which was obtained as a yellow solid. MS m/e: 447.1 [M+H]⁺

### Example 28

### 2-[1-(3-Chloro-4-hydroxy-5-methoxy-phenyl)-meth-(Z)-ylidene]-6-(3-fluoro-4-trifluoromethyl-phenyl)-imidazo[2,1-b]thiazol-3-one

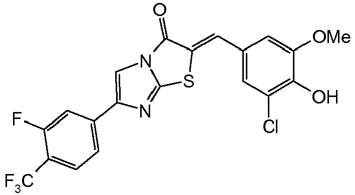

In analogy to the experimental procedure of example 9) 4-(3-fluoro-4-trifluoromethylphenyl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (62 mg, 35 %) which was obtained as an orange solid. MS m/e: 469.1 [M+H]⁺

### Example 29

### 2-[1-(3-Hydroxy-3,5-dimethoxy-phenyl)-meth-(Z)-ylidene]-6-(3-fluoro-4-trifluoromethyl-phenyl)-imidazo[2,1-b]thiazol-3-one

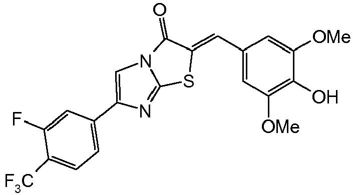

In analogy to the experimental procedure of example 9) 4-(3-fluoro-4-trifluoromethyl-phenyl)-1H-imidazole-2(3H)-thione instead of 4-o-tolyl-1H-imidazole-2(3H)-thione was converted using 4-hydroxy-3,5-dimethoxybenzaldehyde instead of 3-fluoro-4-hydroxy-5-methoxybenzaldehyde into the title compound (50 mg, 28 %) which was obtained as a yellow solid. MS m/e: 467.1 [M+H]⁺

### Example 30

### 6-(4-Chloro-phenyl)-2-[1-(4-hydroxy-3-isopropoxy-5-methoxy-phenyl)-meth-(Z)-ylidene]-imidazo[2,1-b]thiazol-3-one

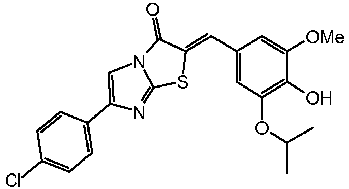

In analogy to the experimental procedure of example 9) 4-(4-chloro-phenyl)-1H-imidazole-2(3H)-thione instead of 4-o-tolyl-1H-imidazole-2(3H)-thione was converted using 4-hydroxy-3-isopropoxy-5-methoxybenzaldehyde instead of 3-fluoro-4-hydroxy-5-methoxybenzaldehyde into the title compound (37 mg, 18 %) which was obtained as a yellow solid. MS m/e: 443.1 [M+H]⁺

### Example 31

### 6-(4-Chloro-phenyl)-2-[1-(4-hydroxy-3-methoxy-5-propoxy-phenyl)-meth-(Z)-ylidene]-imidazo[2,1-b]thiazol-3-one

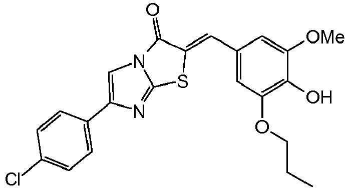

In analogy to the experimental procedure of example 9) 4-(4-chloro-phenyl)-1H-imidazole-2(3H)-thione instead of 4-o-tolyl-1H-imidazole-2(3H)-thione was converted using 4-hydroxy-3-methoxy-5-propoxybenzaldehyde instead of 3-fluoro-4-hydroxy-5-methoxybenzaldehyde into the title compound (45 mg, 21 %) which was obtained as a yellow solid. MS m/e: 443.1 [M+H]⁺

### Example 32

### 6-(4-Chloro-phenyl)-2-[1-(4-hydroxy-3-methoxy-5-methyl-phenyl)-meth-(Z)-ylidene]-imidazo[2,1-b]thiazol-3-one

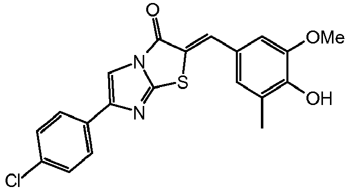

In analogy to the experimental procedure of example 1) 5-(4-chlorophenyl)-1H-imidazole-2-thiol instead of 5-(pyridin-4-yl)-1H-imidazole-2-thiol was converted using 4-hydroxy-3-methoxy-5-methylbenzaldehyde instead of 4-hydroxy-3,5-dimethoxybenzaldehyde into the title compound (31 mg, 26 %) which was obtained as a yellow solid. MS m/e: 399.1 [M+H]⁺

### Example 33

### 2-[1-(4-Hydroxy-3-methoxy-5-propoxy-phenyl)-meth-(Z)-ylidene]-6-(3-trifluoromethoxy-phenyl)-imidazo[2,1-b] thiazol-3-one

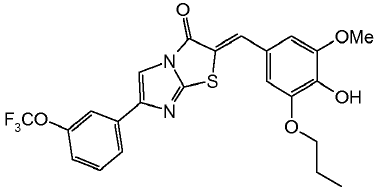

In analogy to the experimental procedure of example 1) 5-(3-trifluoromethoxy-phenyl)-1H-imidazole-2-thiol instead of 5-(pyridin-4-yl)-1H-imidazole-2-thiol was converted using 4-hydroxy-3-methoxy-5-propoxybenzaldehyde instead of 4-hydroxy-3,5-dimethoxybenzaldehyde into the title compound (65 mg, 34 %) which was obtained as a yellow solid. MS m/e: 493.1 [M+H]⁺

### Example 34

### 2-[1-(4-Hydroxy-3-methoxy-5-isopropoxy-phenyl)-meth-(Z)-ylidene]-6-(3-trifluoromethoxy-phenyl)-imidazo[2,1-b]thiazol-3-one

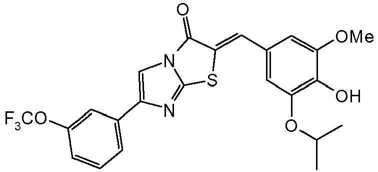

In analogy to the experimental procedure of example 1) 5-(3-trifluoromethoxy-phenyl)-1H-imidazole-2-thiol instead of 5-(pyridin-4-yl)-1H-imidazole-2-thiol was converted using 4-hydroxy-3-methoxy-5-isopropoxybenzaldehyde instead of 4-hydroxy-3,5-dimethoxybenzaldehyde into the title compound (29 mg, 15 %) which was obtained as a yellow solid. MS m/e: 493.1 [M+H]⁺

### Example 35

### 2-[1-(3-Fluoro-4-hydroxy-5-methoxy-phenyl)-meth-(Z)-ylidene]-6-(3-trifluoromethoxy-phenyl)-imidazo[2,1-b]thiazol-3-one

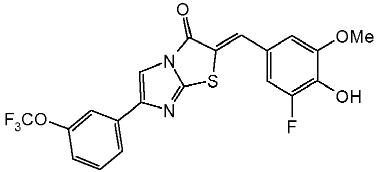

In analogy to the experimental procedure of example 1) 5-(3-trifluoromethoxy-phenyl)-1H-imidazole-2-thiol instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (39 mg, 22 %) which was obtained as a yellow solid. MS m/e: 453.1 [M+H]⁺

### Example 36

### 2-[1-(4-Hydroxy-3-methoxy-phenyl)-meth-(Z)-ylidene]-6-(3-trifluoromethoxy-phenyl)-imidazo[2,1-b]thiazol-3-one

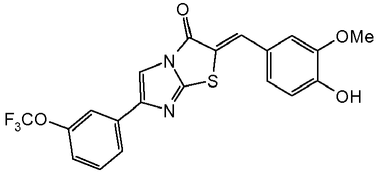

In analogy to the experimental procedure of example 1) 5-(3-trifluoromethoxy-phenyl)-1H-imidazole-2-thiol instead of 5-(pyridin-4-yl)-1H-imidazole-2-thiol was converted using 4-hydroxy-3-methoxybenzaldehyde instead of 4-hydroxy-3,5-dimethoxybenzaldehyde into the title compound (35 mg, 21 %) which was obtained as a yellow solid. MS m/e: 435.1 [M+H]⁺

### Example 37

### 2-[1-(3-Bromo-4-hydroxy-5-methoxy-phenyl)-meth-(Z)-ylidene]-6-(3-trifluoromethoxy-phenyl)-imidazo[2,1-b]thiazol-3-one

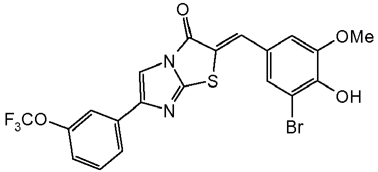

In analogy to the experimental procedure of example 1) 5-(3-trifluoromethoxy-phenyl)-1H-imidazole-2-thiol instead of 5-(pyridin-4-yl)-1H-imidazole-2-thiol was converted using 3-bromo-4-hydroxy-5-methoxybenzaldehyde instead of 4-hydroxy-3,5-dimethoxybenzaldehyde into the title compound (62 mg, 31%) which was obtained as a yellow solid. MS m/e: 514.9 [M+H]⁺

### Example 38

### 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile

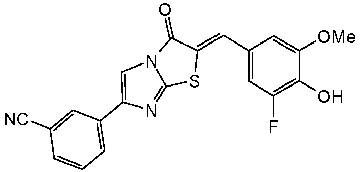

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (62 mg, 32 %) which was obtained as a yellow solid. MS m/e: 394.1 [M+H]⁺

### Example 39

### 2-[1-(3-fluoro-4-hydroxy-5-methoxy-phenyl)-meth-(Z)-ylidene]-6-(3-chloro-phenyl)-imidazo[2,1-b]thiazol-3-one

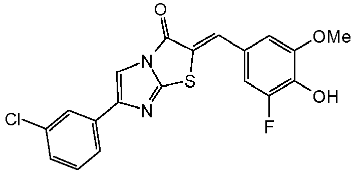

In analogy to the experimental procedure of example 1) 4-(3-chlorophenyl)-1H-imidazole-2(3H)-thione instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (58 mg, 30 %) which was obtained as a yellow solid. MS m/e: 403.0 [M+H]⁺

### Example 40

### 2-[1-(3-Ethyl-4-hydroxy-5-methoxy-phenyl)-meth-(Z)-ylidene]-6-(3-trifluoromethoxy-phenyl)-imidazo[2,1-b]thiazol-3-one

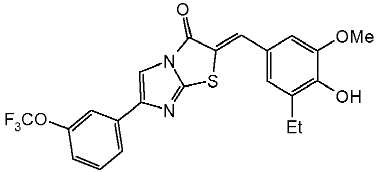

In analogy to the experimental procedure of example 1) 5-(3-trifluoromethoxy-phenyl)-1H-imidazole-2-thiol instead of 5-(pyridin-4-yl)-1H-imidazole-2-thiol was converted using 3-ethyl-4-hydroxy-5-methoxybenzaldehyde instead of 4-hydroxy-3,5-dimethoxybenzaldehyde into the title compound (64 mg, 36 %) which was obtained as a yellow solid. MS m/e: 463.1 [M+H]⁺

### Example 41

### 2-[1-(3-fluoro-4-hydroxy-5-methoxy-phenyl)-meth-(Z)-ylidene]-6-(3-(trifluoromethyl)-phenyl)-imidazo[2,1-b]thiazol-3-one

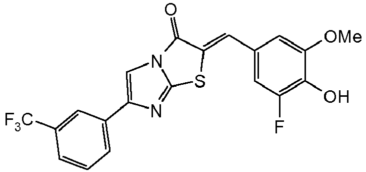

In analogy to the experimental procedure of example 1) 4-(3-(trifluoromethyl)phenyl)-1H-imidazole-2(3H)-thione instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (56 mg, 31 %) which was obtained as a yellow solid. MS m/e: 437.1 [M+H]⁺

### Example 42

### 2-[1-(3-fluoro-4-hydroxy-5-methoxy-phenyl)-meth-(Z)-ylidene]-6-(4-fluoro-3-(trifluoromethyl)-phenyl)-imidazo[2,1-b]thiazol-3-one

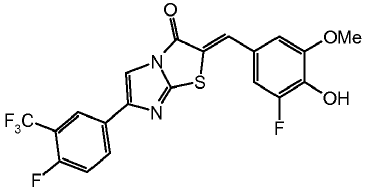

In analogy to the experimental procedure of example 1) 4-(4-fluoro-3-(trifluoromethyl)phenyl)-1H-imidazole-2(3H)-thione instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (45 mg, 19 %) which was obtained as a yellow solid. MS m/e: 455.1 [M+H]⁺

### Example 43

### 2-[1-(3-fluoro-4-hydroxy-5-methoxy-phenyl)-meth-(Z)-ylidene]-6-phenyl-imidazo[2,1-b]thiazol-3-one

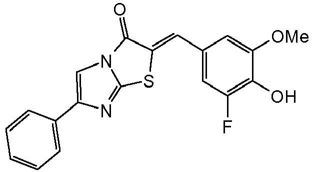

To a solution of acetophenone (19.7 mg, 19.1 µL, 164 µmol) in acetonitrile (1 mL) was added under an atmosphere of nitrogen [hydroxy(tosyloxy)iodo]benzene (64.3 mg, 164 µmol). The reaction mixture was stirred at 80 °C for 3 h. The resulting solution was concentrated in vacuo and a suspension of (Z)-2-amino-5-(3-fluoro-4-hydroxy-5-methoxybenzylidene)thiazol-4(5H)-one (44 mg, 164 µmol) in ethanol (3 mL) was added. The yellow suspension was stirred at 80 °C for 3 h. It was diluted with tetrahydrofurane (2 mL) and the resulting solution was stirred at 80 °C for 18 h. The reaction mixture was furthermore irradiated in the microwave at 140 °C for 60 min. At ambient temperature the solution was diluted with water (10 mL), centrifuged and the upper layer was pipetted off. Purification of the residue by flash chromatography (SiO₂ eluent: Heptane/dichloromethane/ methanol = 20:80:0 to 0:95:5) afforded the title compound (4 mg, 8 %) as a yellow solid. MS m/e: 369.07 [M+H]⁺

### Example 44

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile

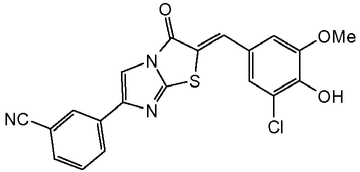

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted using 3-chloro-4-hydroxy-5-methoxybenzaldehyde instead of 4-hydroxy-3,5-dimethoxybenzaldehyde into the title compound (34 mg, 17 %) which was obtained as a yellow solid. MS m/e: 410.0 [M+H]⁺

### Example 45

### 3-{2-[1-(3-Bromo-4-hydroxy-5-methoxy-phenyl)-meth-(Z)-ylidene]-3-oxo-2,3-dihydro-imidazo[2,1-b]thiazol-6-yl}-benzonitrile

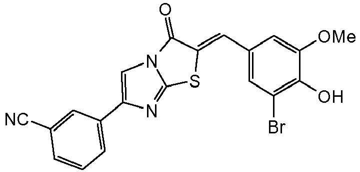

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted using 3-bromo-4-hydroxy-5-methoxybenzaldehyde instead of 4-hydroxy-3,5-dimethoxybenzaldehyde into the title compound (80 mg, 35 %) which was obtained as a yellow solid. MS m/e: 455.9 [M+H]⁺

### Example 46

### 2-[1-(3-bromo-4-hydroxy-5-methoxy-phenyl)-meth-(Z)-ylidene]-6-(4-fluoro-3-(trifluoromethyl)-phenyl)-imidazo[2,1-b]thiazol-3-one

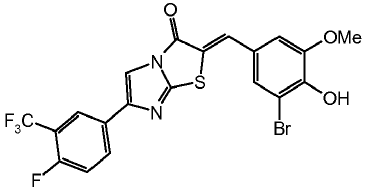

In analogy to the experimental procedure of example 1) 4-(4-fluoro-3-(trifluoromethyl)phenyl)-1H-imidazole-2(3H)-thione instead of 5-(pyridin-4-yl)-1H-imidazole-2-thiol was converted using 3-bromo-4-hydroxy-5-methoxybenzaldehyde instead of 4-hydroxy-3,5-dimethoxybenzaldehyde into the title compound (57 mg, 29 %) which was obtained as a yellow solid. MS m/e: 516.9 [M+H]⁺

### Example 47

### 2-[1-(3-Fluoro-4-hydroxy-5-methoxy-phenyl)-meth-(Z)-ylidene]-6-(3-methoxy-phenyl)-imidazo[2,1-b]thiazol-3-one

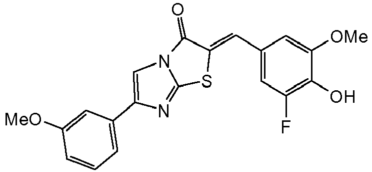

In analogy to the experimental procedure of example 43) 1-(3-methoxy-phenyl)-ethanone instead of acetophenone was converted into the title compound (17 mg, 11 %) which was obtained as a yellow solid. MS m/e: 399.1 [M+H]⁺

### Example 48

### 2-[1-(3-Fluoro-4-hydroxy-5-methoxy-phenyl)-meth-(Z)-ylidene]-6-(6-methoxy-pyridin-2-yl)-imidazo[2,1-b]thiazol-3-one

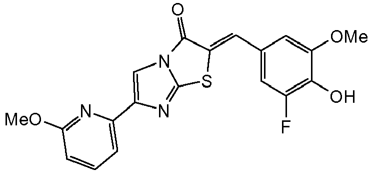

In analogy to the experimental procedure of example 43) 1-(6-methoxypyridin-2-yl)ethanone instead of acetophenone was converted into the title compound (5 mg, 3 %) which was obtained as a yellow solid. MS m/e: 400.1 [M+H]⁺

### Example 49

### 2-[1-(3-Chloro-4-hydroxy-5-methoxy-phenyl)-meth-(Z)-ylidene]-6-(3,5-difluoro-phenyl)-imidazo[2,1-b]thiazol-3-one

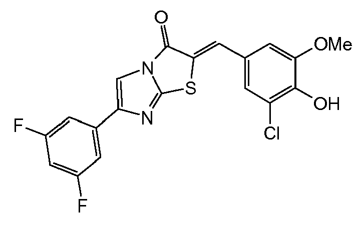

In analogy to the experimental procedure of example 43) 1-(3,5-difluorophenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (24 mg, 16 %) which was obtained as a yellow solid. MS m/e: 421.1 [M+H]⁺

### Example 50

### 3-{2-[1-(4-Hydroxy-3,5-dimethoxy-phenyl)-meth-(Z)-ylidene]-3-oxo-2,3-dihydro-imidazo[2,1-b]thiazol-6-yl}-benzonitrile

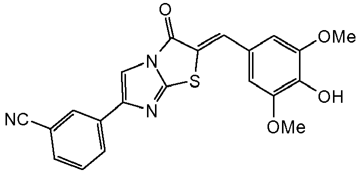

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted into the title compound (53 mg, 26 %) which was obtained as a yellow solid. MS m/e: 406.1 [M+H]⁺

### Example 51

### 3-{2-[1-(4-Hydroxy-3-methoxy-5-isopropoxyphenyl)-meth-(Z)-ylidene]-3-oxo-2,3-dihydro-imidazo[2,1-b]thiazol-6-yl}-benzonitrile

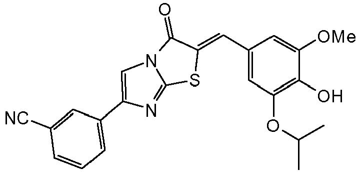

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted using 4-hydroxy-3-methoxy-5-isopropoxy-benzaldehyde instead of 4-hydroxy-3,5-dimethoxybenzaldehyde into the title compound (27 mg, 13 %) which was obtained as a yellow solid. MS m/e: 434.1 [M+H]⁺

### Example 52

### 2-[1-(3-Chloro-4-hydroxy-5-methoxy-phenyl)-meth-(Z)-ylidene]-6-m-tolyl-imidazo[2,1-b]thiazol-3-one

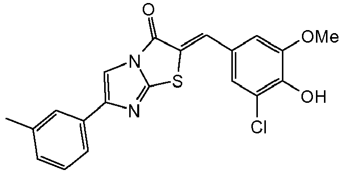

In analogy to the experimental procedure of example 43) 1-m-tolylethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (16 mg, 11 %) which was obtained as a yellow solid. MS m/e: 399.1 [M+H]⁺

### Example 53

### 2-[1-(3-Chloro-4-hydroxy-5-methoxy-phenyl)-meth-(Z)-ylidene]-6-(2-fluoro-phenyl)-imidazo[2,1-b]thiazol-3-one

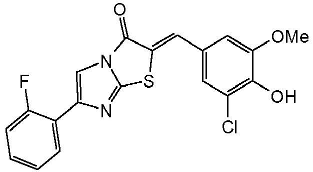

In analogy to the experimental procedure of example 43) 1-(2-fluorophenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (11 mg, 8 %) which was obtained as a yellow solid. MS m/e: 403.0 [M+H]⁺

### Example 54

### 2-[1-(3-Chloro-4-hydroxy-5-methoxy-phenyl)-meth-(Z)-ylidene]-6-(3-ethyl-phenyl)-imidazo[2,1-b]thiazol-3-one

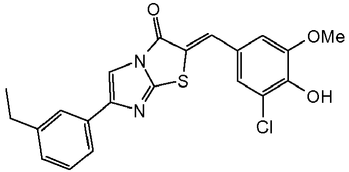

In analogy to the experimental procedure of example 43) 1-(3-ethylphenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (17 mg, 12 %) which was obtained as a yellow solid. MS m/e: 412.1 [M+H]⁺

### Example 55

### 2-[1-(3-Chloro-4-hydroxy-5-methoxy-phenyl)-meth-(Z)-ylidene]-6-(2-methoxy-phenyl)-imidazo[2,1-b]thiazol-3-one

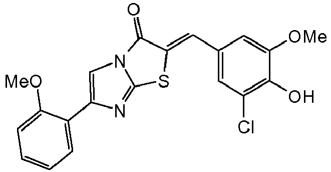

In analogy to the experimental procedure of example 43) 1-(2-methoxyphenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (3 mg, 2 %) which was obtained as a yellow solid. MS m/e: 415.1 [M+H]⁺

### Example 56

### 2-[1-(3-Chloro-4-hydroxy-5-methoxy-phenyl)-meth-(Z)-ylidene]-6-(2,5-difluoro-phenyl)-imidazo[2,1-b]thiazol-3-one

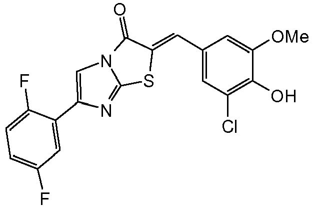

In analogy to the experimental procedure of example 43) 1-(2,5-difluorophenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (5 mg, 4 %) which was obtained as a yellow solid. MS m/e: 421.0 [M+H]⁺

### Example 57

### 2-[1-(3-Chloro-4-hydroxy-5-methoxy-phenyl)-meth-(Z)-ylidene]-6-(3-fluoro-phenyl)-imidazo[2,1-b]thiazol-3-one

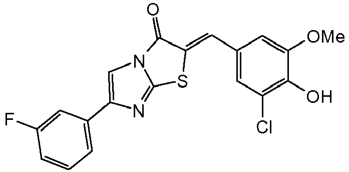

In analogy to the experimental procedure of example 1) 4-(3-fluorophenyl)-1H-imidazole-2(3H)-thione instead of 5-(pyridin-4-yl)-1H-imidazole-2-thiol was converted using 3-chloro-4-hydroxy-5-methoxybenzaldehyde instead of 4-hydroxy-3,5-dimethoxybenzaldehyde into the title compound (58 mg, 28 %) which was obtained as a yellow solid. MS m/e: 403.1 [M+H]⁺

### Example 58

### 2-[1-(3-Fluoro-4-hydroxy-5-methoxy-phenyl)-meth-(Z)-ylidene]-6-(3-fluoro-phenyl)-imidazo[2,1-b]thiazol-3-one

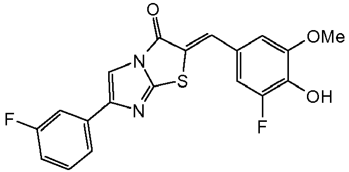

In analogy to the experimental procedure of example 1) 4-(3-fluorophenyl)-1H-imidazole-2(3H)-thione instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (54 mg, 27 %) which was obtained as a yellow solid. MS m/e: 387.1 [M+H]⁺

### Example 59

### 2-[1-(4-Hydroxy-3,5-dimethoxy-phenyl)-meth-(Z)-ylidene]-6-(3-fluoro-phenyl)-imidazo[2,1-b]thiazol-3-one

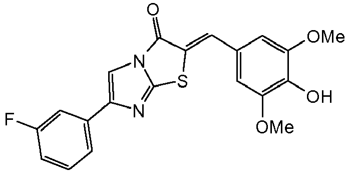

In analogy to the experimental procedure of example 1) 4-(3-fluorophenyl)-1H-imidazole-2(3H)-thione instead of 5-(pyridin-4-yl)-1H-imidazole-2-thiol was converted into the title compound (26 mg, 13 %) which was obtained as a yellow solid. MS m/e: 380.1 [M+H]⁺

### Example 60

### 2-[1-(3-Chloro-4-hydroxy-5-methoxy-phenyl)-meth-(Z)-ylidene]-6-phenyl-imidazo[2,1-b]thiazol-3-one

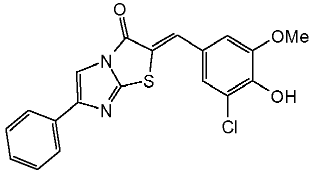

In analogy to the experimental procedure of example 43) acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (17 mg, 13 %) which was obtained as a yellow solid. MS m/e: 385.0 [M+H]⁺

### Example 61

### 2-[1-(3-Chloro-4-hydroxy-5-methoxy-phenyl)-meth-(Z)-ylidene]-6-(3-methoxy-phenyl)-imidazo[2,1-b]thiazol-3-one

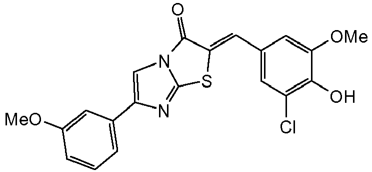

In analogy to the experimental procedure of example 43) 1-(3-methoxyphenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (20 mg, 14 %) which was obtained as a yellow solid. MS m/e: 415.1 [M+H]⁺

### Example 62

### 2-[1-(3-Chloro-4-hydroxy-5-methoxy-phenyl)-meth-(Z)-ylidene]-6-(3-(difluoromethoxy)-phenyl)-imidazo[2,1-b]thiazol-3-one

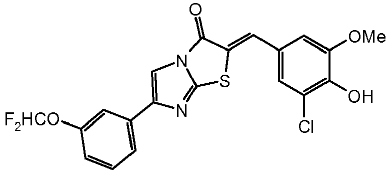

In analogy to the experimental procedure of example 43) 1-(3-(difluoro)methoxyphenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (32 mg, 20 %) which was obtained as a yellow solid. MS m/e: 451.0 [M+H]⁺

### Example 63

### 2-[1-(3-Chloro-4-hydroxy-5-methoxy-phenyl)-meth-(Z)-ylidene]-6-(2,6-dimethyl-pyridin-4-yl)-imidazo[2,1-b]thiazol-3-one

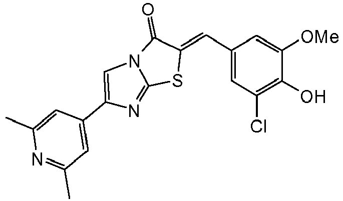

In analogy to the experimental procedure of example 9) 4-(2,6-dimethylpyridin-4-yl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (172 mg, 57 %) which was obtained as a brown solid. MS m/e: 414.1 [M+H]⁺

### Example 64

### 2-[1-(3-Fluoro-4-hydroxy-5-methoxy-phenyl)-meth-(Z)-ylidene]-6-(2,6-dimethyl-pyridin-4-yl)-imidazo[2,1-b]thiazol-3-one

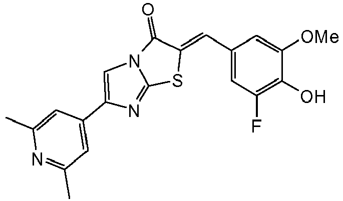

In analogy to the experimental procedure of example 9) 4-(2,6-dimethylpyridin-4-yl)-1H-imidazole-2(3H)-thione instead of 4-o-tolyl-1H-imidazole-2(3H)-thione was converted into the title compound (69 mg, 24 %) which was obtained as a brown solid. MS m/e: 398.1 [M+H]⁺

### Example 65

### 2-[1-(3-Bromo-4-hydroxy-5-methoxy-phenyl)-meth-(Z)-ylidene]-6-(2,6-dimethyl-pyridin-4-yl)-imidazo[2,1-b]thiazol-3-one

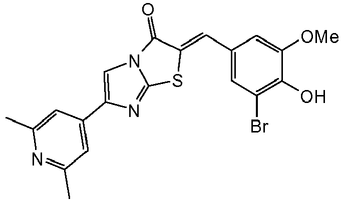

In analogy to the experimental procedure of example 9) 4-(2,6-dimethylpyridin-4-yl)-1H-imidazole-2(3H)-thione instead of 4-o-tolyl-1H-imidazole-2(3H)-thione was converted using 3-bromo-4-hydroxy-5-methoxybenzaldehyde instead of 3-fluoro-4-hydroxy-5-methoxybenzaldehyde into the title compound (217 mg, 65 %) which was obtained as a brown solid. MS m/e: 459.1 [M+H]⁺

### Example 66

### (Z)-3-(2-(3-fluoro-4-hydroxy-5-methoxybenzylidene)-3-oxo-2,3-dihydroimidazo[2,1-b]thiazol-5-yl)benzonitrile

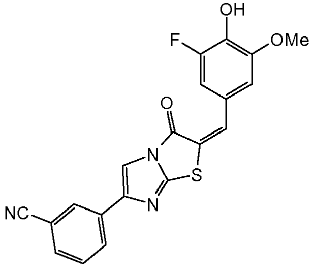

A suspension of (Z)-3-(2-(3-fluoro-4-hydroxy-5-methoxybenzylidene)-3-oxo-2,3-dihydroimidazo[2,1-b]thiazol-5-yl)benzonitrile (40 mg, 102 µmol) in methanol (30 mL) was stirred for 1 h at ambient temperature and then for 24 h at 50 °C. The suspension was concentrated in vacuo and dried. The resulting red solid was suspended in acetonitrile (30 mL) at 50 °C. After cooling to ambient temperature it was filtered over a satorius filter. Concentration in vacuo afforded the title compound (33 mg, 83 %) as a red solid. MS m/e: 394.1 [M+H]⁺

### Example 67

### 2-[1-(3-Chloro-4-hydroxy-5-methoxy-phenyl)-meth-(Z)-ylidene]-6-(3-(chloromethyl)phenyl)-imidazo[2,1-b]thiazol-3-one

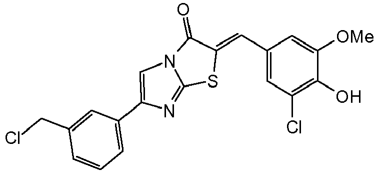

In analogy to the experimental procedure of example 43) 1-(3-(chloromethyl)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (26 mg, 17 %) which was obtained as a yellow solid. MS m/e: 433.2 [M+H]⁺

### Example 68

### 2-[1-(3-Chloro-4-hydroxy-5-methoxy-phenyl)-meth-(Z)-ylidene]-6-(pyridin-4-yl)-imidazo[2,1-b]thiazol-3-one

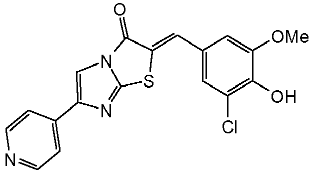

In analogy to the experimental procedure of example 9) 4-(pyridin-4-yl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (74 mg, 23 %) which was obtained as a brown solid. MS m/e: 486.1 [M+H]⁺

### Example 69

### 2-[1-(3-Fluoro-4-hydroxy-5-methoxy-phenyl)-meth-(Z)-ylidene]-6-(pyridin-4-yl)-imidazo[2,1-b]thiazol-3-one

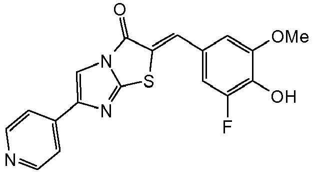

In analogy to the experimental procedure of example 9) 4-(pyridin-4-yl)-1H-imidazole-2(3H)-thione instead of 4-o-tolyl-1H-imidazole-2(3H)-thione was converted into the title compound (74 mg, 24 %) which was obtained as a brown solid. MS m/e: 370.1 [M+H]⁺

### Example 70

### 2-[1-(3-Bromo-4-hydroxy-5-methoxy-phenyl)-meth-(Z)-ylidene]-6-(pyridin-4-yl)-imidazo[2,1-b]thiazol-3-one

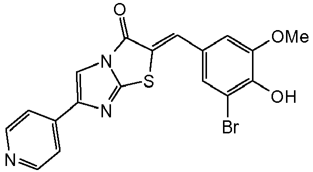

In analogy to the experimental procedure of example 9) 4-(pyridin-4-yl)-1H-imidazole-2(3H)-thione instead of 4-o-tolyl-1H-imidazole-2(3H)-thione was converted using 3-bromo-4-hydroxy-5-methoxybenzaldehyde instead of 3-fluoro-4-hydroxy-5-methoxybenzaldehyde into the title compound (95 mg, 26 %) which was obtained as a brown solid. MS m/e: 431.1 [M+H]⁺

### Example 71

### 2-[1-(3-Fluoro-4-hydroxy-5-methoxy-phenyl)-meth-(Z)-ylidene]-6-(pyridin-3-yl)-imidazo[2,1-b]thiazol-3-one

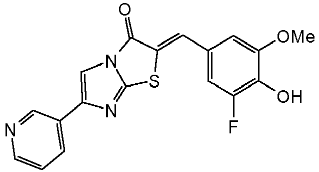

In analogy to the experimental procedure of example 9) 4-(pyridin-3-yl)-1H-imidazole-2(3H)-thione instead of 4-o-tolyl-1H-imidazole-2(3H)-thione was converted into the title compound (101 mg, 32%) which was obtained as a brown solid. MS m/e: 370.1 [M+H]⁺

### Example 72

### 2-[1-(3-Bromo-4-hydroxy-5-methoxy-phenyl)-meth-(Z)-ylidene]-6-(pyridin-3-yl)-imidazo[2,1-b]thiazol-3-one

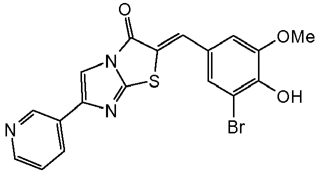

In analogy to the experimental procedure of example 9) 4-(pyridin-3-yl)-1H-imidazole-2(3H)-thione instead of 4-o-tolyl-1H-imidazole-2(3H)-thione was converted using 3-bromo-4-hydroxy-5-methoxybenzaldehyde instead of 3-fluoro-4-hydroxy-5-methoxybenzaldehyde into the title compound (149 mg, 41 %) which was obtained as a brown solid. MS m/e: 431.1 [M+H]⁺

### Example 73

### 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile

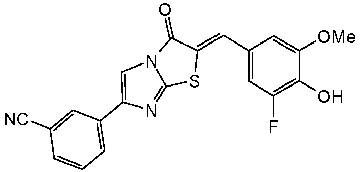

In analogy to the experimental procedure of example 1) 3-(2-thioxo-2,3-dihydro-1H-imidazol-4-yl)benzonitrile instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (126 mg, 17 %) which was obtained as a brown solid. MS m/e: 378.9 [M+H]⁺

### Example 74

### 2-[1-(3-Chloro-4-hydroxy-5-methoxy-phenyl)-meth-(Z)-ylidene]-6-(3-(2-fluoroethoxy)phenyl)-imidazo[2,1-b]thiazol-3-one

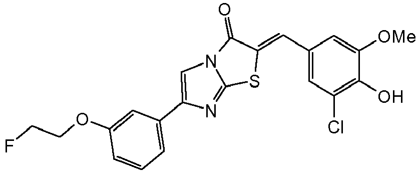

In analogy to the experimental procedure of example 43) 1-(3-(2-fluoroethoxy)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (4 mg, 3 %) which was obtained as a yellow solid. MS m/e: 447.1 [M+H]⁺

### Example 75

### 2-[1-(3-Chloro-4-hydroxy-5-methoxy-phenyl)-meth-(Z)-ylidene]-6-(2-methylpyridin-4-yl)-imidazo[2,1-b]thiazol-3-one

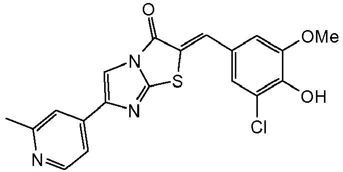

In analogy to the experimental procedure of example 9) 4-(2-methylpyridin-4-yl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (38 mg, 12 %) which was obtained as a brown solid. MS m/e: 400.1 [M+H]⁺

### Example 76

### 2-[1-(3-Fluoro-4-hydroxy-5-methoxy-phenyl)-meth-(Z)-ylidene]-6-(2-methylpyridin-4-yl)-imidazo[2,1-b]thiazol-3-one

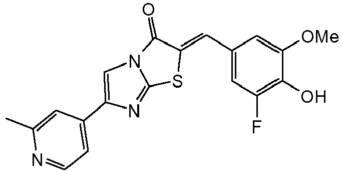

In analogy to the experimental procedure of example 9) 4-(2-methylpyridin-4-yl)-1H-imidazole-2(3H)-thione instead of 4-o-tolyl-1H-imidazole-2(3H)-thione was converted into the title compound (60 mg, 20 %) which was obtained as a brown solid. MS m/e: 384.1 [M+H]⁺

### Example 77

### 2-[1-(3-Bromo-4-hydroxy-5-methoxy-phenyl)-meth-(Z)-ylidene]-6-(2-methylpyridin-4-yl)-imidazo[2,1-b]thiazol-3-one

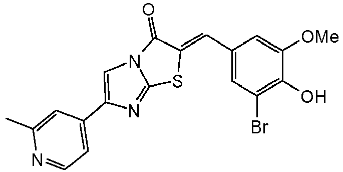

In analogy to the experimental procedure of example 9) 4-(2-methylpyridin-4-yl)-1H-imidazole-2(3H)-thione instead of 4-o-tolyl-1H-imidazole-2(3H)-thione was converted using 3-bromo-4-hydroxy-5-methoxybenzaldehyde instead of 3-fluoro-4-hydroxy-5-methoxybenzaldehyde into the title compound (111 mg, 32 %) which was obtained as a brown solid. MS m/e: 445.1 [M+H]⁺

### Example 78

### 2-[1-(3-Chloro-4-hydroxy-5-methoxy-phenyl)-meth-(Z)-ylidene]-6-(pyridin-3-yl)-imidazo[2,1-b]thiazol-3-one

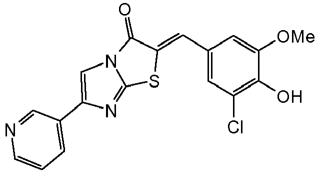

In analogy to the experimental procedure of example 9) 4-(pyridin-3-yl)-1H-imidazole-2(3H)-thione instead of 4-o-tolyl-1H-imidazole-2(3H)-thione was converted using 3-chloro-4-hydroxy-5-methoxybenzaldehyde instead of 3-fluoro-4-hydroxy-5-methoxybenzaldehyde into the title compound (115 mg, 35 %) which was obtained as a brown solid. MS m/e: 386.1 [M+H]⁺

### Example 79

### 2-[1-(3-Chloro-4-hydroxy-5-methoxy-phenyl)-meth-(Z)-ylidene]-6-(4-(2-fluoroethoxy)phenyl)-imidazo[2,1-b]thiazol-3-one

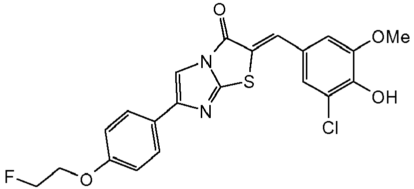

In analogy to the experimental procedure of example 43) 1-(4-(2-fluoroethoxy)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (20 mg, 13 %) which was obtained as a yellow solid. MS m/e: 447.1 [M+H]⁺

### Example 80

### 2-[1-(3-Chloro-4-hydroxy-5-methoxy-phenyl)-meth-(Z)-ylidene]-6-(3-(allyloxy)phenyl)-imidazo[2,1-b]thiazol-3-one

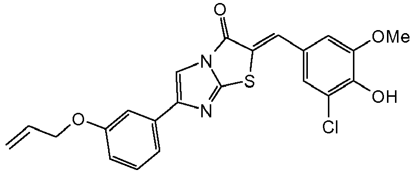

In analogy to the experimental procedure of example 43) 1-(3-(allyloxy)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (42 mg, 16 %) which was obtained as a yellow solid. MS m/e: 441.1 [M+H]⁺

### Example 81

### 2-[1-(3-Chloro-4-hydroxy-5-methoxy-phenyl)-meth-(Z)-ylidene]-6-(3-propoxyphenyl)-imidazo[2,1-b]thiazol-3-one

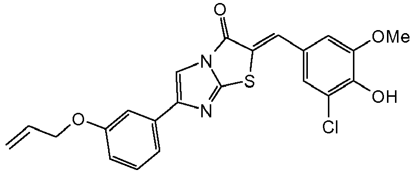

In analogy to the experimental procedure of example 43) 1-(3-propoxyphenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (13 mg, 8 %) which was obtained as a brown solid. MS m/e: 415.1 [M+H]⁺

### Example 82

### 2-[1-(3-Chloro-4-hydroxy-5-methoxy-phenyl)-meth-(Z)-ylidene]-6-(2-(hydroxyethoxy)phenyl)-imidazo[2,1-b]thiazol-3-one

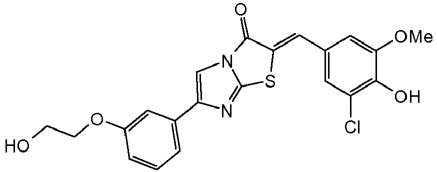

In analogy to the experimental procedure of example 43) 1-(3-(2-hydroxyethoxy)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (23 mg, 9 %) which was obtained as a yellow solid. MS m/e: 445.2 [M+H]⁺

### Example 83

### 2-[1-(3-Chloro-4-hydroxy-5-methoxy-phenyl)-meth-(Z)-ylidene]-6-(2-(2-fluoroethoxy)phenyl)-imidazo[2,1-b]thiazol-3-one

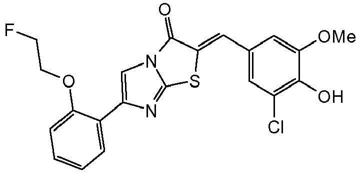

In analogy to the experimental procedure of example 43) 1-(2-(2-fluoroethoxy)phenyl)ethanone instead of acetophenone was converted using (Z)-2-amino-5-(3-chloro-4-hydroxy-5-methoxybenzylidene)thiazol-4(5H)-one instead of (Z)-2-amino-5-(3-fluoro-4-hydroxy-5-methoxybenzylidene)thiazol-4(5H)-one into the title compound (70 mg, 27 %) which was obtained as a yellow solid. MS m/e: 445.1 [M+H]⁻

### Example 84

### 2-[1-(3-Chloro-4-hydroxy-5-methoxy-phenyl)-meth-(Z)-ylidene]-6-(5-methyl-isoxazol-3-yl)-imidazo[2,1-b]thiazol-3-one

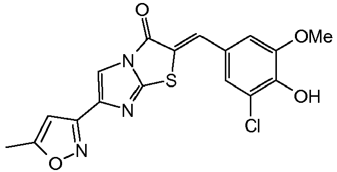

In analogy to the experimental procedure of example 1) 4-(5-methylisoxazol-3-yl)-1H-imidazole-2(3H)-thione instead of 5-(pyridin-4-yl)-1H-imidazole-2-thiol was converted using 3-chloro-4-hydroxy-5-methoxybenzaldehyde instead of 4-hydroxy-3,5-dimethoxybenzaldehyde into the title compound (207 mg, 64 %) which was obtained as a yellow solid. MS m/e: 390.1 [M+H]⁺

### Example 85

### 3-Bromo-5-{2-[1-(3-chloro-4-hydroxy-5-methoxy-phenyl)-meth-(Z)-ylidene]-3-oxo-2,3-dihydro-imidazo[2,1-b]thiazol-6-yl}-benzonitrile

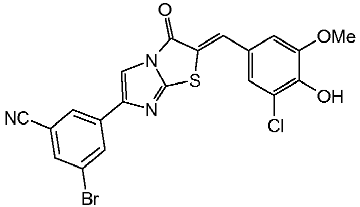

To a mixture of (Z)-2-amino-5-(3-chloro-4-hydroxy-5-methoxybenzylidene)thiazol-4(5H)-one (200 mg, 702 µmol) and 3-bromo-5-(2-bromoacetyl)benzonitrile (213 mg, 702 µmol was added 2-propanol (4 mL). The reaction mixture was irradiated in the microwave at 160 °C for 30 min. The resulting yellow suspension was diluted with water (2 mL) and filtered off The solid was washed twice with an aqueous solution of sodium hydrogen carbonate (1M, 2 mL), twice with an aqueous solution of citric acid (5 %, 2 mL) and twice with water (3 mL) affording the title compound (218 mg, 64 %) as a yellow solid. MS m/e: 490.0 [M+H]+

### Example 86

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide

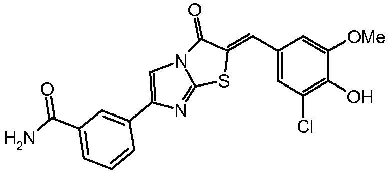

In analogy to the experimental procedure of example 1) 3-(2-thioxo-2,3-dihydro-1H-imidazol-4-yl)benzamide instead of 5-(pyridin-4-yl)-1H-imidazole-2-thiol was converted using 3-chloro-4-hydroxy-5-methoxybenzaldehyde instead of 4-hydroxy-3,5-dimethoxybenzaldehyde into the title compound (102 mg, 29 %) which was obtained as a yellow solid. MS m/e: 428.1 [M+H]⁺

### Example 87

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile

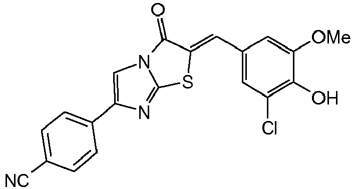

In analogy to the experimental procedure of example 85) (Z)-2-amino-5-(3-chloro-4-hydroxy-5-methoxybenzylidene)thiazol-4(5H)-one was converted using 4-(2-bromoacetyl)benzonitrile instead of 3-bromo-5-(2-bromoacetyl)benzonitrile into the title compound (81 mg, 56 %) which was obtained as a yellow solid. MS m/e: 410.2 [M+H]⁺

### Example 88

### 2-[1-(3-Chloro-4-hydroxy-5-methoxy-phenyl)-meth-(Z)-ylidene]-6-(4-(difluoromethoxy)phenyl)-imidazo[2,1-b]thiazol-3-one

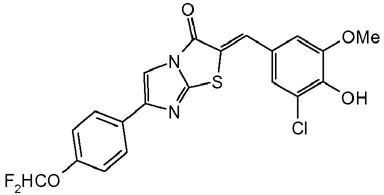

In analogy to the experimental procedure of example 85) (Z)-2-amino-5-(3-chloro-4-hydroxy-5-methoxybenzylidene)thiazol-4(5H)-one was converted using 2-bromo-1-(4-(difluoromethoxy)phenyl)ethanone instead of 3-bromo-5-(2-bromoacetyl)benzonitrile into the title compound (81 mg, 56 %) which was obtained as a yellow solid. MS m/e: 410.2 [M+H]⁺

### Example 89

### 2-[1-(3-Chloro-4-hydroxy-5-methoxy-phenyl)-meth-(Z)-ylidene]-6-(3-morpholinophenyl)-imidazo[2,1-b]thiazol-3-one

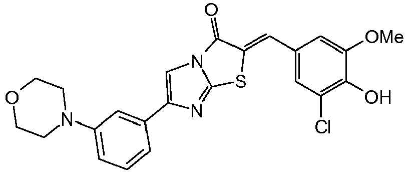

In analogy to the experimental procedure of example 85) (Z)-2-amino-5-(3-chloro-4-hydroxy-5-methoxybenzylidene)thiazol-4(5H)-one was converted using 2-bromo-1-(3-morpholinophenyl)ethanone instead of 3-bromo-5-(2-bromoacetyl)benzonitrile into the title compound (95 mg, 47 %) which was obtained as a yellow solid. MS m/e: 470.1 [M+H]⁺

### Example 90

### 2-[1-(3-Chloro-4-hydroxy-5-methoxy-phenyl)-meth-(Z)-ylidene]-6-(4-(pyrrolidin-1-yl)phenyl)-imidazo[2,1-b]thiazol-3-one

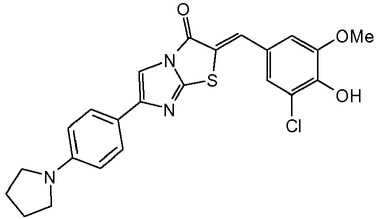

In analogy to the experimental procedure of example 85) (Z)-2-amino-5-(3-chloro-4-hydroxy-5-methoxybenzylidene)thiazol-4(5H)-one was converted using 2-promo-1-(4-(pyrrolidin-1-yl)phenyl)ethanone instead of 3-bromo-5-(2-bromoacetyl)benzonitrile into the title compound (56 mg, 29 %) which was obtained as a brown solid. MS m/e: 454.2 [M+H]⁺

### Example 91

### 2-[1-(3-Chloro-4-hydroxy-5-methoxy-phenyl)-meth-(Z)-ylidene]-6-(4-(diethylamino)phenyl)-imidazo[2,1-b]thiazol-3-one

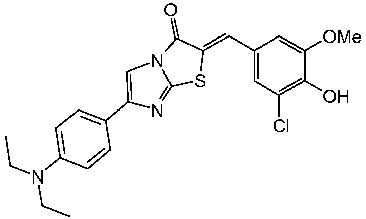

In analogy to the experimental procedure of example 85) (Z)-2-amino-5-(3-chloro-4-hydroxy-5-methoxybenzylidene)thiazol-4(5H)-one was converted using 2-promo-1-(4-(diethylamino)phenyl)ethanone instead of 3-bromo-5-(2-bromoacetyl)benzonitrile into the title compound (89 mg, 54 %) which was obtained as a brown solid. MS m/e: 456.2 [M+H]⁺

### Example 92

### 2-[1-(3-Chloro-4-hydroxy-5-methoxy-phenyl)-meth-(Z)-ylidene]-6-(4-morpholinophenyl)-imidazo[2,1-b]thiazol-3-one

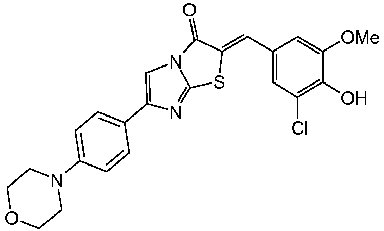

In analogy to the experimental procedure of example 85) (Z)-2-amino-5-(3-chloro-4-hydroxy-5-methoxybenzylidene)thiazol-4(5H)-one was converted using 2-bromo-1-(4-morpholinophenyl)ethanone instead of 3-bromo-5-(2-bromoacetyl)benzonitrile into the title compound (18 mg, 8 %) which was obtained as a brown solid. MS m/e: 470.1 [M+H]⁺

### Example 93

### 2-[1-(3-Chloro-4-hydroxy-5-methoxy-phenyl)-meth-(Z)-ylidene]-6-(4-(dimethylamino)phenyl)-imidazo[2,1-b]thiazol-3-one

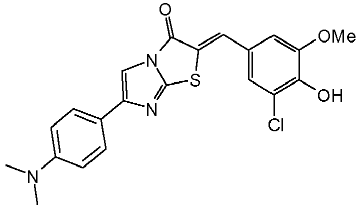

In analogy to the experimental procedure of example 85) (Z)-2-amino-5-(3-chloro-4-hydroxy-5-methoxybenzylidene)thiazol-4(5H)-one was converted using 2-bromo-1-(4-(dimethylamino)phenyl)ethanone instead of 3-bromo-5-(2-bromoacetyl)benzonitrile into the title compound (37 mg, 17 %) which was obtained as a brown solid. MS m/e: 428.2 [M+H]⁺

### Example 94

### 2-[1-(3-Chloro-4-hydroxy-5-methoxy-phenyl)-meth-(Z)-ylidene]-6-[3-(2-oxo-pyrrolidin-1-yl)-phenyl]-imidazo[2,1-b]thiazol-3-one

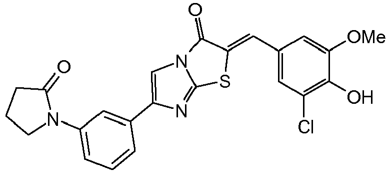

In analogy to the experimental procedure of example 85) (Z)-2-amino-5-(3-chloro-4-hydroxy-5-methoxybenzylidene)thiazol-4(5H)-one was converted using 1-(3-(2-bromoacetyl)phenyl)pyrrolidin-2-one instead of 3-bromo-5-(2-bromoacetyl)benzonitrile into the title compound (99 mg, 54 %) which was obtained as an orange solid. MS m/e: 468.2 [M+H]⁺

### Example 95

### 2-[1-(3-Chloro-4-hydroxy-5-methoxy-phenyl)-meth-(Z)-ylidene]-6-(3-{2-[2-(2-fluoro-ethoxy)-ethoxy]-ethoxy}-phenyl)-imidazo[2,1-b]thiazol-3-one

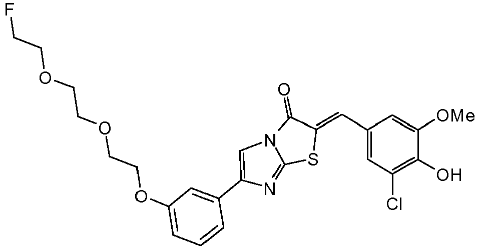

In analogy to the experimental procedure of example 85) (Z)-2-amino-5-(3-chloro-4-hydroxy-5-methoxybenzylidene)thiazol-4(5H)-one was converted using 2-bromo-1-(3-(2-(2-(2-fluoroethoxy)ethoxy)ethoxy)phenyl)ethanone instead of 3-bromo-5-(2-bromoacetyl)benzonitrile into the title compound (24 mg, 31%) which was obtained as a yellow solid. MS m/e: 535.1 [M+H]⁺

### Example 96

### 2-[1-(3-Chloro-4-hydroxy-5-methoxy-phenyl)-meth-(Z)-ylidene]-6-(2-{2-[2-(2-fluoro-ethoxy)-ethoxy]-ethoxy}-phenyl)-imidazo[2,1-b]thiazol-3-one

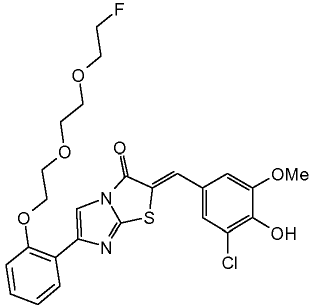

In analogy to the experimental procedure of example 85) (Z)-2-amino-5-(3-chloro-4-hydroxy-5-methoxybenzylidene)thiazol-4(5H)-one was converted using 2-bromo-1-(2-(2-(2-(2-fluoroethoxy)ethoxy)ethoxy)phenyl)ethanone instead of 3-bromo-5-(2-bromoacetyl)benzonitrile into the title compound (34 mg, 20 %) which was obtained as a yellow solid. MS m/e: 470.1 [M+H]⁺

### Example 97

### 2-[1-(3-Chloro-4-hydroxy-5-methoxy-phenyl)-meth-(Z)-ylidene]-6-[6-(4-methyl-3H-imidazol-1-yl)-pyridin-3-yl]-imidazo[2,1-b]thiazol-3-one

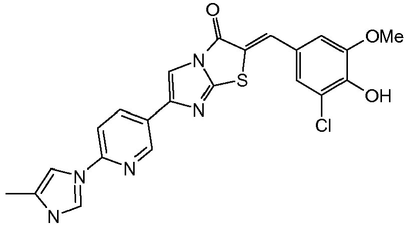

In analogy to the experimental procedure of example 85) (Z)-2-amino-5-(3-chloro-4-hydroxy-5-methoxybenzylidene)thiazol-4(5H)-one was converted using 2-bromo-1-(6-(4-methyl-1H-imidazol-1-yl)pyridin-3-yl)ethanone instead of 3-bromo-5-(2-bromoacetyl)benzonitrile into the title compound (94 mg, 69 %) which was obtained as a yellow solid. MS m/e: 466.2 [M+H]⁺

### Example 98

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid

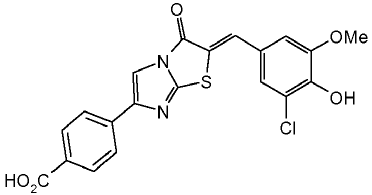

In analogy to the experimental procedure of example 85) (Z)-2-amino-5-(3-chloro-4-hydroxy-5-methoxybenzylidene)thiazol-4(5H)-one was converted using 4-(2-bromoacetyl)benzoic acid instead of 3-bromo-5-(2-bromoacetyl)benzonitrile into the title compound (49 mg, 35 %) which was obtained as a yellow solid. MS m/e: 427.1 [M+H]⁻

### Example 99

### 2-[1-(3-Chloro-4-hydroxy-5-methoxy-phenyl)-meth-(Z)-ylidene]-6-(3,4-dimethoxy-phenyl)-imidazo[2,1-b]thiazol-3-one

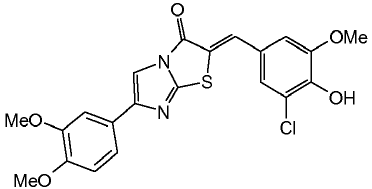

In analogy to the experimental procedure of example 85) (Z)-2-amino-5-(3-chloro-4-hydroxy-5-methoxybenzylidene)thiazol-4(5H)-one was converted using 2-bromo-1-(3,4-dimethoxyphenyl)ethanone instead of 3-bromo-5-(2-bromoacetyl)benzonitrile into the title compound (43 mg, 21 %) which was obtained as a yellow solid. MS m/e: 443.2 [M+H]⁻

### Example 100

### 2-[1-(3-Chloro-4-hydroxy-5-methoxy-phenyl)-meth-(Z)-ylidene]-6-(2,3-dihydro-benzo[1,4]dioxin-6-yl)-imidazo[2,1-b]thiazol-3-one

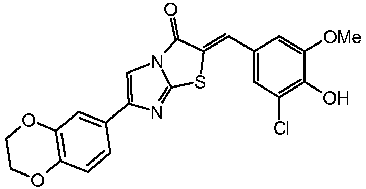

In analogy to the experimental procedure of example 85) (Z)-2-amino-5-(3-chloro-4-hydroxy-5-methoxybenzylidene)thiazol-4(5H)-one was converted using 2-bromo-1-(2,3-dihydrobenzo[b][1,4]dioxin-6-yl)ethanone instead of 3-bromo-5-(2-bromoacetyl)benzonitrile into the title compound (40 mg, 25 %) which was obtained as a yellow solid. MS m/e: 443.2 [M+H]⁺

### Example 101

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide

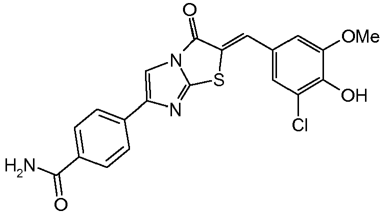

In analogy to the experimental procedure of example 85) (Z)-2-amino-5-(3-chloro-4-hydroxy-5-methoxybenzylidene)thiazol-4(5H)-one was converted using 4-(2-bromoacetyl)benzamide instead of 3-bromo-5-(2-bromoacetyl)benzonitrile into the title compound (140 mg, 79 %) which was obtained as a yellow solid. MS m/e: 426.1 [M+H]⁻

### Example 102

### 5-{2-[1-(3-Chloro-4-hydroxy-5-methoxy-phenyl)-meth-(Z)-ylidene]-3-oxo-2,3-dihydro-imidazo[2,1-b]thiazol-6-yl}-1,3-dihydro-indol-2-one

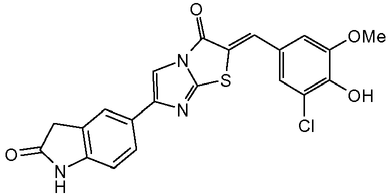

In analogy to the experimental procedure of example 85) (Z)-2-amino-5-(3-chloro-4-hydroxy-5-methoxybenzylidene)thiazol-4(5H)-one was converted using 5-(2-bromoacetyl)indolin-2-one instead of 3-bromo-5-(2-bromoacetyl)benzonitrile into the title compound (96 mg, 67 %) which was obtained as an orange solid. MS m/e: 440.2 [M+H]⁺

### Example 103

### N-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-phenyl)-acetamide

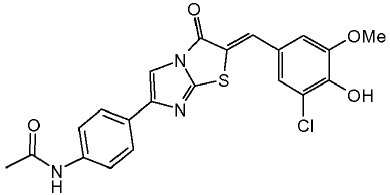

In analogy to the experimental procedure of example 85) (Z)-2-amino-5-(3-chloro-4-hydroxy-5-methoxybenzylidene)thiazol-4(5H)-one was converted using N-(4-(2-bromoacetyl)phenyl)acetamide instead of 3-bromo-5-(2-bromoacetyl)benzonitrile into the title compound (64 mg, 44 %) which was obtained as an orange solid. MS m/e: 442.2 [M+H]⁺

### Example 104

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid

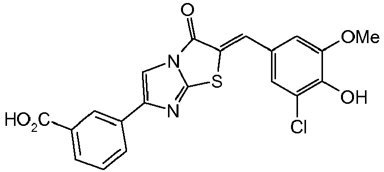

In analogy to the experimental procedure of example 85) (Z)-2-amino-5-(3-chloro-4-hydroxy-5-methoxybenzylidene)thiazol-4(5H)-one was converted using 3-(2-bromoacetyl)benzoic acid instead of 3-bromo-5-(2-bromoacetyl)benzonitrile into the title compound (952 mg, 65 %) which was obtained as a yellow solid. MS m/e: 429.1 [M+H]⁺

### Example 105

### 2-[1-(3-Chloro-4-hydroxy-5-methoxy-phenyl)-meth-(Z)-ylidene]-6-(2,6-difluoro-phenyl)-imidazo[2,1-b]thiazol-3-one

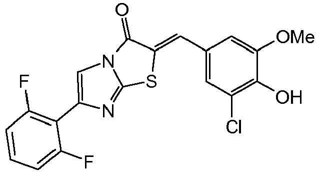

In analogy to the experimental procedure of example 85) (Z)-2-amino-5-(3-chloro-4-hydroxy-5-methoxybenzylidene)thiazol-4(5H)-one was converted using 2-bromo-1-(2,6-difluorophenyl)ethanone instead of 3-bromo-5-(2-bromoacetyl)benzonitrile into the title compound (38 mg, 25 %) which was obtained as a yellow solid. MS m/e: 421.0 [M+H]⁺

### Example 106

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide

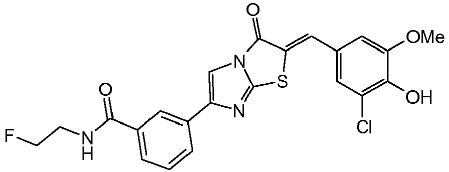

To a solution of (Z)-3-(2-(3-chloro-4-hydroxy-5-methoxybenzylidene)-3-oxo-2,3-dihydroimidazo[2,1-b]thiazol-5-yl)benzoic acid (96 mg, 224 µmol) in N-methyl-2-pyrrolidinone (1 mL) was added under a nitrogen atmosphere 2-(1H-benzo[d][1,2,3]triazol-1-yl)-1,1,3,3-tetramethylisouronium tetrafluoroborate (79.1 mg, 246 µmol) and 2-fluoroethylamine hydrochloride (22.3 mg, 224 µmol). After the addition ofN,N-diisopropylethylamine (63.7 mg, 86.0 µL, 492 µmol) the color changed from yellow to red. The solution was stirred for 3 h at ambient temperature. Thereaction mixture was diluted with dichloromethane (15 mL) and was washed with water (15 ml) and an aqueous solution of citric acid (5 %, 15 mL). The aqueous layers were extracted three times with dichloromethane (15 mL). The combined organic layers were dried over sodium sulfate. Purification by chromatography (SiO₂, eluent: dichloromethane:ethyl acetate = 100:0 to 70:30) afforded the title compound (28 mg, 26 %) as a yellow solid. MS m/e: 474.1 [M+H]⁺

### Example 107

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide

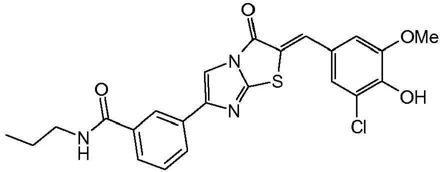

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using propan-1-amine instead of 2-fluoroethylamine hydrochloride into the title compound (35 mg, 27 %) which was obtained as a yellow solid. MS m/e: 470.2 [M+H]⁺

### Example 108

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide

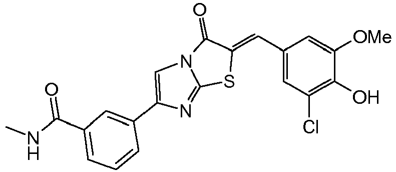

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using methylamine hydrochloride instead of 2-fluoroethylamine hydrochloride into the title compound (30 mg, 18 %) which was obtained as a yellow solid. MS m/e: 442.1 [M+H]⁺

### Example 109

### 2-[1-(3-Chloro-4-hydroxy-5-propoxy-phenyl)-meth-(Z)-ylidene]-6-(4-methyl-pyridin-3-yl)-imidazo[2,1-b]thiazol-3-one

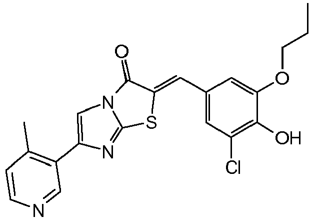

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-promo-1-(4-methylpyridin-3-yl)ethanone hydrobromide instead of 3-bromo-5-(2-bromoacetyl)benzonitrile into the title compound (17 mg, 9 %) which was obtained as a yellow solid. MS m/e: 428.1 [M+H]⁺

### Example 110

### 2-[1-(3-Chloro-4-hydroxy-5-propoxy-phenyl)-meth-(Z)-ylidene]-6-pyridin-3-yl-imidazo[2,1-b]thiazol-3-one

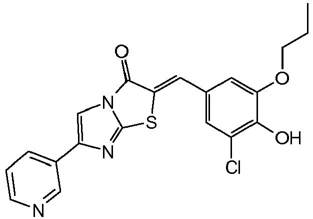

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(pyridin-3-yl)ethanone hydrobromide instead of 3-bromo-5-(2-bromoacetyl)benzonitrile into the title compound (35 mg, 19 %) which was obtained as a yellow solid. MS m/e: 414.2 [M+H]⁺

### Example 111

### 2-[1-(3-Chloro-4-hydroxy-5-propoxy-phenyl)-meth-(Z)-ylidene]-6-pyridin-4-yl-imidazo[2,1-b]thiazol-3-one

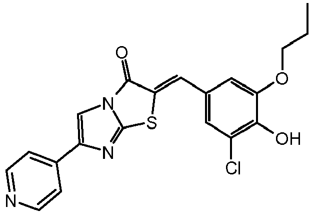

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(pyridin-4-yl)ethanone hydrobromide instead of 3-bromo-5-(2-bromoacetyl)benzonitrile into the title compound (15 mg, 9 %) which was obtained as a yellow solid. MS m/e: 414.1 [M+H]⁺

### Example 112

### 2-[1-(3-Chloro-4-hydroxy-5-propoxy-phenyl)-meth-(Z)-ylidene]-6-[6-(4-methyl-imidazol-1-yl)-pyridin-3-yl]-imidazo[2,1-b]thiazol-3-one

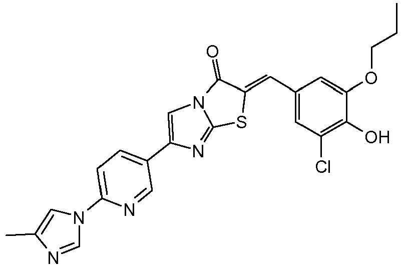

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(6-(4-methyl-1H-imidazol-1-yl)pyridin-3-yl)ethanone acetate instead of 3-bromo-5-(2-bromoacetyl)benzonitrile into the title compound (39 mg, 23 %) which was obtained as a yellow solid. MS m/e: 494.3 [M+H]⁺

### Example 113

### N-Allyl-3-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide

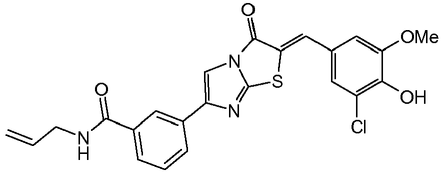

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using prop-2-en-1-amine instead of 2-fluoroethylamine hydrochloride into the title compound (49 mg, 42 %) which was obtained as a yellow solid. MS m/e: 468.2 [M+H]⁺

### Example 114

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide

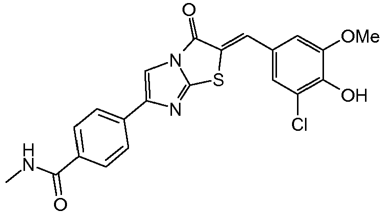

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using methylamine hydrochloride instead of 2-fluoroethylamine hydrochloride into the title compound (27 mg, 18 %) which was obtained as a yellow solid. MS m/e: 442.1 [M+H]⁺

### Example 115

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide

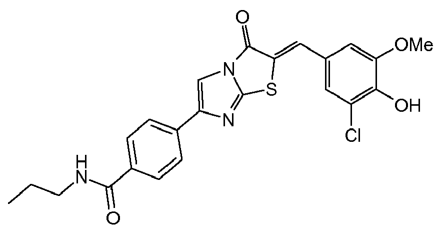

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using propan-1-amine instead of 2-fluoroethylamine hydrochloride into the title compound (46 mg, 28 %) which was obtained as a yellow solid. MS m/e: 470.2 [M+H]⁺

### Example 116

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide

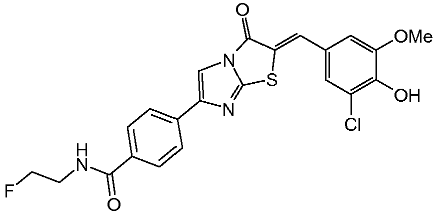

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted into the title compound (80 mg, 48 %) which was obtained as a yellow solid. MS m/e: 474.2 [M+H]⁺

### Example 117

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoro-propyl)-benzamide

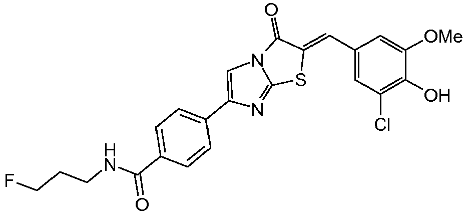

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoropropan-1-amine hydrochloride instead of 2-fluoroethylamine hydrochloride into the title compound (55 mg, 42 %) which was obtained as a yellow solid. MS m/e: 488.2 [M+H]⁺

### Example 118

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(3-fluoro-phenyl)-ethyl]-N-methyl-benzamide

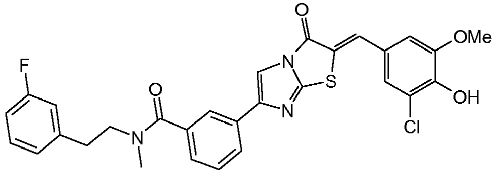

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using [2-(3-fluoro-phenyl)-ethyl]-methyl-amine hydrochloride instead of 2-fluoroethylamine hydrochloride into the title compound (59 mg, 36 %) which was obtained as a yellow solid. MS m/e: 564.3 [M+H]⁺

### Example 119

### 2-[1-(3-Chloro-4-hydroxy-5-propoxy-phenyl)-meth-(Z)-ylidene]-6-(3H-imidazol-4-yl)-imidazo[2,1-b]thiazol-3-one

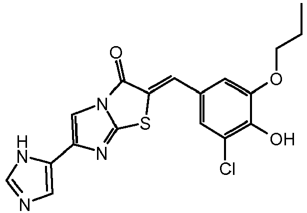

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(1H-imidazol-5-yl)ethanone hydrobromide instead of 3-bromo-5-(2-bromoacetyl)benzonitrile into the title compound (8 mg, 7 %) which was obtained as a yellow solid. MS m/e: 403.3 [M+H]⁺

### Example 120

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide

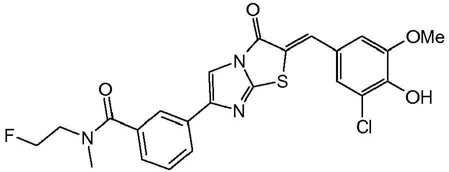

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using 2-fluoro-N-methylethanamine hydrochloride instead of 2-fluoroethylamine hydrochloride into the title compound (54 mg, 38 %) which was obtained as a yellow solid. MS m/e: 488.1 [M+H]⁺

### Example 121

### 3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzamide

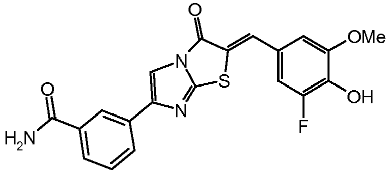

In analogy to the experimental procedure of example 1) 3-(2-thioxo-2,3-dihydro-1H-imidazol-4-yl)benzamide instead of 5-(pyridin-4-yl)-1H-imidazole-2-thiol was converted using 3-fluoro-4-hydroxy-5-methoxybenzaldehyde instead of 4-hydroxy-3,5-dimethoxybenzaldehyde into the title compound (19 mg, 6 %) which was obtained as a dark red solid. MS m/e: 412.2 [M+H]⁺

### Example 122

### 2-[1-(3-Fluoro-4-hydroxy-5-propoxy-phenyl)-meth-(Z)-ylidene]-6-pyridin-4-yl-imidazo[2,1-b]thiazol-3-one

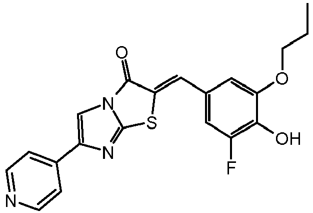

In analogy to the experimental procedure of example 9) 4-(pyridin-4-yl)-1H-imidazole-2(3H)-thione instead of 4-o-tolyl-1H-imidazole-2(3H)-thione was converted using 3-fluoro-4-hydroxy-5-methoxybenzaldehyde instead of 3-fluoro-4-hydroxy-5-methoxybenzaldehyde into the title compound (28 mg, 14 %) which was obtained as a red solid. MS m/e: 398.2 [M+H]⁺

### Example 123

### 2-[1-(3-Chloro-5-(3-fluoropropoxy)-4-hydroxy-phenyl)-meth-(Z)-ylidene]-6-pyridin-4-yl-imidazo[2,1-b]thiazol-3-one

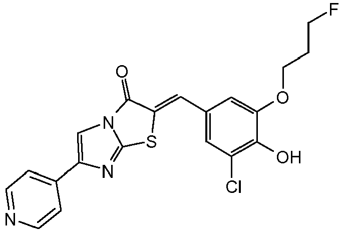

In analogy to the experimental procedure of example 9) 4-(pyridin-4-yl)-1H-imidazole-2(3H)-thione instead of 4-o-tolyl-1H-imidazole-2(3H)-thione was converted using 3-chloro-5-(3-fluoropropoxy)-4-hydroxybenzaldehyde instead of 3-fluoro-4-hydroxy-5-methoxybenzaldehyde into the title compound (32 mg, 13 %) which was obtained as a red solid. MS m/e: 432.3 [M+H]⁺

### Example 124

### 2-[1-(3-Fluoro-5-(3-fluoropropoxy)-4-hydroxy-phenyl)-meth-(Z)-ylidene]-6-pyridin-4-yl-imidazo[2,1-b]thiazol-3-one

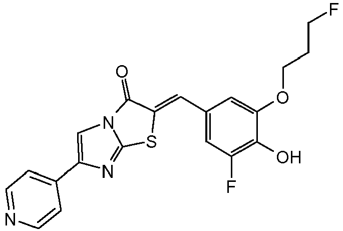

In analogy to the experimental procedure of example 9) 4-(pyridin-4-yl)-1H-imidazole-2(3H)-thione instead of 4-o-tolyl-1H-imidazole-2(3H)-thione was converted using 3-fluoro-5-(3-fluoropropoxy)-4-hydroxybenzaldehyde instead of 3-fluoro-4-hydroxy-5-methoxybenzaldehyde into the title compound (13 mg, 5 %) which was obtained as a brown solid. MS m/e: 416.2 [M+H]⁺

### Example 125

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide

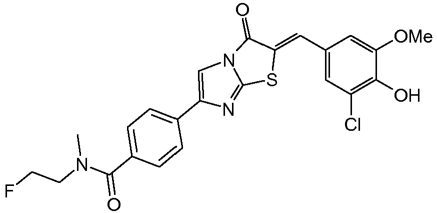

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 2-fluoro-N-methylethanamine hydrochloride instead of 2-fluoroethylamine hydrochloride into the title compound (45 mg, 36 %) which was obtained as a red solid. MS m/e: 488.2 [M+H]⁺

### Example 126

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N,N-dimethyl-benzamide

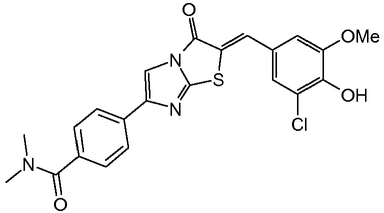

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using dimethylamine (2 M in tetrahydrofurane) instead of 2-fluoroethylamine hydrochloride into the title compound (43 mg, 38 %) which was obtained as a yellow solid. MS m/e: 456.2 [M+H]⁺

### Example 127

### 2-[1-(3-Chloro-4-hydroxy-5-methoxy-phenyl)-meth-(Z)-ylidene]-6-[4-(4-fluoropiperidine-1-carbonyl)-phenyl]-imidazo[2,1-b]thiazol-3-one

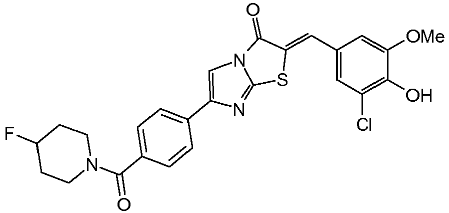

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 4-fluoropiperidine instead of 2-fluoroethylamine hydrochloride into the title compound (34 mg, 31%) which was obtained as a yellow solid. MS m/e: 514.3 [M+H]⁺

### Example 128

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-N-propyl-benzamide

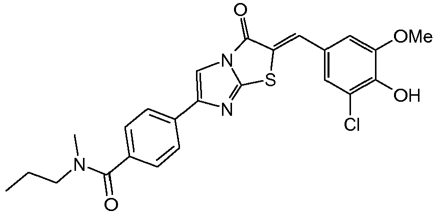

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using N-methylpropan-1-amine instead of 2-fluoroethylamine hydrochloride into the title compound (45 mg, 32 %) which was obtained as a red solid. MS m/e: 484.3 [M+H]⁺

### Example 129

### 2-[1-(3-Chloro-4-hydroxy-5-propoxy-phenyl)-meth-(Z)-ylidene]-6-pyrazin-2-yl-imidazo[2,1-b]thiazol-3-one

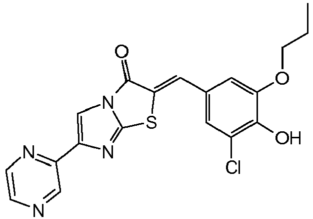

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(pyrazin-2-yl)ethanone hydrobromide instead of 3-bromo-5-(2-bromoacetyl)benzonitrile into the title compound (8 mg, 6 %) which was obtained as a yellow solid. MS m/e: 415.1 [M+H]⁺

### Example 130

### 2-[1-(3-Chloro-4-hydroxy-5-propoxy-phenyl)-meth-(Z)-ylidene]-6-pyrimidin-5-yl-imidazo[2,1-b]thiazol-3-one

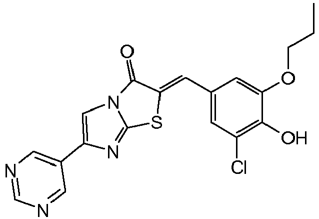

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-chloro-1-(pyrimidin-5-yl)ethanone instead of 3-bromo-5-(2-bromoacetyl)benzonitrile into the title compound (1 mg, 1 %) which was obtained as a yellow solid. MS m/e: 415.1 [M+H]⁺

### Example 131

### 2-[1-(3-Chloro-4-hydroxy-5-propoxy-phenyl)-meth-(Z)-ylidene]-6-thiophen-3-yl-imidazo[2,1-b]thiazol-3-one

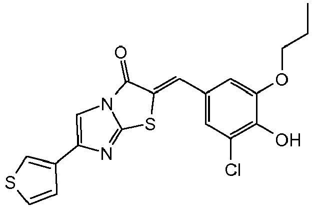

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(thiophen-3-yl)ethanone instead of 3-bromo-5-(2-bromoacetyl)benzonitrile into the title compound (51 mg, 32 %) which was obtained as a yellow solid. MS m/e: 419.1 [M+H]⁺

### Example 132

### 2-[1-(3-Chloro-4-hydroxy-5-propoxy-phenyl)-meth-(Z)-ylidene]-6-thiophen-2-yl-imidazo[2,1-b]thiazol-3-one

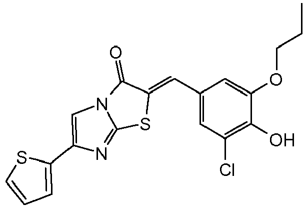

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(thiophen-2-yl)ethanone instead of 3-bromo-5-(2-bromoacetyl)benzonitrile into the title compound (58 mg, 37 %) which was obtained as a yellow solid. MS m/e: 419.1 [M+H]⁺

### Example 133

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-benzamide

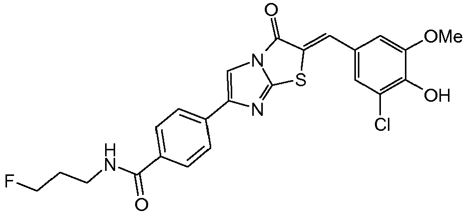

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoropropan-1-amine hydrochloride instead of 2-fluoroethylamine hydrochloride into the title compound (39 mg, 31 %) which was obtained as a red solid. MS m/e: 488.2 [M+H]⁺

### Example 134

### 2-[1-(3-Chloro-4-hydroxy-5-methoxy-phenyl)-meth-(Z)-ylidene]-6-[4-(3-fluoro-azetidine-1-carbonyl)-phenyl]-imidazo[2,1-b]thiazol-3-one

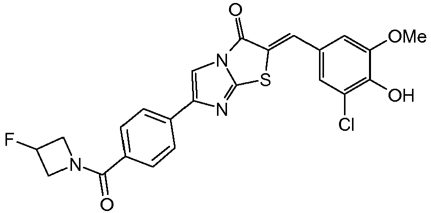

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoroazetidine hydrochloride instead of 2-fluoroethylamine hydrochloride into the title compound (49 mg, 33 %) which was obtained as a red solid. MS m/e: 486.3 [M+H]⁺

### Example 135

### 2-[1-(3-Chloro-4-hydroxy-5-propoxy-phenyl)-meth-(Z)-ylidene]-6-(2,4-dimethylthiophen-3-yl)-imidazo[2,1-b]thiazol-3-one

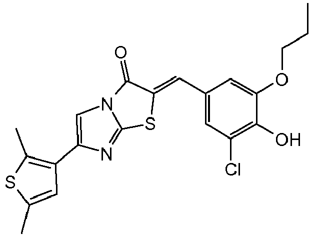

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(2,4-dimethylthiophen-3-yl)ethanone instead of 3-bromo-5-(2-bromoacetyl)benzonitrile into the title compound (34 mg, 23 %) which was obtained as a yellow solid. MS m/e: 447.1 [M+H]⁺

### Example 136

### 2-[1-(3-Chloro-4-hydroxy-5-propoxy-phenyl)-meth-(Z)-ylidene]-6-pyridin-2-yl-imidazo[2,1-b]thiazol-3-one

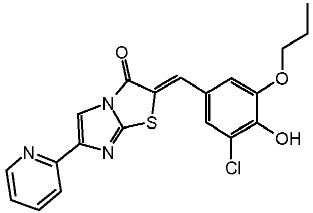

In analogy to the experimental procedure of example 85) (Z)-2-amino-5-(3-chloro-4-hydroxy-5-propoxybenzylidene)thiazol-4(5H)-one instead of (Z)-2-amino-5-(3-chloro-4-hydroxy-5-methoxybenzylidene)thiazol-4(5H)-one was converted using 2-bromo-1-(pyridin-2-yl)ethanone hydrobromide instead of 3-bromo-5-(2-bromoacetyl)benzonitrile into the title compound (19 mg, 13 %) which was obtained as a yellow solid. MS m/e: 414.2 [M+H]⁺

### Example 137

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide

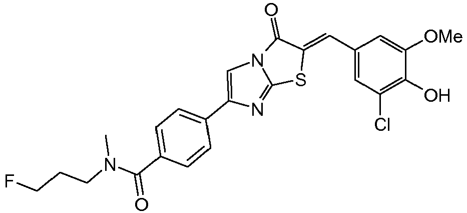

In analogy to the experimental procedure of example 105) (Z)-4-(2-(3-chloro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoro-N-methylpropan-1-amine hydrochloride instead of 2-fluoroethylamine hydrochloride into the title compound (49 mg, 38 %) which was obtained as a yellow solid. MS m/e: 502.2 [M+H]⁺

### Example 138

### 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzoic acid

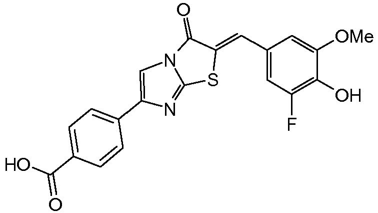

In analogy to the experimental procedure of example 85) (Z)-2-amino-5-(3-fluoro-4-hydroxy-5-methoxybenzylidene)thiazol-4(5H)-one instead of (Z)-2-amino-5-(3-chloro-4-hydroxy-5-methoxybenzylidene)thiazol-4(5H)-one was converted using 4-(2-bromoacetyl)benzoic acid instead of 3-bromo-5-(2-bromoacetyl)benzonitrile into the title compound (497 mg, 53 %) which was obtained as a yellow solid. MS m/e: 413.2 [M+H]⁺

### Example 139

### 6-[4-(3-Fluoro-azetidine-1-carbonyl)-phenyl]-2-[1-(3-fluoro-4-hydroxy-5-methoxy-phenyl)-meth-(Z)-ylidene]-imidazo[2,1-b]thiazol-3-one

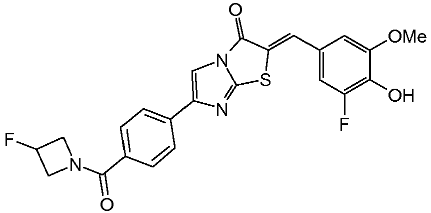

In analogy to the experimental procedure of example 105) (Z)-4-(2-(3-fluoro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoroazetidine hydrochloride instead of 2-fluoroethylamine hydrochloride into the title compound (72 mg, 59 %) which was obtained as a yellow solid. MS m/e: 470.2 [M+H]⁺

### Example 140

### 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-benzamide

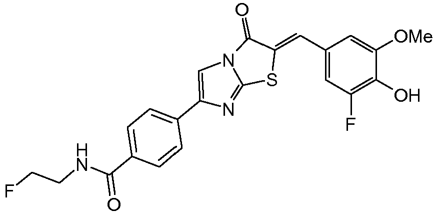

In analogy to the experimental procedure of example 105) (Z)-4-(2-(3-fluoro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted into the title compound (40 mg, 33 %) which was obtained as a yellow solid. MS m/e: 458.3 [M+H]⁺

### Example 141

### 3-{2-[1-(3-FIuoro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide

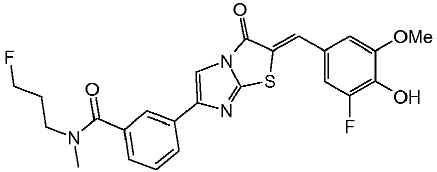

In analogy to the experimental procedure of example 105) (Z)-4-(2-(3-fluoro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoro-N-methylpropan-1-amine hydrochloride instead of 2-fluoroethanamine hydrochloride into the title compound (53 mg, 37 %) which was obtained as a yellow solid. MS m/e: 502.2 [M+H]⁺

### Example 142

### 2-[1-(3-Chloro-4-hydroxy-5-methoxy-phenyl)-meth-(Z)-ylidene]-6-[3-(3-fluoro-azetidine-1-carbonyl)-phenyl]-imidazo[2,1-b]thiazol-3-one

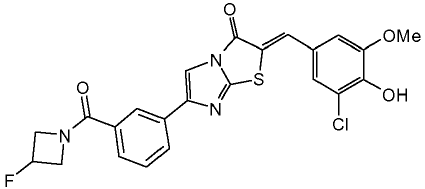

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using 3-fluoroazetidine hydrochloride instead of 2-fluoroethylamine hydrochloride into the title compound (73 mg, 52 %) which was obtained as a yellow solid. MS m/e: 486.2 [M+H]⁺

### Example 143

### 2-[1-(3-Chloro-4-hydroxy-5-methoxy-phenyl)-meth-(Z)-ylidene]-6-[3-(4-fluoro-piperidine-1-carbon-phenyl]-imidazo[2,1-b]thiazol-3-one

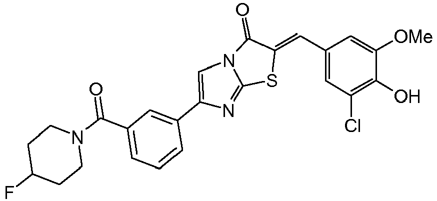

In analogy to the experimental procedure of example 105) of (Z)-3-(2-(3-chloro-4-hydroxy-5-methoxybenzylidene)-3-oxo-2,3-dihydroimidazo[2,1-b]thiazol-5-yl)benzoic acid was converted using 4-fluoropiperidine hydrochloride instead of 2-fluoroethylamine hydrochloride into the title compound (52 mg, 38 %) which was obtained as a yellow solid. MS m/e: 514.3 [M+H]⁺

### Example 144

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide

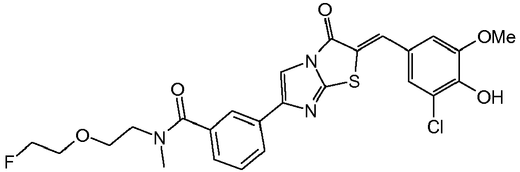

In analogy to the experimental procedure of example 105) of (Z)-3-(2-(3-chloro-4-hydroxy-5-methoxybenzylidene)-3-oxo-2,3-dihydroimidazo[2,1-b]thiazol-5-yl)benzoic acid was converted using 2-(2-fluoroethoxy)-N-methylethanamine hydrochloride instead of 2-fluoroethylamine hydrochloride into the title compound (38 mg, 26 %) which was obtained as a yellow solid. MS m/e: 532.1 [M+H]⁺

### Example 145

### 2-[1-(3-Chloro-4-hydroxy-5-methoxy-phenyl)-meth-(Z)-ylidene]-6-(2-chloro-pyridin-4-yl)-imidazo[2,1-b]thiazol-3-one

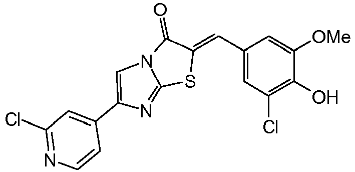

In analogy to the experimental procedure of example 85) (Z)-2-amino-5-(3-chloro-4-hydroxy-5-methoxybenzylidene)thiazol-4(5H)-one was converted using 2-bromo-1-(2-chloropyridin-4-yl)ethanone instead of 3-bromo-5-(2-bromoacetyl)benzonitrile into the title compound (214 mg, 73 %) which was obtained as a yellow solid. MS m/e: 420.0 [M+H]⁺

### Example 146

### 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-N-methyl-benzamide

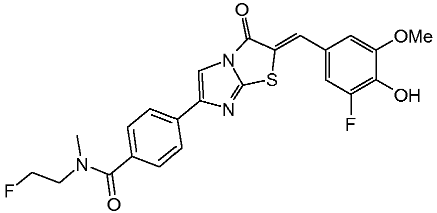

In analogy to the experimental procedure of example 105) (Z)-4-(2-(3-fluoro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 2-fluoro-N-methylethanamine hydrochloride instead of 2-fluoroethanamine hydrochloride into the title compound (69 mg, 66 %) which was obtained as a yellow solid. MS m/e: 472.3 [M+H]⁺

### Example 147

### 4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoropropoxy)-N-methyl-benzamide

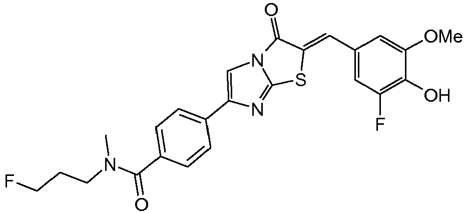

In analogy to the experimental procedure of example 105) (Z)-4-(2-(3-fluoro-4-hydroxy-5-methoxybenzylidene)-3-oxo-2,3-dihydroimidazo[2,1-b]thiazol-5-yl)benzoic acid instead of (Z)-3-(2-(3-chloro-4-hydroxy-5-methoxybenzylidene)-3-oxo-2,3-dihydroimidazo[2,1-b]thiazol-5-yl)benzoic acid was converted using 3-fluoro-N-methylpropan-1-amine hydrochloride instead of 2-fluoroethanamine hydrochloride into the title compound (49 mg, 46 %) which was obtained as a yellow solid. MS m/e: 486.4 [M+H]⁺

### Example 148

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 2-fluoro-ethyl ester

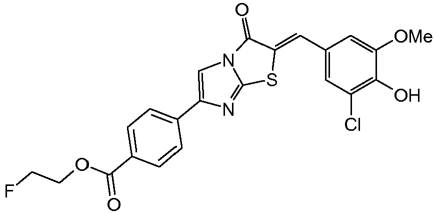

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using 2-fluoroethanol instead of 2-fluoroethanamine hydrochloride into the title compound (26 mg, 19 %) which was obtained as an orange solid. MS m/e: 475.1 [M+H]⁺

### Example 149

### 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 3-fluoro-propyl ester

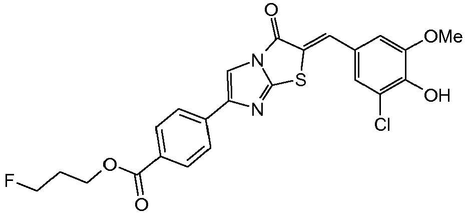

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using 3-fluoropropan-1-ol instead of 2-fluoroethanamine hydrochloride into the title compound (15 mg, 12 %) which was obtained as an orange solid. MS m/e: 489.3 [M+H]⁺

### Example 150

### [2-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoylamino)-ethyl]-carbamic acid tert-butyl ester

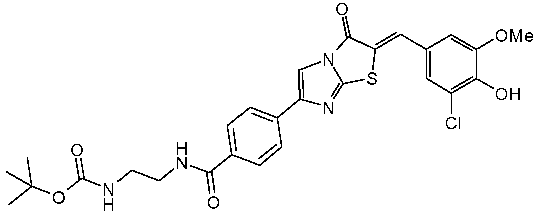

In analogy to the experimental procedure of example 105) (Z)-3-(2-(3-chloro-4-hydroxy-5-methoxybenzylidene)-3-oxo-2,3-dihydroimidazo[2,1-b]thiazol-5-yl)benzoic acid was converted using tert-butyl 2-aminoethylcarbamate instead of 2-fluoroethanamine hydrochloride into the title compound (101 mg, 38 %) which was obtained as a yellow solid. MS m/e: 571.2 [M+H]⁺

### Example 151

### N-(2-Amino-ethyl)-4-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide

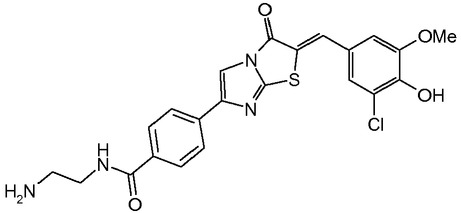

To a suspension of (Z)-tert-butyl 2-(4-(2-(3-chloro-4-hydroxy-5-methoxybenzylidene)-3-oxo-2,3-dihydroimidazo[2,1-b]thiazol-5-yl)benzamido)ethylcarbamate (95 mg, 166 µmol) in tetrahydrofurane (2 mL) was added under an atmosphere of nitrogen aqueous hydrochloric acid (4 M in dioxane, 416 µL, 1.66 mmol). The reaction mixture was stirred at ambient temperature for 8 h. The suspension was centrifuged and the upper layer was pipetted off Drying of the residue in vacuo afforded the title compound (76 mg, 90 %) as a yellow solid. MS m/e: 469.07 [M-H]⁻

### Example 152

### 3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzenesulfonyl fluoride

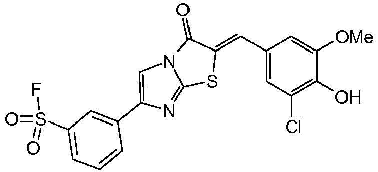

In analogy to the experimental procedure of example 85) (Z)-2-amino-5-(3-chloro-4-hydroxy-5-methoxybenzylidene)thiazol-4(5H)-one was converted using 3-(2-bromoacetyl)benzene-1-sulfonyl fluoride instead of 3-bromo-5-(2-bromoacetyl)benzonitrile into the title compound (82 mg, 28 %) which was obtained as a yellow solid. MS m/e: 467.2 [M+H]⁺

### Example 153

### 2-[1-[3-Chloro-5-(3-fluoro-propoxy)-4-hydroxy-phenyl]-meth-(Z)-ylidene]-6-(2,6-dimethyl-pyridin-4-yl)-imidazo[2,1-b]thiazol-3-one

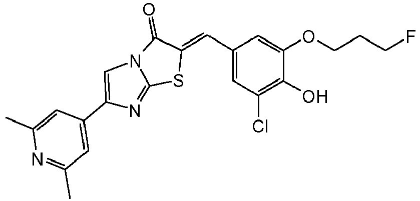

In analogy to the experimental procedure of example 9) 4-(2,6-dimethylpyridin-4-yl)-1H-imidazole-2(3H)-thione instead of 4-o-tolyl-1H-imidazole-2(3H)-thione was converted using 3-chloro-5-(3-fluoropropoxy)-4-hydroxybenzaldehyde instead of 3-fluoro-4-hydroxy-5-methoxybenzaldehyde into the title compound (83 mg, 22 %) which was obtained as a red solid. MS m/e: 460.5 [M+H]⁺

### Example 154

### 3-{2-[1-[3-Chloro-5-(3-fluoro-propoxy)-4-hydroxy-phenyl]-meth-(Z)-ylidene]-3-oxo-2,3-dihydro-imidazo[2,1-b]thiazol-6-yl}-benzonitrile

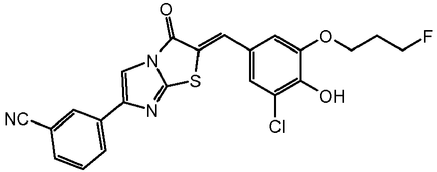

In analogy to the experimental procedure of example 9) 3-(2-thioxo-2,3-dihydro-1H-imidazol-4-yl)benzonitrile instead of 4-o-tolyl-1H-imidazole-2(3H)-thione was converted using 3-chloro-5-(3-fluoropropoxy)-4-hydroxybenzaldehyde instead of 3-fluoro-4-hydroxy-5-methoxybenzaldehyde into the title compound (35 mg, 8 %) which was obtained as a yellow solid. MS m/e: 456.5 [M+H]⁺

## Claims

1. A compound of formula 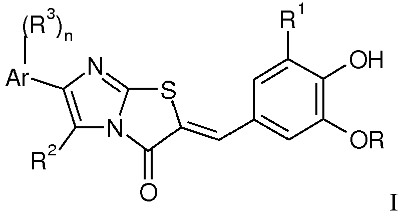 wherein
Ar is phenyl, pyridinyl, 2,3-dihydro-benzo[1,4]dioxinyl, 1,3-dihydro-indol-2-one, pyrazinyl, isoxazol-3-yl, imidazolyl, thiophenyl or pyrimidinyl;
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
R³ is hydrogen, halogen, C₁₋₇-alkyl, C₁₋₇-alkyl substituted by halogen, C₁₋₇-alkoxy, C₁₋₇-alkoxy substituted by halogen, O(CH₂)ₘO(CH₂)ₘO-C₁₋₇-alkyl substituted by halogen, cyano, C₁₋₇-alkoxy substituted by hydroxy, C₁₋₇-alkenyloxy, C(O)OH, heterocycloalkyl selected from morpholinyl, pyrrolidinyl or pyrrolidin-2-one, or is heteroaryl selected from imidazolyl substituted by C₁₋₇-alkyl, or is NR'R" and R'/R" are independently from each other hydrogen or C₁₋₇-alkyl or -C(O)- C₁₋₇-alkyl; or is -C(O)NR⁴R⁵ and R⁴ is hydrogen or C₁₋₇-alkyl and R⁵ is hydrogen, C₁₋₇-alkyl, C₁₋₇-alkenyl, -(CH₂)ₘO-C₁₋₇-alkyl substituted by halogen, C₁₋₇-alkyl substituted by halogen, -(CH₂)ₙ-phenyl optionally substituted by halogen, -CH₂)ₘNHC(O)-C₁₋₇-alkyl, or -(CH₂)ₘNH₂, or R⁴ and R⁵ may form together with the N-atom to which they are attached a piperidine or azetidine ring, which may be substituted by halogen; or is -C(O)O-C₁₋₇-alkyl substituted by halogen;
n is 1 or 2;
m is 1, 2 or 3;
or a pharmaceutically acceptable acid addition salt, a racemic mixture or its corresponding enantiomer and/or optical isomers thereof.

2. A compound of formula IA according to claim 1 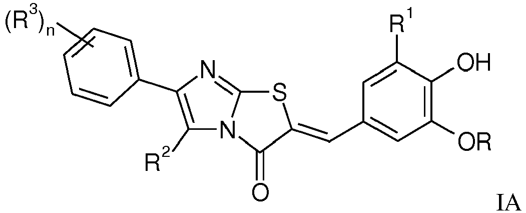 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
R³ is hydrogen, halogen, C₁₋₇-alkyl, C₁₋₇-alkyl substituted by halogen, C₁₋₇-alkoxy, C₁₋₇-alkoxy substituted by halogen, O(CH₂)ₘO(CH₂)ₘO-C₁₋₇-alkyl substituted by halogen, cyano, C₁₋₇-alkoxy substituted by hydroxy, C₁₋₇-alkenyloxy, C(O)OH, heterocycloalkyl selected from morpholinyl, pyrrolidinyl or pyrrolidin-2-one, or is heteroaryl selected from imidazolyl substituted by C₁₋₇-alkyl, or is NR'R" and R'/R" are independently from each other hydrogen or C₁₋₇-alkyl or -C(O)-C₁₋₇-alkyl; or is -C(O)NR⁴R⁵ and
R⁴ is hydrogen or C₁₋₇-alkyl and
R⁵ is hydrogen, C₁₋₇-alkyl, C₁₋₇-alkenyl, -(CH2)ₘO-C₁₋₇-alkyl substituted by halogen, C₁₋₇-alkyl substituted by halogen, -(CH₂)ₙ-phenyl optionally substituted by halogen, -CH₂)ₘNHC(O)-C₁₋₇-alkyl, or -(CH₂)ₘNH₂, or
R⁴ and R⁵ may form together with the N-atom to which they are attached a piperidine or azetidine ring, which may be substituted by halogen; or is -C(O)O- C₁₋₇-alkyl substituted by halogen;
n is 1 or 2;
m is 1, 2 or 3;
or a pharmaceutically acceptable acid addition salt, a racemic mixture or its corresponding enantiomer and/or optical isomers thereof.

3. A compound of formula IA according to any one of claims 1 or 2, wherein the compounds are
6-(4-Chloro-phenyl)-2-[1-(4-hydroxy-3,5-dimethoxy-phenyl)-meth-(Z)-ylidene]-imidazo[2,1-b]thiazol-3-one
6-(4-Chloro-phenyl)-2-[1-(4-hydroxy-3-methoxy-phenyl)-meth-(Z)-ylidene]-imidazo[2,1-b]thiazol-3-one
6-(4-Chloro-phenyl)-2-[1-(3-fluoro-4-hydroxy-5-methoxy-phenyl)-meth-(Z)-ylidene]-imidazo[2,1-b]thiazol-3-one
6-(4-Chloro-phenyl)-2-[1-(3-ethoxy-4-hydroxy-phenyl)-meth-(Z)-ylidene]-imidazo[2,1-b]thiazol-3-one
6-(4-Chloro-phenyl)-2-[1-(3-chloro-4-hydroxy-5-methoxy-phenyl)-meth-(Z)-ylidene]-imidazo[2,1-b]thiazol-3-one
6-(4-Chloro-phenyl)-2-[1-(3-bromo-4-hydroxy-5-methoxy-phenyl)-meth-(Z)-ylidene]-imidazo[2,1-b]thiazol-3-one
6-(4-Chloro-phenyl)-2-[1-(3,4-dihydroxy-5-methoxy-phenyl)-meth-(Z)-ylidene]-imidazo[2,1-b]thiazol-3-one
2-[1-(3-Fluoro-4-hydroxy-5-methoxy-phenyl)-meth-(Z)-ylidene]-6-o-tolyl-imidazo[2,1-b]thiazol-3-one
2-[1-(3-Chloro-4-hydroxy-5-methoxy-phenyl)-meth-(Z)-ylidene]-6-o-tolyl-imidazo[2,1-b]thiazol-3-one
2-[1-(4-hydroxy-3-methoxy-phenyl)-meth-(Z)-ylidene]-6-o-tolyl-imidazo[2,1-b]thiazol-3-one 2-[1-(3-Chloro-4-hydroxy-5-methoxy-phenyl)-meth-(Z)-ylidene]-6-(4-methoxy-phenyl)-5-methyl-imidazo[2,1-b]thiazol-3-one
2-[1-(3-Fluoro-4-hydroxy-5-methoxy-phenyl)-meth-(Z)-ylidene]-6-(4-methoxy-phenyl)-5-methyl-imidazo[2,1-b]thiazol-3-one
2-[1-(4-Hydroxy-3,5-dimethoxy-phenyl)-meth-(Z)-ylidene]-6-(3-trifluoromethoxy-phenyl)-imidazo[2,1-b]thiazol-3-one
2-[1-(4-Hydroxy-3,5-dimethoxy-phenyl)-meth-(Z)-ylidene]-6-(3-trifluoromethyl-phenyl)-imidazo[2,1-b]thiazol-3-one
2-[1-(4-Hydroxy-3,5-dimethoxy-phenyl)-meth-(Z)-ylidene]-6-m-tolyl-imidazo[2,1-b]thiazol-3-one
6-(3-Chloro-phenyl)-2-[1-(4-hydroxy-3,5-dimethoxy-phenyl)-meth-(Z)-ylidene]-imidazo[2,1-b]thiazol-3-one
2-[1-(3-Chloro-4-hydroxy-5-methoxy-phenyl)-meth-(Z)-ylidene]-6-(3-trifluoromethoxy-phenyl)-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide
2-[1-(3-Chloro-4-hydroxy-5-methoxy-phenyl)-meth-(Z)-ylidene]-6-(3-trifluoromethyl-phenyl)-imidazo[2,1-b]thiazol-3-one
6-(3-Chloro-phenyl)-2-[1-(3-chloro-4-hydroxy-5-methoxy-phenyl)-meth-(Z)-ylidene]-imidazo[2,1-b]thiazol-3-one
6-(4-Chloro-phenyl)-2-[1-(3-ethoxy-4-hydroxy-5-methoxy-phenyl)-meth-(Z)-ylidene]-imidazo[2,1-b]thiazol-3-one
6-(4-Chloro-phenyl)-2-[1-(3-(2-fluoro-ethoxy)-4-hydroxy-5-methoxy-phenyl)-meth-(Z)-ylidene]-imidazo[2,1-b]thiazol-3-one
2-[1-(3-Chloro-4-hydroxy-5-methoxy-phenyl)-meth-(Z)-ylidene]-6-(3-fluoro-4-trifluoromethyl-phenyl)-imidazo[2,1-b]thiazol-3-one
2-[1-(3-Hydroxy-3,5-dimethoxy-phenyl)-meth-(Z)-ylidene]-6-(3-fluoro-4-trifluoromethyl-phenyl)-imidazo[2,1-b]thiazol-3-one
6-(4-Chloro-phenyl)-2-[1-(4-hydroxy-3-isopropoxy-5-methoxy-phenyl)-meth-(Z)-ylidene]-imidazo[2,1-b]thiazol-3-one
6-(4-Chloro-phenyl)-2-[1-(4-hydroxy-3-methoxy-5-propoxy-phenyl)-meth-(Z)-ylidene]-imidazo[2,1-b]thiazol-3-one
6-(4-Chloro-phenyl)-2-[1-(4-hydroxy-3-methoxy-5-methyl-phenyl)-meth-(Z)-ylidene]-imidazo[2,1-b]thiazol-3-one
2-[1-(4-Hydroxy-3-methoxy-5-propoxy-phenyl)-meth-(Z)-ylidene]-6-(3-trifluoromethoxy-phenyl)-imidazo[2,1-b]thiazol-3-one
2-[1-(4-Hydroxy-3-methoxy-5-isopropoxy-phenyl)-meth-(Z)-ylidene]-6-(3-tufluoromethoxy-phenyl)-imidazo[2,1-b]thiazol-3-one
2-[1-(3-Fluoro-4-hydroxy-5-methoxy-phenyl)-meth-(Z)-ylidene]-6-(3-trifluoromethoxy-phenyl)-imidazo[2,1-b]thiazol-3-one
2-[1-(4-Hydroxy-3-methoxy-phenyl)-meth-(Z)-ylidene]-6-(3-trifluoromethoxy-phenyl)-imidazo[2,1-b]thiazol-3-one
2-[1-(3-Bromo-4-hydroxy-5-methoxy-phenyl)-meth-(Z)-ylidene]-6-(3-trifluoromethoxy-phenyl)-imidazo[2,1-b]thiazol-3-one
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile
2-[1-(3-fluoro-4-hydroxy-5-methoxy-phenyl)-meth-(Z)-ylidene]-6-(3-chloro-phenyl)-imidazo[2,1-b]thiazol-3-one
2-[1-(3-Ethyl-4-hydroxy-5-methoxy-phenyl)-meth-(Z)-ylidene]-6-(3-trifluoromethoxy-phenyl)-imidazo[2,1-b]thiazol-3-one
2-[1-(3-fluoro-4-hydroxy-5-methoxy-phenyl)-meth-(Z)-ylidene]-6-(3-(trifluoromethyl)-phenyl)-imidazo[2,1-b]thiazol-3-one
2-[1-(3-fluoro-4-hydroxy-5-methoxy-phenyl)-meth-(Z)-ylidene]-6-(4-fluoro-3-(trifluoromethyl)-phenyl)-imidazo[2,1-b]thiazol-3-one
2-[1-(3-fluoro-4-hydroxy-5-methoxy-phenyl)-meth-(Z)-ylidene]-6-phenyl-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile
3-{2-[1-(3-Bromo-4-hydroxy-5-methoxy-phenyl)-meth-(Z)-ylidene]-3-oxo-2,3-dihydro-imidazo[2,1-b]thiazol-6-yl}-benzonitrile
2-[1-(3-bromo-4-hydroxy-5-methoxy-phenyl)-meth-(Z)-ylidene]-6-(4-fluoro-3-(trifluoromethyl)-phenyl)-imidazo[2,1-b]thiazol-3-one
2-[1-(3-Fluoro-4-hydroxy-5-methoxy-phenyl)-meth-(Z)-ylidene]-6-(3-methoxy-phenyl)-imidazo[2,1-b]thiazol-3-one
2-[1-(3-Chloro-4-hydroxy-5-methoxy-phenyl)-meth-(Z)-ylidene]-6-(3,5-difluoro-phenyl)-imidazo[2,1-b]thiazol-3-one
3-{2-[1-(4-Hydroxy-3,5-dimethoxy-phenyl)-meth-(Z)-ylidene]-3-oxo-2,3-dihydro-imidazo[2,1-b]thiazol-6-yl}-benzonitrile
3-{2-[1-(4-Hydroxy-3-methoxy-5-isopropoxyphenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-m-tolyl-imidazo[2,1-b]thiazol-3-one
2-[1-(3-Chloro-4-hydroxy-5-methoxy-phenyl)-meth-(Z)-ylidene]-6-(2-fluoro-phenyl)-imidazo[2,1-b]thiazol-3-one
2-[1-(3-Chloro-4-hydroxy-5-methoxy-phenyl)-meth-(Z)-ylidene]-6-(3-ethyl-phenyl)-imidazo[2,1-b]thiazol-3-one
2-[1-(3-Chloro-4-hydroxy-5-methoxy-phenyl)-meth-(Z)-ylidene]-6-(2-methoxy-phenyl)-imidazo[2,1-b]thiazol-3-one
2-[1-(3-Chloro-4-hydroxy-5-methoxy-phenyl)-meth-(Z)-ylidene]-6-(2,5-difluoro-phenyl)-imidazo[2,1-b]thiazol-3-one
2-[1-(3-Chloro-4-hydroxy-5-methoxy-phenyl)-meth-(Z)-ylidene]-6-(3-fluoro-phenyl)-imidazo[2,1-b]thiazol-3-one
2-[1-(3-Fluoro-4-hydroxy-5-methoxy-phenyl)-meth-(Z)-ylidene]-6-(3-fluoro-phenyl)-imidazo[2,1-b]thiazol-3-one
2-[1-(4-Hydroxy-3,5-dimethoxy-phenyl)-meth-(Z)-ylidene]-6-(3-fluoro-phenyl)-imidazo[2,1-b]thiazol-3-one
2-[1-(3-Chloro-4-hydroxy-5-methoxy-phenyl)-meth-(Z)-ylidene]-6-phenyl-imidazo[2,1-b]thiazol-3-one
2-[1-(3-Chloro-4-hydroxy-5-methoxy-phenyl)-meth-(Z)-ylidene]-6-(3-methoxy-phenyl)-imidazo[2,1-b]thiazol-3-one
2-[1-(3-Chloro-4-hydroxy-5-methoxy-phenyl)-meth-(Z)-ylidene]-6-(3-(difluoromethoxy)-phenyl)-imidazo[2,1-b]thiazol-3-one
(Z)-3-(2-(3-fluoro-4-hydroxy-5-methoxybenzylidene)-3-oxo-2,3-dihydroimidazo[2,1-b]thiazol-5-yl)benzonitrile
2-[1-(3-Chloro-4-hydroxy-5-methoxy-phenyl)-meth-(Z)-ylidene]-6-(3-(chloromethyl)phenyl)-imidazo[2,1-b]thiazol-3-one
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-(3-(2-fluoroethoxy)phenyl)-imidazo[2,1-b]thiazol-3-one
2-[1-(3-Chloro-4-hydroxy-5-methoxy-phenyl)-meth-(Z)-ylidene]-6-(4-(2-fluoroethoxy)phenyl)-imidazo[2,1-b]thiazol-3-one
2-[1-(3-Chloro-4-hydroxy-5-methoxy-phenyl)-meth-(Z)-ylidene]-6-(2-(2-fluoroethoxy)phenyl)-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-(4-(difluoromethoxy)phenyl)-imidazo[2,1-b]thiazol-3-one
2-[1-(3-Chloro-4-hydroxy-5-methoxy-phenyl)-meth-(Z)-ylidene]-6-(3-morpholinophenyl)-imidazo[2,1-b]thiazol-3-one
2-[1-(3-Chloro-4-hydroxy-5-methoxy-phenyl)-meth-(Z)-ylidene]-6-(4-(pyrrolidin-1-yl)phenyl)-imidazo[2,1-b]thiazol-3-one
2-[1-(3-Chloro-4-hydroxy-5-methoxy-phenyl)-meth-(Z)-ylidene]-6-(4-(diethylamino)phenyl)-imidazo[2,1-b]thiazol-3-one
2-[1-(3-Chloro-4-hydroxy-5-methoxy-phenyl)-meth-(Z)-ylidene]-6-(4-morpholinophenyl)-imidazo[2,1-b]thiazol-3-one
2-[1-(3-Chloro-4-hydroxy-5-methoxy-phenyl)-meth-(Z)-ylidene]-6-(4-(dimethylamino)phenyl)-imidazo[2,1-b]thiazol-3-one
2-[1-(3-Chloro-4-hydroxy-5-methoxy-phenyl)-meth-(Z)-ylidene]-6-[3-(2-oxo-pyrrolidin-1-yl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid
2-[1-(3-Chloro-4-hydroxy-5-methoxy-phenyl)-meth-(Z)-ylidene]-6-(3,4-dimethoxy-phenyl)-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzamide
N-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-phenyl)-acetamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid
2-[1-(3-Chloro-4-hydroxy-5-methoxy-phenyl)-meth-(Z)-ylidene]-6-(2,6-difluoro-phenyl)-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide
N-Allyl-3-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-propyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoro-propyl)-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(3-fluoro-phenyl)-ethyl]-N-methyl-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(2-fluoro-ethyl)-N-methyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N,N-dimethyl-benzamide
2-[1-(3-Chloro-4-hydroxy-5-methoxy-phenyl)-meth-(Z)-ylidene]-6-[4-(4-fluoro-piperidine-1-carbonyl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-methyl-N-propyl-benzamide
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-benzamide
2-[1-(3-Chloro-4-hydroxy-5-methoxy-phenyl)-meth-(Z)-ylidene]-6-[4-(3-fluoro-azetidine-1-carbonyl)-phenyl]-imidazo[2,1-b]thiazol-3-one
4-{2-[1-(3-Chloro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-benzoic acid
6-[4-(3-Fluoro-azetidine-1-carbonyl)-phenyl]-2-[1-(3-fluoro-4-hydroxy-5-methoxy-phenyl)-meth-(Z)-ylidene]-imidazo[2,1-b]thiazol-3-one
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-benzamide
3-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(3-fluoropropoxy)-N-methyl-benzamide
2-[1-(3-Chloro-4-hydroxy-5-methoxy-phenyl)-meth-(Z)-ylidene]-6-[3-(3-fluoro-azetidine-1-carbonyl)-phenyl]-imidazo[2,1-b]thiazol-3-one
2-[1-(3-Chloro-4-hydroxy-5-methoxy-phenyl)-meth-(Z)-ylidene]-6-[3-(4-fluoro-piperidine-1-carbon-phenyl]-imidazo[2,1-b]thiazol-3-one
3-{2-[1-(3-Chloro-4-hydroxy-5-methoxy-phenyl)-meth-(Z)-ylidene]-3-oxo-2,3-dihydro-imidazo[2,1-b]thiazol-6-yl}-N-[2-(2-fluoro-ethoxy)-ethyl]-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoroethoxy)-N-methyl-benzamide
4-{2-[1-(3-Fluoro-4-hydroxy-5-methoxy-phenyl)-meth-(Z)-ylidene]-3-oxo-2,3-dihydro-imidazo[2,1-b]thiazol-6-yl}-N-(2-fluoropropoxy)-N-methyl-benzamide 4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 2-fluoro-ethyl ester
4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoic acid 3-fluoro-propyl ester
[2-(4-{2-[1-(3-Chloro-4-hydroxy-5-methoxy-phenyl)-meth-(Z)-ylidene]-3-oxo-2,3-dihydro-imidazo[2,1-b]thiazol-6-yl}-benzoylamino)-ethyl]-carbamic acid tert-butyl ester
N-(2-Amino-ethyl)-4-{2-[1-(3-chloro-4-hydroxy-5-methoxy-phenyl)-meth-(Z)-ylidene]-3-oxo-2,3-dihydro-imidazo[2,1-b]thiazol-6-yl}-benzamide
3-{2-[1-(3-Chloro-4-hydroxy-5-methoxy-phenyl)-meth-(Z)-ylidene]-3-oxo-2,3-dihydro-imidazo[2,1-b]thiazol-6-yl}-benzenesulfonyl fluoride
3-{2-[1-[3-Chloro-5-(3-fluoro-propoxy)-4-hydroxy-phenyl]-meth-(Z)-ylidene]-3-oxo-2,3-dihydro-imidazo[2,1-b]thiazol-6-yl}-benzonitrile
3-Bromo-5-{2-[1-(3-chloro-4-hydroxy-5-methoxy-phenyl)-meth-(Z)-ylidene]-3-oxo-2,3-dihydro-imidazo[2,1-b]thiazol-6-yl}-benzonitrile
2-[1-(3-Chloro-4-hydroxy-5-methoxy-phenyl)-meth-(Z)-ylidene]-6-(3-{2-[2-(2-fluoro-ethoxy)-ethoxy]-ethoxy}-phenyl)-imidazo[2,1-b]thiazol-3-one or
2-[1-(3-Chloro-4-hydroxy-5-methoxy-phenyl)-meth-(Z)-ylidene]-6-(2-{2-[2-(2-fluoro-ethoxy)-ethoxy]-ethoxy}-phenyl)-imidazo[2,1-b]thiazol-3-one.

4. A compound of formula IB according to claim 1 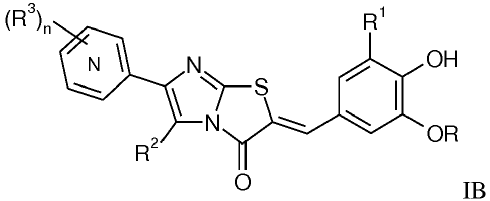 wherein the N-atom of the pyridinyl group may be in different positions, and wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
R³ is hydrogen, halogen, C₁₋₇-alkyl, C₁₋₇-alkyl substituted by halogen, C₁₋₇-alkoxy, C₁₋₇-alkoxy substituted by halogen, O(CH₂)ₘO(CH₂)ₘO-C₁₋₇-alkyl substituted by halogen, cyano, C₁₋₇-alkoxy substituted by hydroxy, C₁₋₇-alkenyloxy, C(O)OH, heterocycloalkyl selected from morpholinyl, pyrrolidinyl or pyrrolidin-2-one, or is heteroaryl selected from imidazolyl substituted by C₁₋₇-alkyl, or is NR'R" and R'/R" are independently from each other hydrogen or C₁₋₇-alkyl or -C(O)- C₁₋₇-alkyl; or is -C(O)NR⁴R⁵ and R⁴ is hydrogen or C₁₋₇-alkyl and R⁵ is hydrogen, C₁₋₇-alkyl, C₁₋₇-alkenyl, -(CH₂)ₘO-C₁₋₇-alkyl substituted by halogen, C₁₋₇-alkyl substituted by halogen, -(CH₂)ₙ-phenyl optionally substituted by halogen, -CH₂)ₘ,NHC(O)-C₁₋₇-alkyl, or -(CH₂)ₘNH₂, or R⁴ and R⁵ may form together with the N-atom to which they are attached a piperidine or azetidine ring, which may be substituted by halogen; or is -C(O)O-C₁₋₇-alkyl substituted by halogen;
n is 1 or 2;
m is 1, 2 or 3;
or a pharmaceutically acceptable acid addition salt, a racemic mixture or its corresponding enantiomer and/or optical isomers thereof.

5. A compound of formula IB according to any one of claims 1 or 4, wherein the compounds are
2-[1-(4-Hydroxy-3,5-dimethoxy-phenyl)-meth-(Z)-ylidene]-6-pyridin-4-yl-imidazo[2,1-b]thiazol-3-one
2-[1-(4-Hydroxy-3-methoxy-phenyl)-meth-(Z)-ylidene]-6-(2-methyl-pyridin-4-yl)-imidazo[2,1-b]thiazol-3-one
2-[1-(4-Hydroxy-3-methoxy-phenyl)-meth-(Z)-ylidene]-6-(2,6-dimethyl-pyridin-4-yl)-imidazo[2,1-b]thiazol-3-one
2-[1-(4-Hydroxy-3,5-dimethoxy-phenyl)-meth-(Z)-ylidene]-6-(2-methyl-pyridin-4-yl)-imidazo[2,1-b]thiazol-3-one
2-[1-(4-Hydroxy-3,5-dimethoxy-phenyl)-meth-(Z)-ylidene]-6-(2,6-dimethyl-pyridin-4-yl)-imidazo[2,1-b]thiazol-3-one
2-[1-(3-Fluoro-4-hydroxy-5-methoxy-phenyl)-meth-(Z)-ylidene]-6-(6-methoxy-pyridin-2-yl)-imidazo[2,1-b]thiazol-3-one
2-[1-(3-Chloro-4-hydroxy-5-methoxy-phenyl)-meth-(Z)-ylidene]-6-(2,6-dimethyl-pyridin-4-yl)-imidazo[2,1-b]thiazol-3-one
2-[1-(3-Fluoro-4-hydroxy-5-methoxy-phenyl)-meth-(Z)-ylidene]-6-(2,6-dimethyl-pyridin-4-yl)-imidazo[2,1-b]thiazol-3-one
2-[1-(3-Bromo-4-hydroxy-5-methoxy-phenyl)-meth-(Z)-ylidene]-6-(2,6-dimethyl-pyridin-4-yl)-imidazo[2,1-b]thiazol-3-one
2-[1-(3-Chloro-4-hydroxy-5-methoxy-phenyl)-meth-(Z)-ylidene]-6-(pyridin-4-yl)-imidazo[2,1-b]thiazol-3-one
2-[1-(3-Fluoro-4-hydroxy-5-methoxy-phenyl)-meth-(Z)-ylidene]-6-(pyridin-4-yl)-imidazo[2,1-b]thiazol-3-one
2-[1-(3-Bromo-4-hydroxy-5-methoxy-phenyl)-meth-(Z)-ylidene]-6-(pyridin-4-yl)-imidazo[2,1-b]thiazol-3-one
2-[1-(3-Fluoro-4-hydroxy-5-methoxy-phenyl)-meth-(Z)-ylidene]-6-(pyridin-3-yl)-imidazo[2,1-b]thiazol-3-one
2-[1-(3-Bromo-4-hydroxy-5-methoxy-phenyl)-meth-(Z)-ylidene]-6-(pyridin-3-yl)-imidazo[2,1-b]thiazol-3-one
2-[1-(3-Chloro-4-hydroxy-5-methoxy-phenyl)-meth-(Z)-ylidene]-6-(2-methylpyridin-4-yl)-imidazo[2,1-b]thiazol-3-one
2-[1-(3-Fluoro-4-hydroxy-5-methoxy-phenyl)-meth-(Z)-ylidene]-6-(2-methylpyridin-4-yl)-imidazo[2,1-b]thiazol-3-one
2-[1-(3-Bromo-4-hydroxy-5-methoxy-phenyl)-meth-(Z)-ylidene]-6-(2-methylpyridin-4-yl)-imidazo[2,1-b]thiazol-3-one
2-[1-(3-Chloro-4-hydroxy-5-methoxy-phenyl)-meth-(Z)-ylidene]-6-(pyridin-3-yl)-imidazo[2,1-b]thiazol-3-one
2-[1-(3-Chloro-4-hydroxy-5-methoxy-phenyl)-meth-(Z)-ylidene]-6-[6-(4-methyl-3H-imidazol-1-yl)-pyridin-3-yl]-imidazo[2,1-b]thiazol-3-one
2-[1-(3-Chloro-4-hydroxy-5-propoxy-phenyl)-meth-(Z)-ylidene]-6-(4-methyl-pyridin-3-yl)-imidazo[2,1-b]thiazol-3-one
2-[1-(3-Chloro-4-hydroxy-5-propoxy-phenyl)-meth-(Z)-ylidene]-6-pyridin-3-yl-imidazo[2,1-b]thiazol-3-one
2-[1-(3-Chloro-4-hydroxy-5-propoxy-phenyl)-meth-(Z)-ylidene]-6-pyridin-4-yl-imidazo[2,1-b]thiazol-3-one
2-[1-(3-Chloro-4-hydroxy-5-propoxy-phenyl)-meth-(Z)-ylidene]-6-[6-(4-methyl-imidazol-1-yl)-pyridin-3-yl]-imidazo[2,1-b]thiazol-3-one
2-[1-(3-Fluoro-4-hydroxy-5-propoxy-phenyl)-meth-(Z)-ylidene]-6-pyridin-4-yl-imidazo[2,1-b]thiazol-3-one
2-[1-(3-Chloro-5-(3-fluoropropoxy)-4-hydroxy-phenyl)-meth-(Z)-ylidene]-6-pyridin-4-yl-imidazo[2,1-b]thiazol-3-one
2-[1-(3-Fluoro-5-(3-fluoropropoxy)-4-hydroxy-phenyl)-meth-(Z)-ylidene]-6-pyridin-4-yl-imidazo[2,1-b]thiazol-3-one
2-[1-(3-Chloro-4-hydroxy-5-propoxy-phenyl)-meth-(Z)-ylidene]-6-pyridin-2-yl-imidazo[2,1-b]thiazol-3-one
2-[1-(3-Chloro-4-hydroxy-5-methoxy-phenyl)-meth-(Z)-ylidene]-6-(2-chloro-pyridin-4-yl)-imidazo[2,1-b]thiazol-3-one or
2-[1-[3-Chloro-5-(3-fluoro-propoxy)-4-hydroxy-phenyl]-meth-(Z)-ylidene]-6-(2,6-dimethyl-pyridin-4-yl)-imidazo[2,1-b]thiazol-3-one.

6. A compound of formula IC according to claim 1, 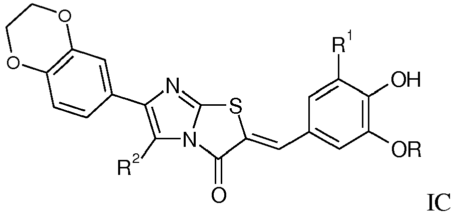 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

7. A compound of any one of claims 1 or 6, wherein the compound is 2-[1-(3-Chloro-4-hydroxy-5-methoxy-phenyl)-meth-(Z)-ylidene]-6-(2,3-dihydro-benzo[1,4]dioxin-6-yl)-imidazo[2,1-b]thiazol-3-one.

8. A compound of formula ID according to claim 1, 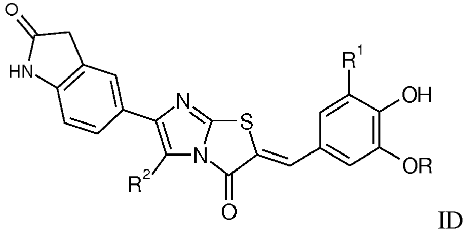 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

9. A compound of formula ID according to any one of claims 1 or 8, wherein the compound is
5-{2-[1-(3-Chloro-4-hydroxy-5-methoxy-phenyl)-meth-(Z)-ylidene]-3-oxo-2,3-dihydro-imidazo[2,1-b]thiazol-6-yl}-1,3-dihydro-indol-2-one.

10. A compound of formula IE according to claim 1, 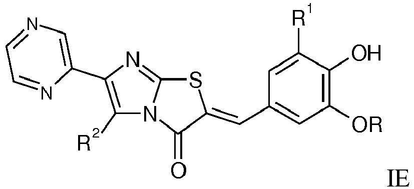 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

11. A compound of formula IE according to any one of claims 1 or 10 2-[1-(3-Chloro-4-hydroxy-5-propoxy-phenyl)-meth-(Z)-ylidene]-6-pyrazin-2-yl-imidazo[2,1-b]thiazol-3-one.

12. A compound of formula IF according to claim 1, 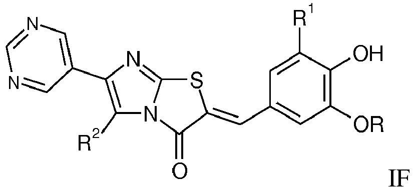 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

13. A compound of formula IF according to any one of claims 1 or 12, wherein the compound is
2-[1-(3-Chloro-4-hydroxy-5-propoxy-phenyl)-meth-(Z)-ylidene]-6-pyrimidin-5-yl-imidazo[2,1-b]thiazol-3-one.

14. A compound of formula IG according to claim 1, 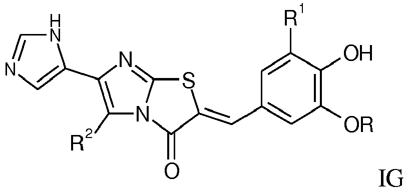 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

15. A compound of formula IG according to any one of claims 1 or 14, wherein the compound is
2-[1-(3-Chloro-4-hydroxy-5-propoxy-phenyl)-meth-(Z)-ylidene]-6-(3H-imidazol-4-yl)-imidazo[2,1-b]thiazol-3-one.

16. A compound of formula IH according to claim 1, 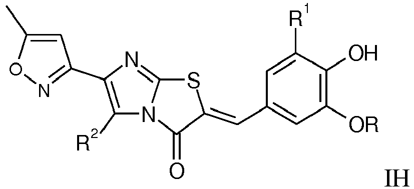 wherein
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

17. A compound of formula IH according to any one of claims 1 or 16, wherein the compound is
2-[1-(3-Chloro-4-hydroxy-5-methoxy-phenyl)-meth-(Z)-ylidene]-6-(5-methyl-isoxazol-3-yl)-imidazo[2,1-b]thiazol-3-one.

18. A compound of formula IJ according to claim 1, 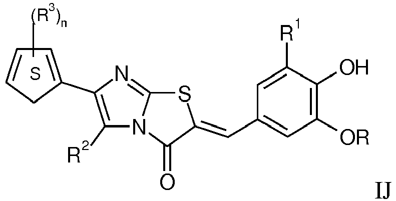 wherein
R³ is hydrogen or C₁₋₇-alkyl
n is 1 or 2
R is C₁₋₇-alkyl or C₁₋₇-alkyl substituted by halogen;
R¹ is hydrogen, halogen, hydroxy, C₁₋₇-alkoxy, C₁₋₇-alkyl and C₁₋₇-alkoxy substituted by halogen;
R² is hydrogen, C₁₋₇-alkyl;
or a pharmaceutically acceptable acid addition salt, a racemic mixture or its corresponding enantiomer and/or optical isomers thereof.

19. A compound of formula IJ according to any one of claims 1 or 18, wherein the compounds are
2-[1-(3-Chloro-4-hydroxy-5-propoxy-phenyl)-meth-(Z)-ylidene]-6-thiophen-3-yl-imidazo[2,1-b]thiazol-3-one
2-[1-(3-Chloro-4-hydroxy-5-propoxy-phenyl)-meth-(Z)-ylidene]-6-thiophen-2-yl-imidazo[2,1-b]thiazol-3-one or
2-[1-(3-Chloro-4-hydroxy-5-propoxy-phenyl)-meth-(Z)-ylidene]-6-(2,4-dimethylthiophen-3-yl)-imidazo[2,1-b]thiazol-3-one.

20. A compound of formula I according to any one of claims 1 - 19 for use in binding and imaging tau aggregates, beta-amyloid aggregates or alpha-synuclein aggregates

21. A compound of formula I according to any one of claims 1 - 19 for use in binding and imaging tau aggregates in Alzheimer patients.

22. A compound of formula I according to any one of claims 1 - 19 for use in a tau binding study.

23. A compound of formula I according to any one of claims 1 - 19 for use of diagnostic imaging of tau-aggregates in the brain of a mammal.

24. A pharmaceutical composition comprising a compound of formula I according to any one of claims 1 - 19 and a pharmaceutical acceptable carrier.

25. A method of imaging tau-aggregate deposits, comprising
- introducing into a mammal a detectable quantity of a composition according to claim 24;
- allowing sufficient time for the compound of formula I to be associated with tau-aggregate deposit, and
- detecting the compound associated with one or more tau-aggregate deposits.

26. The use of a compound as defined in any one of claims 1 - 19 for diagnostic imaging of tau-aggregate deposits in the brain of a mammal.

## Patentansprüche

1. Eine Verbindung der Formel 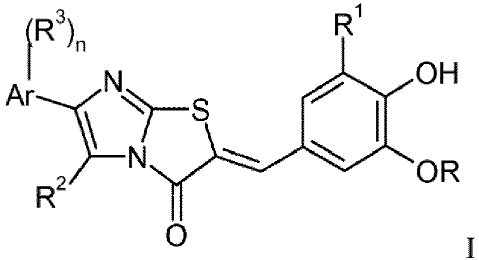 wobei
Ar Phenyl, Pyridinyl, 2,3-Dihydro-benzo[1,4]dioxinyl, 1,3-Dihydro-indol-2-on, Pyrazinyl, Isoxazol-3-yl, Imidazolyl, Thiophenyl oder Pyrimidinyl ist;
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
R³ Wasserstoff, Halogen, C₁₋₇-Alkyl, mit Halogen substituiertes C₁₋₇-Alkyl, C₁₋₇-Alkoxy, mit Halogen substituiertes C₁₋₇-Alkoxy, mit Halogen substituiertes O(CH₂)ₘO(CH₂)ₘO-C₁₋₇-Alkyl, Cyano, mit Hydroxy substituiertes C₁₋₇-Alkoxy, C₁₋₇-Alkenyloxy, C(O)OH, Heterocycloalkyl, ausgewählt aus Morpholinyl, Pyrrolidinyl oder Pyrrolidin-2-on, oder Heteroaryl, ausgewählt aus mit C₁₋₇-Alkyl substituiertem Imidazolyl ist, oder NR'R" ist und R'/R" unabhängig voneinander Wasserstoff oder C₁₋₇-Alkyl oder -C(O)-C₁₋₇-Alkyl sind; oder -C(O)NR⁴R⁵ ist und R⁴ Wasserstoff oder C₁₋₇-Alkyl ist und R⁵ Wasserstoff, C₁₋₇-Alkyl, C₁₋₇-Alkenyl, mit Halogen substituiertes -(CH₂)ₘO-C₁₋₇-Alkyl, mit Halogen substituiertes C₁₋₇-Alkyl, gegebenenfalls mit Halogen substituiertes -(CH₂)ₙ-Phenyl, -(CH₂)ₘNHC(O)-C₁₋₇Alkyl oder - (CH₂)ₘNH₂ ist, oder
R⁴ und R⁵ zusammen mit dem N-Atom, an das sie gebunden sind, einen Piperidin- oder Azetidin-Ring bilden können, der mit Halogen substituiert sein kann; oder mit Halogen substituiertes -C(O)O-C₁₋₇-Alkyl ist;
n 1 oder 2 ist;
m 1, 2 oder 3 ist;
oder ein pharmazeutisch verträgliches Säureadditionssalz, ein racemisches Gemisch oder das entsprechende Enantiomer und/oder optische Isomere davon.

2. Eine Verbindung der Formel IA gemäß Anspruch 1 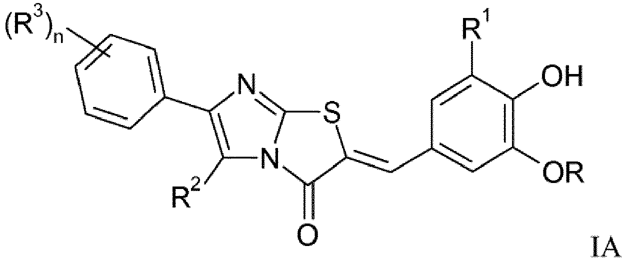 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
R³ Wasserstoff, Halogen, C₁₋₇-Alkyl, mit Halogen substituiertes C₁₋₇-Alkyl, C₁₋₇-Alkoxy, mit Halogen substituiertes C₁₋₇-Alkoxy, mit Halogen substituiertes O(CH₂)ₘO(CH₂)ₘO-C₁₋₇-Alkyl, Cyano, mit Hydroxy substituiertes C₁₋₇-Alkoxy, C₁₋₇-Alkenyloxy, C(O)OH, Heterocycloalkyl, ausgewählt aus Morpholinyl, Pyrrolidinyl oder Pyrrolidin-2-on, oder Heteroaryl, ausgewählt aus mit C₁₋₇-Alkyl substituiertem Imidazolyl ausgewählt ist, oder NR'R" ist und R'/R" unabhängig voneinander Wasserstoff oder C₁₋₇-Alkyl oder -C(O)-C₁₋₇-Alkyl sind; oder -C(O)NR⁴R⁵ ist und
R⁴ Wasserstoff oder C₁₋₇-Alkyl ist und
R⁵ Wasserstoff, C₁₋₇-Alkyl, C₁₋₇-Alkenyl, mit Halogen substituiertes -(CH₂)ₘO-C₁₋₇-Alkyl, mit Halogen substituiertes C₁₋₇-Alkyl, gegebenenfalls mit Halogen substituiertes -(CH₂)ₙ-Phenyl, -(CH₂)ₘNHC(O)-C₁₋₇-Alkyl oder -(CH₂)ₘNH₂ ist, oder R⁴ und R⁵ zusammen mit dem N-Atom, an das sie gebunden sind, einen Piperidin- oder Azetidin-Ring bilden können, der mit Halogen substituiert sein kann; oder mit Halogen substituiertes -C(O)O-C₁₋₇-Alkyl ist;
n 1 oder 2 ist;
m 1, 2 oder 3 ist;
oder ein pharmazeutisch verträgliches Säureadditionssalz, ein racemisches Gemisch oder das entsprechende Enantiomer und/oder optische Isomere davon.

3. Eine Verbindung der Formel IA gemäß einem der Ansprüche 1 oder 2, wobei die Verbindungen
6-(4-Chlor-phenyl)-2-[1-(4-hydroxy-3,5-dimethoxy-phenyl)-meth-(Z)-yliden]-imidazo[2,1-b]thiazol-3-on
6-(4-Chlor-phenyl)-2-[1-(4-hydroxy-3-methoxy-phenyl)-meth-(Z)-yliden]-imidazo[2,1-b]thiazol-3-on
6-(4-Chlor-phenyl)-2-[1-(3-fluor-4-hydroxy-5-methoxy-phenyl)-meth-(Z)-yliden]-imidazo[2,1-b]thiazol-3-on
6-(4-Chlor-phenyl)-2-[1-(3-ethoxy-4-hydroxy-phenyl)-meth-(Z)-yliden]-imidazo[2,1-b]thiazol-3-on
6-(4-Chlor-phenyl)-2-[1-(3-chlor-4-hydroxy-5-methoxy-phenyl)-meth-(Z)-yliden]-imidazo[2,1-b]thiazol-3-on
6-(4-Chlor-phenyl)-2-[1-(3-brom-4-hydroxy-5-methoxy-phenyl)-meth-(Z)-yliden]-imidazo[2,1-b]thiazol-3-on
6-(4-Chlor-phenyl)-2-[1-(3,4-dihydroxy-5-methoxy-phenyl)-meth-(Z)-yliden]-imidazo[2,1-b]thiazol-3-on
2-[1-(3-Fluor-4-hydroxy-5-methoxy-phenyl)-meth-(Z)-yliden]-6-o-tolyl-imidazo[2,1-b]thiazol-3-on
2-[1-(3-Chlor-4-hydroxy-5-methoxy-phenyl)-meth-(Z)-yliden]-6-o-tolyl-imidazo[2,1-b]thiazol-3-on
2-[1-(4-Hydroxy-3-methoxy-phenyl)-meth-(Z)-yliden]-6-o-tolyl-imidazo[2,1-b]thiazol-3-on
2-[1-(3-Chlor-4-hydroxy-5-methoxy-phenyl)-meth-(Z)-yliden]-6-(4-methoxy-phenyl)-5-methyl-imidazo[2,1-b]thiazol-3-on
2-[1-(3-Fluor-4-hydroxy-5-methoxy-phenyl)-meth-(Z)-yliden]-6-(4-methoxy-phenyl)-5-methyl-imidazo[2,1-b]thiazol-3-on
2-[1-(4-Hydroxy-3,5-dimethoxy-phenyl)-meth-(Z)-yliden]-6-(3-trifluormethoxy-phenyl)-imidazo[2,1-b]thiazol-3-on
2-[1-(4-Hydroxy-3,5-dimethoxy-phenyl)-meth-(Z)-yliden]-6-(3-trifluormethyl-phenyl)-imidazo[2,1-b]thiazol-3-on
2-[1-(4-Hydroxy-3,5-dimethoxy-phenyl)-meth-(Z)-yliden]-6-m-tolyl-imidazo[2,1-b]thiazol-3-on
6-(3-Chlor-phenyl)-2-[1-(4-hydroxy-3,5-dimethoxy-phenyl)-meth-(Z)-yliden]-imidazo[2,1-b]thiazol-3-on
2-[1-(3-Chlor-4-hydroxy-5-methoxy-phenyl)-meth-(Z)-yliden]-6-(3-trifluormethoxy-phenyl)-imidazo[2,1-b]thiazol-3-on
4-{2-[1-(3-Chlor-4-hydroxy-5-methoxy-phenyl)-meth-(Z)-yliden]-3-oxo-2,3-dihydro-imidazo[2,1-b]thiazol-6-yl}-N-[2-(2-fluor-ethoxy)-ethyl]-N-methyl-benzamid
2-[1-(3-Chlor-4-hydroxy-5-methoxy-phenyl)-meth-(Z)-yliden]-6-(3-trifluormethylphenyl)-imidazo[2,1-b]thiazol-3-on
6-(3-Chlor-phenyl)-2-[1-(3-chlor-4-hydroxy-5-methoxy-phenyl)-meth-(Z)-yliden]-imidazo[2,1-b]thiazol-3-on
6-(4-Chlor-phenyl)-2-[1-(3-ethoxy-4-hydroxy-5-methoxy-phenyl)-meth-(Z)-yliden]-imidazo[2,1-b]thiazol-3-on
6-(4-Chlor-phenyl)-2-[1-(3-(2-fluor-ethoxy)-4-hydroxy-5-methoxy-phenyl)-meth-(Z)-yliden]-imidazo[2,1-b]thiazol-3-on
2-[1-(3-Chlor-4-hydroxy-5-methoxy-phenyl)-meth-(Z)-yliden]-6-(3-fluor-4-trifluormethyl-phenyl)-imidazo[2,1-b]thiazol-3-on
2-[1-(3-Hydroxy-3,5-dimethoxy-phenyl)-meth-(Z)-yliden]-6-(3-fluor-4-trifluormethyl-phenyl)-imidazo[2,1-b]thiazol-3-on
6-(4-Chlor-phenyl)-2-[1-(4-hydroxy-3-isopropoxy-5-methoxy-phenyl)-meth-(Z)-yliden]-imidazo[2,1-b]thiazol-3-on
6-(4-Chlor-phenyl)-2-[1-(4-hydroxy-3-methoxy-5-propoxy-phenyl)-meth-(Z)-yliden]-imidazo[2,1-b]thiazol-3-on
6-(4-Chlor-phenyl)-2-[1-(4-hydroxy-3-methoxy-5-methyl-phenyl)-meth-(Z)-yliden]-imidazo[2,1-b]thiazol-3-on
2-[1-(4-Hydroxy-3-methoxy-5-propoxy-phenyl)-meth-(Z)-yliden]-6-(3-trifluormethoxy-phenyl)-imidazo[2,1-b]thiazol-3-on
2-[1-(4-Hydroxy-3-methoxy-5-isopropoxy-phenyl)-meth-(Z)-yliden]-6-(3-trifluormethoxy-phenyl)-imidazo[2,1-b]thiazol-3-on
2-[1-(3-Fluor-4-hydroxy-5-methoxy-phenyl)-meth-(Z)-yliden]-6-(3-trifluormethoxy-phenyl)-imidazo[2,1-b]thiazol-3-on
2-[1-(4-Hydroxy-3-methoxy-phenyl)-meth-(Z)-yliden]-6-(3-trifluormethoxy-phenyl)-imidazo[2,1-b]thiazol-3-on
2-[1-(3-Brom-4-hydroxy-5-methoxy-phenyl)-meth-(Z)-yliden]-6-(3-trifluormethoxy-phenyl)-imidazo[2,1-b]thiazol-3-on
3-{2-[1-(3-Fluor-4-hydroxy-5-methoxy-phenyl)-meth-(Z)-yliden]-3-oxo-2,3-dihydro-imidazo[2,1-b]thiazol-6-yl}-benzonitril
2-[1-(3-Fluor-4-hydroxy-5-methoxy-phenyl)-meth-(Z)-yliden]-6-(3-chlor-phenyl)-imidazo[2,1-b]thiazol-3-on
2-[1-(3-Ethyl-4-hydroxy-5-methoxy-phenyl)-meth-(Z)-yliden]-6-(3-trifluormethoxy-phenyl)-imidazo[2,1-b]thiazol-3-on
2-[1-(3-Fluor-4-hydroxy-5-methoxy-phenyl)-meth-(Z)-yliden]-6-(3-(trifluormethyl)-phenyl)-imidazo[2,1-b]thiazol-3-on
2-[1-(3-Fluor-4-hydroxy-5-methoxy-phenyl)-meth-(Z)-yliden]-6-(4-fluor-3-(trifluormethyl)-phenyl)-imidazo[2,1-b]thiazol-3-on
2-[1-(3-Fluor-4-hydroxy-5-methoxy-phenyl)-meth-(Z)-yliden]-6-phenyl-imidazo[2,1-b]thiazol-3-on
3-{2-[1-(3-Chlor-4-hydroxy-5-methoxy-phenyl)-meth-(Z)-yliden]-3-oxo-2,3-dihydro-imidazo[2,1-b]thiazol-6-yl}-benzonitril
3-{2-[1-(3-Brom-4-hydroxy-5-methoxy-phenyl)-meth-(Z)-yliden]-3-oxo-2,3-dihydro-imidazo[2,1-b]thiazol-6-yl}-benzonitril
2-[1-(3-Brom-4-hydroxy-5-methoxy-phenyl)-meth-(Z)-yliden]-6-(4-fluor-3-(trifluormethyl)-phenyl)-imidazo[2,1-b]thiazol-3-on
2-[1-(3-Fluor-4-hydroxy-5-methoxy-phenyl)-meth-(Z)-yliden]-6-(3-methoxy-phenyl)-imidazo[2,1-b]thiazol-3-on
2-[1-(3-Chlor-4-hydroxy-5-methoxy-phenyl)-meth-(Z)-yliden]-6-(3,5-difluor-phenyl)-imidazo[2,1-b]thiazol-3-on
3-{2-[1-(4-Hydroxy-3,5-dimethoxy-phenyl)-meth-(Z)-yliden]-3-oxo-2,3-dihydro-imidazo[2,1-b]thiazol-6-yl}-benzonitril
3-{2-[1-(4-Hydroxy-3-methoxy-5-isopropoxyphenyl)-meth-(Z)-yliden]-3-oxo-2,3-dihydro-imidazo[2,1-b]thiazol-6-yl}-benzonitril
2-[1-(3-Chlor-4-hydroxy-5-methoxy-phenyl)-meth-(Z)-yliden]-6-m-tolyl-imidazo[2,1-b]thiazol-3-on
2-[1-(3-Chlor-4-hydroxy-5-methoxy-phenyl)-meth-(Z)-yliden]-6-(2-fluor-phenyl)-imidazo[2,1-b]thiazol-3-on
2-[1-(3-Chlor-4-hydroxy-5-methoxy-phenyl)-meth-(Z)-yliden]-6-(3-ethyl-phenyl)-imidazo[2,1-b]thiazol-3-on
2-[1-(3-Chlor-4-hydroxy-5-methoxy-phenyl)-meth-(Z)-yliden]-6-(2-methoxy-phenyl)-imidazo[2,1-b]thiazol-3-on
2-[1-(3-Chlor-4-hydroxy-5-methoxy-phenyl)-meth-(Z)-yliden]-6-(2,5-difluor-phenyl)-imidazo[2,1-b]thiazol-3-on
2-[1-(3-Chlor-4-hydroxy-5-methoxy-phenyl)-meth-(Z)-yliden]-6-(3-fluor-phenyl)-imidazo[2,1-b]thiazol-3-on
2-[1-(3-Fluor-4-hydroxy-5-methoxy-phenyl)-meth-(Z)-yliden]-6-(3-fluor-phenyl)-imidazo[2,1-b]thiazol-3-on
2-[1-(4-Hydroxy-3,5-dimethoxy-phenyl)-meth-(Z)-yliden]-6-(3-fluor-phenyl)-imidazo[2,1-b]thiazol-3-on
2-[1-(3-Chlor-4-hydroxy-5-methoxy-phenyl)-meth-(Z)-yliden]-6-phenyl-imidazo[2,1-b]thiazol-3-on
2-[1-(3-Chlor-4-hydroxy-5-methoxy-phenyl)-meth-(Z)-yliden]-6-(3-methoxy-phenyl)-imidazo[2,1-b]thiazol-3-on
2-[1-(3-Chlor-4-hydroxy-5-methoxy-phenyl)-meth-(Z)-yliden]-6-(3-(difluormethoxy)-phenyl)-imidazo[2,1-b]thiazol-3-on
(Z)-3-(2-(3-Fluor-4-hydroxy-5-methoxybenzyliden)-3-oxo-2,3-dihydroimidazo[2,1-b]thiazol-5-yl)benzonitril
2-[1-(3-Chlor-4-hydroxy-5-methoxy-phenyl)-meth-(Z)-yliden]-6-(3-(chlormethyl)-phenyl)-imidazo[2,1-b]thiazol-3-on
3-{2-[1-(3-Fluor-4-hydroxy-5-methoxy-phenyl)-meth-(Z)-yliden]-3-oxo-2,3-dihydro-imidazo[2,1-b]thiazol-6-yl}-benzonitril
2-[1-(3-Chlor-4-hydroxy-5-methoxy-phenyl)-meth-(Z)-yliden]-6-(3-(2-fluorethoxy)phenyl)-imidazo[2,1-b]thiazol-3-on
2-[1-(3-Chlor-4-hydroxy-5-methoxy-phenyl)-meth-(Z)-yliden]-6-(4-(2-fluorethoxy)phenyl)-imidazo[2,1-b]thiazol-3-on
2-[1-(3-Chlor-4-hydroxy-5-methoxy-phenyl)-meth-(Z)-yliden]-6-(2-(2-fluorethoxy)phenyl)-imidazo[2,1-b]thiazol-3-on
3-{2-[1-(3-Chlor-4-hydroxy-5-methoxy-phenyl)-meth-(Z)-yliden]-3-oxo-2,3-dihydro-imidazo[2,1-b]thiazol-6-yl}-benzamid
4-{2-[1-(3-Chlor-4-hydroxy-5-methoxy-phenyl)-meth-(Z)-yliden]-3-oxo-2,3-dihydro-imidazo[2,1-b]thiazol-6-yl}-benzonitril
2-[1-(3-Chlor-4-hydroxy-5-methoxy-phenyl)-meth-(Z)-yliden]-6-(4-(difluormethoxy)phenyl)-imidazo[2,1-b]thiazol-3-on
2-[1-(3-Chlor-4-hydroxy-5-methoxy-phenyl)-meth-(Z)-yliden]-6-(3-morpholinophenyl)-imidazo[2,1-b]thiazol-3-on
2-[1-(3-Chlor-4-hydroxy-5-methoxy-phenyl)-meth-(Z)-yliden]-6-(4-(pyrrolidin-1-yl)phenyl)-imidazo[2,1-b]thiazol-3-on
2-[1-(3-Chlor-4-hydroxy-5-methoxy-phenyl)-meth-(Z)-yliden]-6-(4-(diethylamino)phenyl)-imidazo[2,1-b]thiazol-3-on
2-[1-(3-Chlor-4-hydroxy-5-methoxy-phenyl)-meth-(Z)-yliden]-6-(4-morpholinophenyl)-imidazo[2,1-b]thiazol-3-on
2-[1-(3-Chlor-4-hydroxy-5-methoxy-phenyl)-meth-(Z)-yliden]-6-(4-(dimethylamino)phenyl)-imidazo[2,1-b]thiazol-3-on
2-[1-(3-Chlor-4-hydroxy-5-methoxy-phenyl)-meth-(Z)-yliden]-6-[3-(2-oxo-pyrrolidin-1-yl)-phenyl]-imidazo[2,1-b]thiazol-3-on
4-{2-[1-(3-Chlor-4-hydroxy-5-methoxy-phenyl)-meth-(Z)-yliden]-3-oxo-2,3-dihydro-imidazo[2,1-b]thiazol-6-yl}-benzoesäure
2-[1-(3-Chlor-4-hydroxy-5-methoxy-phenyl)-meth-(Z)-yliden]-6-(3,4-dimethoxy-phenyl)-imidazo[2,1-b]thiazol-3-on
4-{2-[1-(3-Chlor-4-hydroxy-5-methoxy-phenyl)-meth-(Z)-yliden]-3-oxo-2,3-dihydroimidazo[2,1-b]thiazol-6-yl}-benzamid
N-(4-{2-[1-(3-Chlor-4-hydroxy-5-methoxy-phenyl)-meth-(Z)-yliden]-3-oxo-2,3-dihydroimidazo[2,1-b]thiazol-6-yl}-phenyl)-acetamid
3-{2-[1-(3-Chlor-4-hydroxy-5-methoxy-phenyl)-meth-(Z)-yliden]-3-oxo-2,3-dihydroimidazo[2,1-b]thiazol-6-yl}-benzoesäure
2-[1-(3-Chlor-4-hydroxy-5-methoxy-phenyl)-meth-(Z)-yliden]-6-(2,6-difluor-phenyl)-imidazo[2,1-b]thiazol-3-on
3-{2-[1-(3-Chlor-4-hydroxy-5-methoxy-phenyl)-meth-(Z)-yliden]-3-oxo-2,3-dihydroimidazo[2,1-b]thiazol-6-yl}-N-(2-fluor-ethyl)-benzamid
3-{2-[1-(3-Chlor-4-hydroxy-5-methoxy-phenyl)-meth-(Z)-yliden]-3-oxo-2,3-dihydroimidazo[2,1-b]thiazol-6-yl}-N-propyl-benzamid
3-{2-[1-(3-Chlor-4-hydroxy-5-methoxy-phenyl)-meth-(Z)-yliden]-3-oxo-2,3-dihydroimidazo[2,1-b]thiazol-6-yl}-N-methyl-benzamid
N-Allyl-3-{2-[1-(3-chlor-4-hydroxy-5-methoxy-phenyl)-meth-(Z)-yliden]-3-oxo-2,3-dihydro-imidazo[2,1-b]thiazol-6-yl}-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-methyl-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-propyl-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-(2-fluor-ethyl)-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-(3-fluor-propyl)-benzamid
3-{2-[1-(3-Chlor-4-hydroxy-5-methoxy-phenyl)-meth-(Z)-yliden]-3-oxo-2,3-dihydroimidazo[2,1-b] thiazol-6-yl}-N-[2-(3-fluor-phenyl)-ethyl]-N-methyl-benzamid
3-{2-[1-(3-Chlor-4-hydroxy-5-methoxy-phenyl)-meth-(Z)-yliden]-3-oxo-2,3-dihydroimidazo[2,1-b]thiazol-6-yl}-N-(2-fluor-ethyl)-N-methyl-benzamid
3-{2-[1-(3-Fluor-4-hydroxy-5-methoxy-phenyl)-meth-(Z)-yliden]-3-oxo-2,3-dihydroimidazo[2,1-b]thiazol-6-yl}-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-(2-fluor-ethyl)-N-methyl-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N,N-dimethyl-benzamid
2-[1-(3-Chlor-4-hydroxy-5-methoxy-phenyl)-meth-(Z)-yliden]-6-[4-(4-fluor-piperidin-1-carbonyl)-phenyl]-imidazo[2,1-b]thiazol-3-on
4-{2-[1-(3-Chlor-4-hydroxy-5-methoxy-phenyl)-meth-(Z)-yliden]-3-oxo-2,3-dihydroimidazo[2,1-b] thiazol-6-yl}-N-methyl-N-propyl-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-N-(3-fluorpropoxy)-benzamid
2-[1-(3-Chlor-4-hydroxy-5-methoxy-phenyl)-meth-(Z)-yliden]-6-[4-(3-fluor-azetidin-1-carbonyl)-phenyl]-imidazo[2,1-b]thiazol-3-on
4-{2-[1-(3-Chlor-4-hydroxy-5-methoxy-phenyl)-meth-(Z)-yliden]-3-oxo-2,3-dihydroimidazo[2,1-b]thiazol-6-yl}-N-(3-fluorpropoxy)-N-methyl-benzamid
4-{2-[1-(3-Fluor-4-hydroxy-5-methoxy-phenyl)-meth-(Z)-yliden]-3-oxo-2,3-dihydroimidazo[2,1-b]thiazol-6-yl}-benzoesäure
6-[4-(3-Fluor-azetidin-1-carbonyl)-phenyl]-2-[1-(3-fluor-4-hydroxy-5-methoxy-phenyl)-meth-(Z)-yliden]-imidazo[2,1-b]thiazol-3-on
4-{2-[1-(3-Fluor-4-hydroxy-5-methoxy-phenyl)-meth-(Z)-yliden]-3-oxo-2,3-dihydroimidazo[2,1-b]thiazol-6-yl}-N-(2-fluorethoxy)-benzamid
3-{2-[1-(3-Fluor-4-hydroxy-5-methoxy-phenyl)-meth-(Z)-yliden]-3-oxo-2,3-dihydroimidazo[2,1b]thiazol-6-yl}-N-(3-fluorpropoxy-N-methyl-benzamid
2-[1-(3-Chlor-4-hydroxy-5-methoxy-phenyl)-meth-(Z)-yliden]-6-[3-(3-fluor-azetidin-1-carbonyl)-phenyl]-imidazo[2,1-b]thiazol-3-on
2-[1-(3-Chlor-4-hydroxy-5-methoxy-phenyl)-meth-(Z)-yliden]-6-[3-(4-fluor-piperidin-1-carbonyl-phenyl]-imidazo[2,1-b]thiazol-3-on
3-{2-[1-(3-Chlor-4-hydroxy-5-methoxy-phenyl)-meth-(Z)-yliden]-3-oxo-2,3-dihydroimidazo[2,1-b]thiazol-6-yl}-N-[2-(2-fluor-ethoxy)-ethyl]-N-methyl-benzamid
4-{2-[1-(3-Fluor-4-hydroxy-5-methoxy-phenyl)-meth-(Z)-yliden]-3-oxo-2,3-dihydroimidazo[2,1-b]thiazol-6-yl}-N-(2-fluorethoxy)-N-methyl-benzamid
4-{2-[1-(3-Fluor-4-hydroxy-5-methoxy-phenyl)-meth-(Z)-yliden]-3-oxo-2,3-dihydroimidazo[2,1-b]thiazol-6-yl}-N-(2-fluoipropoxy)-N-methyl-benzamid
4-{2-[1-(3-Chlor-4-hydroxy-5-methoxy-phenyl)-meth-(Z)-yliden]-3-oxo-2,3-dihydroimidazo[2,1-b]thiazol-6-yl}-benzoesäure-2-fluor-ethylester
4-{2-[1-(3-Chlor-4-hydroxy-5-methoxy-phenyl)-meth-(Z)-yliden]-3-oxo-2,3-dihydroimidazo[2,1-b]thiazol-6-yl}-benzoesäure-3-fluor-propylester
[2-(4-{2-[1-(3-Chlor-4-hydroxy-5-methoxy-phenyl)-meth-(Z)-yliden]-3-oxo-2,3-dihydro-imidazo[2,1-b]thiazol-6-yl}-benzoylamino)-ethyl]-carbamidsäure-tert-butylester
N-(2-Amino-ethyl)-4-{2-[1-(3-chlor-4-hydroxy-5-methoxy-phenyl)-meth-(Z)-yliden]-3-oxo-2,3-dihydro-imidazo[2,1-b]thiazol-6-yl}-benzamid
3-{2-[1-(3-Chlor-4-hydroxy-5-methoxy-phenyl)-meth-(Z)-yliden]-3-oxo-2,3-dihydroimidazo[2,1-b]thiazol-6-yl}-benzolsulfonylfluorid
3-{2-[1-[3-Chlor-5-(3-fluor-propoxy)-4-hydroxy-phenyl]-meth-(Z)-yliden]-3-oxo-2,3-dihydro-imidazo[2,1-b]thiazol-6-yl}-benzonitril
3-Brom-5-{2-[1-(3-chlor-4-hydroxy-5-methoxy-phenyl)-meth-(Z)-yliden]-3-oxo-2,3-dihydro-imidazo[2,1-b]thiazol-6-yl}-benzonitril
2-[1-(3-Chlor-4-hydroxy-5-methoxy-phenyl)-meth-(Z)-yliden]-6-(3-{2-[2-(2-fluorethoxy)-ethoxy]-ethoxy}-phenyl)-imidazo[2,1-b]thiazol-3-on oder
2-[1-(3-Chlor-4-hydroxy-5-methoxy-phenyl)-meth-(Z)-yliden]-6-(2-{2-[2-(2-fluorethoxy)-ethoxy]-ethoxy}-phenyl)-imidazo[2,1-b]thiazol-3-on sind.

4. Eine Verbindung der Formel IB gemäß Anspruch 1, 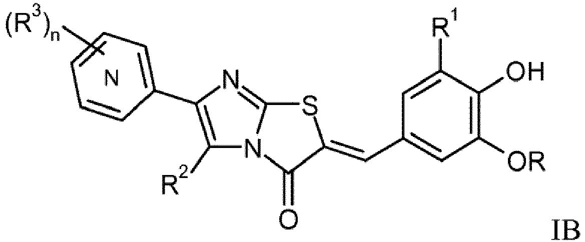 wobei das N-Atom der Pyridinylgruppe in verschiedenen Positionen sein kann und wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
R³ Wasserstoff, Halogen, C₁₋₇-Alkyl, mit Halogen substituiertes C₁₋₇-Alkyl, C₁₋₇-Alkoxy, mit Halogen substituiertes C₁₋₇-Alkoxy, mit Halogen substituiertes O(CH₂)ₘO(CH₂)ₘO-C₁₋₇-Alkyl, Cyano, mit Hydroxy substituiertes C₁₋₇-Alkoxy, C₁₋₇-Alkenyloxy, C(O)OH, Heterocycloalkyl, ausgewählt aus Morpholinyl, Pyrrolidinyl oder Pyrrolidin-2-on, oder Heteroaryl, ausgewählt aus mit C₁₋₇-Alkyl substituiertem Imidazolyl ist oder NR'R" ist und R'/R" unabhängig voneinander Wasserstoff oder C₁₋₇-Alkyl oder -C(O)-C₁₋₇-Alkyl sind; oder
-C(O)NR⁴R⁵ ist und
R⁴ Wasserstoff oder C₁₋₇-Alkyl ist und
R⁵ Wasserstoff, C₁₋₇-Alkyl, C₁₋₇-Alkenyl, mit Halogen substituiertes -(CH₂)ₘO-C₁₋₇-Alkyl, mit Halogen substituiertes C₁₋₇-Alkyl, gegebenenfalls mit Halogen substituiertes -(CH₂)ₙ-Phenyl, -(CH₂)ₘNHC(O)-C₁₋₇-Alkyl oder -(CH₂)ₘNH₂ ist, oder
R⁴ und R⁵ zusammen mit dem N-Atom, an das sie gebunden sind, einen Piperidin- oder Azetidin-Ring bilden können, der mit Halogen substituiert sein kann; oder mit Halogen substituiertes -C(O)O-C₁₋₇-Alkyl ist;
n 1 oder 2 ist;
m 1, 2 oder 3 ist;
oder ein pharmazeutisch verträgliches Säureadditionssalz, ein racemisches Gemisch oder das entsprechende Enantiomer und/oder optische Isomere davon.

5. Eine Verbindung der Formel IB gemäß einem der Ansprüche 1 oder 4, wobei die Verbindungen
2-[1-(4-Hydroxy-3,5-dimethoxy-phenyl)-meth-(Z)-yliden]-6-pyridin-4-yl-imidazo[2,1-b]thiazol-3-on
2-[1-(4-Hydroxy-3-methoxy-phenyl)-meth-(Z)-yliden]-6-(2-methyl-pyridin-4-yl)-imidazo[2,1-b]thiazol-3-on
2-[1-(4-Hydroxy-3-methoxy-phenyl)-meth-(Z)-yliden]-6-(2,6-dimethyl-pyridin-4-yl)-imidazo[2,1-b]thiazol-3-on
2-[1-(4-Hydroxy-3,5-dimethoxy-phenyl)-meth-(Z)-yliden]-6-(2-methyl-pyridin-4-yl)-imidazo[2,1-b]thiazol-3-on
2-[1-(4-Hydroxy-3,5-dimethoxy-phenyl)-meth-(Z)-yliden]-6-(2,6-dimethyl-pyridin-4-yl)-imidazo[2,1-b]thiazol-3-on
2-[1-(3-Fluor-4-hydroxy-5-methoxy-phenyl)-meth-(Z)-yliden]-6-(6-methoxy-pyridin-2-yl)-imidazo[2,1-b]thiazol-3-on
2-[1-(3-Chlor-4-hydroxy-5-methoxy-phenyl)-meth-(Z)-yliden]-6-(2,6-dimethyl-pyridin-4-yl)-imidazo[2,1-b]thiazol-3-on
2-[1-(3-Fluor-4-hydroxy-5-methoxy-phenyl)-meth-(Z)-yliden]-6-(2,6-dimethyl-pyridin-4-yl)-imidazo[2,1-b]thiazol-3-on
2-[1-(3-Brom-4-hydroxy-5-methoxy-phenyl)-meth-(Z)-yliden]-6-(2,6-dimethyl-pyridin-4-yl)-imidazo[2,1-b]thiazol-3-on
2-[1-(3-Chlor-4-hydroxy-5-methoxy-phenyl)-meth-(Z)-yliden]-6-(pyridin-4-yl)-imidazo[2,1-b]thiazol-3-on
2-[1-(3-Fluor-4-hydroxy-5-methoxy-phenyl)-meth-(Z)-yliden]-6-(pyridin-4-yl)-imidazo[2,1-b]thiazol-3-on
2-[1-(3-Brom-4-hydroxy-5-methoxy-phenyl)-meth-(Z)-yliden]-6-(pyridin-4-yl)-imidazo[2,1-b]thiazol-3-on
2-[1-(3-Fluor-4-hydroxy-5-methoxy-phenyl)-meth-(Z)-yliden]-6-(pyridin-3-yl)-imidazo[2,1-b]thiazol-3-on
2-[1-(3-Brom-4-hydroxy-5-methoxy-phenyl)-meth-(Z)-yliden]-6-(pyridin-3-yl)-imidazo[2,1-b]thiazol-3-on
2-[1-(3-Chlor-4-hydroxy-5-methoxy-phenyl)-meth-(Z)-yliden]-6-(2-methylpyridin-4-yl)-imidazo[2,1-b]thiazol-3-on
2-[1-(3-Fluor-4-hydroxy-5-methoxy-phenyl)-meth-(Z)-yliden]-6-(2-methylpyridin-4-yl)-imidazo[2,1-b]thiazol-3-on
2-[1-(3-Brom-4-hydroxy-5-methoxy-phenyl)-meth-(Z)-yliden]-6-(2-methylpyridin-4-yl)-imidazo[2,1-b]thiazol-3-on
2-[1-(3-Chlor-4-hydroxy-5-methoxy-phenyl)-meth-(Z)-yliden]-6-(pyridin-3-yl)-imidazo[2,1-b]thiazol-3-on
2-[1-(3-Chlor-4-hydroxy-5-methoxy-phenyl)-meth-(Z)-yliden]-6-[6-(4-methyl-3H-imidazol-1-yl)-pyridin-3-yl]-imidazo[2,1-b]thiazol-3-on
2-[1-(3-Chlor-4-hydroxy-5-propoxy-phenyl)-meth-(Z)-yliden]-6-(4-methyl-pyridin-3-yl)-imidazo[2,1-b]thiazol-3-on
2-[1-(3-Chlor-4-hydroxy-5-propoxy-phenyl)-meth-(Z)-yliden]-6-pyridin-3-yl-imidazo[2,1-b]thiazol-3-on
2-[1-(3-Chlor-4-hydroxy-5-propoxy-phenyl)-meth-(Z)-yliden]-6-pyridin-4-yl-imidazo[2,1-b]thiazol-3-on
2-[1-(3-Chlor-4-hydroxy-5-propoxy-phenyl)-meth-(Z)-yliden]-6-[6-(4-methyl-imidazol-1-yl)-pyridin-3-yl]-imidazo[2,1-b]thiazol-3-on
2-[1-(3-Fluor-4-hydroxy-5-propoxy-phenyl)-meth-(Z)-yliden]-6-pyridin-4-yl-imidazo[2,1-b]thiazol-3-on
2-[1-(3-Chlor-5-(3-fluorpropoxy)-4-hydroxy-phenyl)-meth-(Z)-yliden]-6-pyridin-4-yl-imidazo[2,1-b]thiazol-3-on
2-[1-(3-Fluor-5-(3-fluorpropoxy)-4-hydroxy-phenyl)-meth-(Z)-yliden]-6-pyridin-4-yl-imidazo[2,1-b]thiazol-3-on
2-[1-(3-Chlor-4-hydroxy-5-propoxy-phenyl)-meth-(Z)-yliden]-6-pyridin-2-yl-imidazo[2,1-b]thiazol-3-on
2-[1-(3-Chlor-4-hydroxy-5-methoxy-phenyl)-meth-(Z)-yliden]-6-(2-chlor-pyridin-4-yl)-imidazo[2,1-b]thiazol-3-on oder
2-[1-[3-Chlor-5-(3-fluor-propoxy)-4-hydroxy-phenyl]-meth-(Z)-yliden]-6-(2,6-dimethylpyridin-4-yl)-imidazo[2,1-b]thiazol-3-on sind.

6. Eine Verbindung der Formel IC gemäß Anspruch 1, 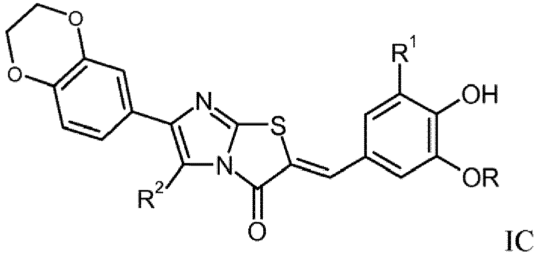 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

7. Eine Verbindung nach einem der Ansprüche 1 oder 6, wobei die Verbindung 2-[1-(3-Chlor-4-hydroxy-5-methoxy-phenyl)-meth-(Z)-yliden]-6-(2,3-dihydro-benzo[1,4]dioxin-6-yl)-imidazo[2,1-b]thiazol-3-on ist.

8. Eine Verbindung der Formel ID gemäß Anspruch 1, 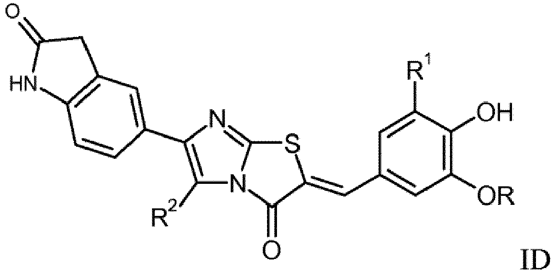 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

9. Eine Verbindung der Formel ID gemäß einem der Ansprüche 1 oder 8, wobei die Verbindung 5-{2-[1-(3-Chlor-4-hydroxy-5-methoxy-phenyl)-meth-(Z)-yliden]-3-oxo-2,3-dihydro-imidazo[2,1-b]thiazol-6-yl}-1,3-dihydro-indol-2-on ist.

10. Eine Verbindung der Formel IE gemäß Anspruch 1, 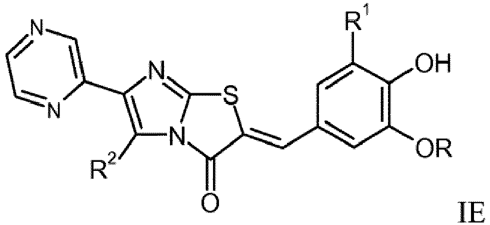 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

11. Eine Verbindung der Formel IE gemäß einem der Ansprüche 1 oder 10, die 2-[1-(3-Chlor-4-hydroxy-5-propoxy-phenyl)-meth-(Z)-yliden]-6-pyrazin-2-yl-imidazo[2,1-b]thiazol-3-on ist.

12. Eine Verbindung der Formel IF gemäß Anspruch 1, 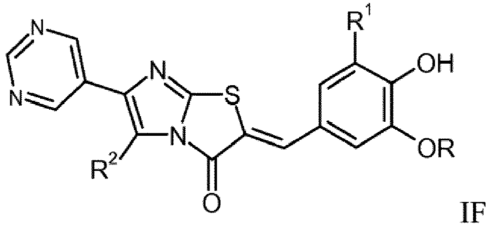 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

13. Eine Verbindung der Formel IF gemäß einem der Ansprüche 1 oder 12, wobei die Verbindung 2-[1-(3-Chlor-4-hydroxy-5-propoxy-phenyl)-meth-(Z)-yliden]-6-pyrimidin-5-yl-imidazo[2,1-b]thiazol-3-on ist.

14. Eine Verbindung der Formel IG gemäß Anspruch 1, 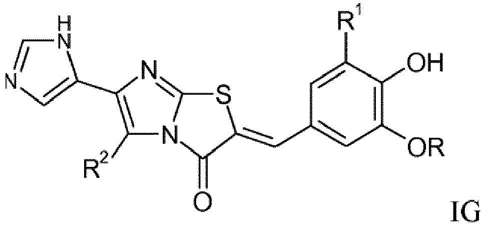 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

15. Eine Verbindung der Formel IG gemäß einem der Ansprüche 1 oder 14, wobei die Verbindung 2-[1-(3-Chlor-4-hydroxy-5-propoxy-phenyl)-meth-(Z)-yliden]-6-(3H-imidazol-4-yl)-imidazo[2,1-b]thiazol-3-on ist.

16. Eine Verbindung der Formel IH gemäß Anspruch 1, 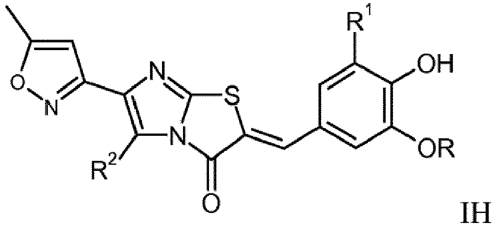 wobei
R C₁₋₇-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

17. Eine Verbindung der Formel IH gemäß einem der Ansprüche 1 oder 16, wobei die Verbindung 2-[1-(3-Chlor-4-hydroxy-5-methoxy-phenyl)-meth-(Z)-yliden]-6-(5-methylisoxazol-3-yl)-imidazo[2,1-b]thiazol-3-on ist.

18. Eine Verbindung der Formel IJ gemäß Anspruch 1, 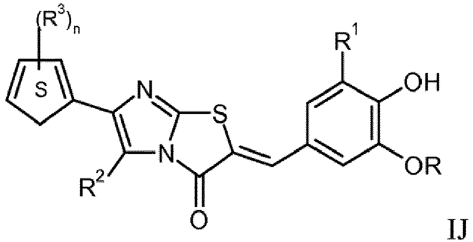 wobei
R³ Wasserstoff oder C₁₋₇-Alkyl ist
n 1 oder 2 ist
R C1_7-Alkyl oder mit Halogen substituiertes C₁₋₇-Alkyl ist;
R¹ Wasserstoff, Halogen, Hydroxy, C₁₋₇-Alkoxy, C₁₋₇-Alkyl und mit Halogen substituiertes C₁₋₇-Alkoxy ist;
R² Wasserstoff, C₁₋₇-Alkyl ist;
oder ein pharmazeutisch verträgliches Säureadditionssalz, ein racemisches Gemisch oder das entsprechende Enantiomer und/oder optische Isomere davon.

19. Eine Verbindung der Formel IJ gemäß einem der Ansprüche 1 oder 18, wobei die Verbindungen
2-[1-(3-Chlor-4-hydroxy-5-propoxy-phenyl)-meth-(Z)-yliden]-6-thiophen-3-yl-imidazo[2,1-b]thiazol-3-on
2-[1-(3-Chlor-4-hydroxy-5-propoxy-phenyl)-meth-(Z)-yliden]-6-thiophen-2-yl-imidazo[2,l-b]thiazol-3-on oder
2-[1-(3-Chlor-4-hydroxy-5-propoxy-phenyl)-meth-(Z)-yliden]-6-(2,4-dimethylthiophen-3-yl)-imidazo[2,1-b]thiazol-3-on sind.

20. Eine Verbindung der Formel I gemäß einem der Ansprüche 1 bis 19 zur Verwendung bei der Bindung und Bildgebung von tau-Aggregaten, beta-Amyloidaggregaten oder alpha-Synucleinaggregaten.

21. Eine Verbindung der Formel I gemäß einem der Ansprüche 1 bis 19 zur Verwendung bei der Bindung und Bildgebung von tau-Aggregaten bei Alzheimerpatienten.

22. Eine Verbindung der Formel I gemäß einem der Ansprüche 1 bis 19 zur Verwendung in einer tau-Bindungsstudie.

23. Eine Verbindung der Formel I gemäß einem der Ansprüche 1 bis 19 zur Verwendung bei der diagnostischen Bildgebung von tau-Aggregaten im Gehirn eines Säugetiers.

24. Ein Arzneimittel, umfassend eine Verbindung der Formel I gemäß einem der Ansprüche 1 bis 19 und einen pharmazeutisch verträglichen Träger.

25. Ein Verfahren zur Bildgebung von tau-Aggregatablagerungen, umfassend
- Einführen einer nachweisbaren Menge einer Zusammensetzung gemäß Anspruch 24 in ein Säugetier;
- ausreichend Zeit Lassen, damit sich die Verbindung der Formel I mit der tau-Aggregatablagerung assoziieren kann, und
- Detektieren der Verbindung, die mit einer oder mehreren tau-Aggregatablagerungen assoziiert ist.

26. Die Verwendung einer Verbindung wie in einem der Ansprüche 1 bis 19 definiert, zur diagnostischen Bildgebung von tau-Aggregatablagerungen im Gehirn eines Säugetiers.

## Revendications

1. Composé de formule 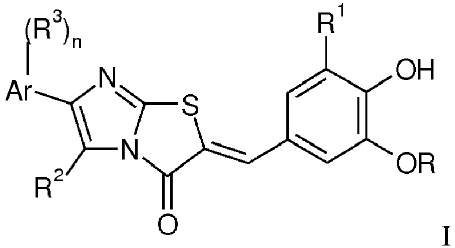 dans laquelle
Ar représente un groupe phényle, pyridinyle, 2,3-dihydro-benzo[1,4]dioxinyle, 1,3-dihydro-indol-2-one, pyrazinyle, isoxazol-3-yle, imidazolyle, thiophényle ou pyrimidinyle ;
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇
R³ représente un atome d'hydrogène, un atome d'halogène, ou un groupe alkyle en C₁ à C₇, alkyle en C₁ à C₇ substitué par un atome d'halogène, alcoxy en C₁ à C₇, alcoxy en C₁ à C₇ substitué par un atome d'halogène, O(CH₂)ₘO(CH₂)ₘO- alkyle en C₁ à C₇ substitué par un atome d'halogène, cyano, alcoxy en C₁ à C₇ substitué par un groupe hydroxy, alcényloxy en C₁ à C₇, C(O)OH, hétérocycloalkyle choisi parmi un groupe morpholinyle, pyrrolidinyle ou pyrrolidin-2-one, ou représente un groupe hétéroaryle choisi parmi un groupe imidazolyle substitué par un groupe alkyle en C₁ à C₇, ou représente un groupe NR'R" et R'/R" représentent indépendamment l'un de l'autre un atome d'hydrogène ou un groupe alkyle en C₁ à C₇ ou -C(O)- alkyle en C₁ à C₇ ; ou représente un groupe -C(O)NR⁴R⁵ et
R⁴ représente un atome d'hydrogène ou un groupe alkyle en C₁ à C₇ et
R⁵ représente un atome d'hydrogène, ou un groupe alkyle en C₁ à C₇, alcényle en C₁ à C₇, -(CH₂)ₘO-alkyle en C₁ à C₇ substitué par un atome d'halogène, alkyle en C₁ à C₇ substitué par un atome d'halogène, -(CH₂)ₙ-phényle facultativement substitué par un atome d'halogène, -CH₂)ₘNHC(O)-alkyle en C₁ à C₇ ou -(CH₂)ₘNH₂, ou
R⁴ et R⁵ peuvent former conjointement avec l'atome de N auquel ils sont fixés un noyau pipéridine ou azétidine, qui peut être substitué par un atome d'halogène ; ou représente
un groupe -C(O)O-alkyle en C₁ à C₇ substitué par un atome d'halogène ;
n vaut 1 ou 2 ;
m vaut 1, 2 ou 3 ;
ou un sel d'addition d'acide pharmaceutiquement acceptable, un mélange racémique ou l'énantiomère et/ou les isomères correspondants de celui-ci.

2. Composé de formule IA selon la revendication 1 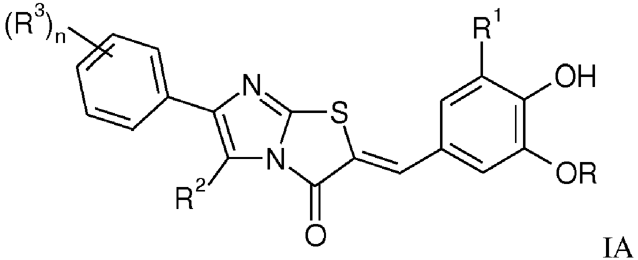 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
R³ représente un atome d'hydrogène, un atome d'halogène, ou un groupe alkyle en C₁ à C₇, alkyle en C₁ à C₇ substitué par un atome d'halogène, alcoxy en C₁ à C₇, alcoxy en C₁ à C₇ substitué par un atome d'halogène, O(CH₂)ₘO(CH₂)ₘO- alkyle en C₁ à C₇ substitué par un atome d'halogène, cyano, alcoxy en C₁ à C₇ substitué par un groupe hydroxy, alcényloxy en C₁ à C₇, C(O)OH, hétérocycloalkyle choisi parmi un groupe morpholinyle, pyrrolidinyle ou pyrrolidin-2-one, ou représente un groupe hétéroaryle choisi parmi un groupe imidazolyle substitué par un groupe alkyle en C₁ à C₇, ou représente
un groupe NR'R" et R'/R" représentent indépendamment l'un de l'autre un atome d'hydrogène ou un groupe alkyle en C₁ à C₇ ou -C(O)-alkyle en C₁ à C₇ ; ou représente
un groupe-C(O)NR⁴R⁵ et
R⁴ représente un atome d'hydrogène ou un groupe alkyle en C₁ à C₇ et
R⁵ représente un atome d'hydrogène, ou un groupe alkyle en C₁ à C₇, alcényle en C₁ à C₇, -(CH₂)ₘO-alkyle en C₁ à C₇ substitué par un atome d'halogène, alkyle en C₁ à C₇ substitué par un atome d'halogène, -(CH₂)ₙ-phényle facultativement substitué par un atome d'halogène, -CH-₂)ₘNHC(O)-alkyle en C₁ à C₇ ou -(CH₂)ₘNH₂, ou
R⁴ et R⁵ peuvent former conjointement avec l'atome de N auquel ils sont fixés un noyau pipéridine ou azétidine, qui peut être substitué par un atome d'halogène ; ou représente
un groupe -C(O)O-alkyle en C₁ à C₇ substitué par un atome d'halogène ;
n vaut 1 ou 2 ;
m vaut 1, 2 ou 3 ;
ou un sel d'addition d'acide pharmaceutiquement acceptable, un mélange racémique ou l'énantiomère et/ou les isomères correspondants de celui-ci.

3. Composé de formule IA selon l'une quelconque des revendications 1 ou 2, lesquels composés sont les suivants
6-(4-Chloro-phényl)-2-[1-(4-hydroxy-3,5-diméthoxy-phényl)-méth-(Z)-ylidène]-imidazo[2,1-b]thiazol-3-one
6-(4-Chloro-phényl)-2-[1-(4-hydroxy-3-méthoxy-phényl)-méth-(Z)-ylidène]-imidazo[2,1-b]thiazol-3-one
6-(4-Chloro-phényl)-2-[1-(3-fluoro-4-hydroxy-5-méthoxy-phényl)-méth-(Z)-ylidène]-imidazo[2,1-b]thiazol-3-one
6-(4-Chloro-phényl)-2-[1-(3-éthoxy-4-hydroxy-phényl)-méth-(Z)-ylidène]-imidazo[2,1-b]thiazol-3-one
6-(4-Chloro-phényl)-2-[1-(3-chloro-4-hydroxy-5-méthoxy-phényl)-méth-(Z)-ylidène]-imidazo[2,1-b]thiazol-3-one
6-(4-Chloro-phényl)-2-[1-(3-bromo-4-hydroxy-5-méthoxy-phényl)-méth-(Z)-ylidène]-imidazo[2,1-b]thiazol-3-one
6-(4-Chloro-phényl)-2-[1-(3,4-dihydroxy-5-méthoxy-phényl)-méth-(Z)-ylidène]-imidazo[2,1-b]thiazol-3-one
2-[1-(3-Fluoro-4-hydroxy-5-méthoxy-phényl)-méth-(Z)-ylidène]-6-o-tolyl-imidazo[2,1-b]thiazol-3-one
2-[1-(3-Chloro-4-hydroxy-5-méthoxy-phényl)-méth-(Z)-ylidène]-6-o-tolyl-imidazo[2,1-b]thiazol-3-one
2-[1-(4-hydroxy-3-méthoxy-phényl)-méth-(Z)-ylidène]-6-o-tolyl-imidazo[2,1-b]thiazol-3-one
2-[1-(3-Chloro-4-hydroxy-5-méthoxy-phényl)-méth-(Z)-ylidène]-6-(4-méthoxyphényl)-5-méthyl-imidazo[2,1-b]thiazol-3-one
2-[1-(3-Fluoro-4-hydroxy-5-méthoxy-phényl)-méth-(Z)-ylidène]-6-(4-méthoxyphényl)-5-méthyl-imidazo[2,1-b]thiazol-3-one
2-[1-(4-Hydroxy-3,5-diméthoxy-phényl)-méth-(Z)-ylidène]-6-(3-trifluorométhoxyphényl)-imidazo[2,1-b]thiazol-3-one
2-[1-(4-Hydroxy-3,5-diméthoxy-phényl)-méth-(Z)-ylidène]-6-(3-trifluorométhylphényl)-imidazo[2,1-b]thiazol-3-one
2-[1-(4-Hydroxy-3,5-diméthoxy-phényl)-méth-(Z)-ylidène]-6-m-tolyl-imidazo[2,1-b]thiazol-3-one
6-(3-Chloro-phényl)-2-[1-(4-hydroxy-3,5-diméthoxy-phényl)-méth-(Z)-ylidène]-imidazo[2,1-b]thiazol-3-one
2-[1-(3-Chloro-4-hydroxy-5-méthoxy-phényl)-méth-(Z)-ylidène]-6-(3-trifluorométhoxy-phényl)-imidazo[2,1-b]thiazol-3-one
4-{2-[1-(3-Chloro-4-hydroxy-5-méthoxy-phényl)-méth-(Z)-ylidène]-3-oxo-2,3-dihydro-imidazo[2,1-b] thiazol-6-yl}-N-[2-(2-fluoro-éthoxy)-éthyl]-N-méthylbenzamide
2-[1-(3-Chloro-4-hydroxy-5-méthoxy-phényl)-méth-(Z)-ylidène]-6-(3-trifluorométhyl-phényl)-imidazo[2,1-b]thiazol-3-one
6-(3-Chloro-phényl)-2-[1-(3-chloro-4-hydroxy-5-méthoxy-phényl)-méth-(Z)-ylidène]-imidazo[2,1-b]thiazol-3-one
6-(4-Chloro-phényl)-2-[1-(3-éthoxy-4-hydroxy-5-méthoxy-phényl)-méth-(Z)-ylidène]-imidazo[2,1-b]thiazol-3-one
6-(4-Chloro-phényl)-2-[1-(3-(2-fluoro-éthoxy)-4-hydroxy-5-méthoxy-phényl)-méth-(Z)-ylidène]-imidazo[2,1-b]thiazol-3-one
2-[1-(3-Chloro-4-hydroxy-5-méthoxy-phényl)-méth-(Z)-ylidène]-6-(3-fluoro-4-trifluorométhyl-phényl)-imidazo[2,1-b]thiazol-3-one
2-[1-(3-Hydroxy-3,5-diméthoxy-phényl)-méth-(Z)-ylidène]-6-(3-fluoro-4-trifluorométhyl-phényl)-imidazo[2,1-b]thiazol-3-one
6-(4-Chloro-phényl)-2-[1-(4-hydroxy-3-isopropoxy-5-méthoxy-phényl)-méth-(Z)-ylidène]-imidazo[2,1-b]thiazol-3-one
6-(4-Chloro-phényl)-2-[1-(4-hydroxy-3-méthoxy-5-propoxy-phényl)-méth-(Z)-ylidène]-imidazo[2,1-b]thiazol-3-one
6-(4-Chloro-phényl)-2-[1-(4-hydroxy-3-méthoxy-5-méthyl-phényl)-méth-(Z)-ylidène]-imidazo[2,1-b]thiazol-3-one
2-[1-(4-Hydroxy-3-méthoxy-5-propoxy-phényl)-méth-(Z)-ylidène]-6-(3-trifluorométhoxy-phényl)-imidazo[2,1-b]thiazol-3-one
2-[1-(4-Hydroxy-3-méthoxy-5-isopropoxy-phényl)-méth-(Z)-ylidène]-6-(3-trifluorométhoxy-phényl)-imidazo[2,1-b]thiazol-3-one
2-[1-(3-Fluoro-4-hydroxy-5-méthoxy-phényl)-méth-(Z)-ylidène]-6-(3-trifluorométhoxy-phényl)-imidazo[2,1-b]thiazol-3-one
2-[1-(4-Hydroxy-3-méthoxy-phényl)-méth-(Z)-ylidène]-6-(3-trifluorométhoxyphényl)-imidazo[2,1-b]thiazol-3-one
2-[1-(3-Bromo-4-hydroxy-5-méthoxy-phényl)-méth-(Z)-ylidène]-6-(3-trifluorométhoxy-phényl)-imidazo[2,1-b]thiazol-3-one
3-{2-[1-(3-Fluoro-4-hydroxy-5-méthoxy-phényl)-méth-(Z)-ylidène]-3-oxo-2,3-dihydro-imidazo[2,1-b]thiazol-6-yl}-benzonitrile
2-[1-(3-fluoro-4-hydroxy-5-méthoxy-phényl)-méth-(Z)-ylidène]-6-(3-chlorophényl)-imidazo[2,1-b]thiazol-3-one
2-[1-(3-Éthyl-4-hydroxy-5-méthoxy-phényl)-méth-(Z)-ylidène]-6-(3-trifluorométhoxy-phényl)-imidazo[2,1-b]thiazol-3-one
2-[1-(3-fluoro-4-hydroxy-5-méthoxy-phényl)-méth-(Z)-ylidène]-6-(3-(trifluorométhyl)-phényl)-imidazo[2,1-b]thiazol-3-one
2-[1-(3-fluoro-4-hydroxy-5-méthoxy-phényl)-méth-(Z)-ylidène]-6-(4-fluoro-3-(trifluorométhyl)-phényl)-imidazo[2,1-b]thiazol-3-one
2-[1-(3-fluoro-4-hydroxy-5-méthoxy-phényl)-méth-(Z)-ylidène]-6-phénylimidazo[2,1-b]thiazol-3-one
3-{2-[1-(3-Chloro-4-hydroxy-5-méthoxy-phényl)-méth-(Z)-ylidène]-3-oxo-2,3-dihydro-imidazo[2,1-b]thiazol-6-yl}-benzonitrile
3-{2-[1-(3-Bromo-4-hydroxy-5-méthoxy-phényl)-méth-(Z)-ylidène]-3-oxo-2,3-dihydro-imidazo[2,1-b]thiazol-6-yl}-benzonitrile
2-[1-(3-bromo-4-hydroxy-5-méthoxy-phényl)-méth-(Z)-ylidène]-6-(4-fluoro-3-(trifluorométhyl)-phényl)-imidazo[2,1-b]thiazol-3-one
2-[1-(3-Fluoro-4-hydroxy-5-méthoxy-phényl)-méth-(Z)-ylidène]-6-(3-méthoxyphényl)-imidazo[2,1-b]thiazol-3-one
2-[1-(3-Chloro-4-hydroxy-5-méthoxy-phényl)-méth-(Z)-ylidène]-6-(3,5-difluorophényl)-imidazo[2,1-b]thiazol-3-one
3-{2-[1-(4-Hydroxy-3,5-diméthoxy-phényl)-méth-(Z)-ylidène]-3-oxo-2,3-dihydroimidazo[2,1-b]thiazol-6-yl}-benzonitrile
3-{2-[1-(4-Hydroxy-3-méthoxy-5-isopropoxyphényl)-méth-(Z)-ylidène]-3-oxo-2,3-dihydroimidazo[2,1-b]thiazol-6-yl}-benzonitrile
2-[1-(3-Chloro-4-hydroxy-5-méthoxy-phényl)-méth-(Z)-ylidène]-6-m-tolyl-imidazo[2,1-b]thiazol-3-one
2-[1-(3-Chloro-4-hydroxy-5-méthoxy-phényl)-méth-(Z)-ylidène]-6-(2-fluorophényl)-imidazo [2,1-b] thiazol-3-one
2-[1-(3-Chloro-4-hydroxy-5-méthoxy-phényl)-méth-(Z)-ylidène]-6-(3-éthyl-phényl)-imidazo[2,1-b]thiazol-3-one
2-[1-(3-Chloro-4-hydroxy-5-méthoxy-phényl)-méth-(Z)-ylidène]-6-(2-méthoxyphényl)-imidazo[2,1-b]thiazol-3-one
2-[1-(3-Chloro-4-hydroxy-5-méthoxy-phényl)-méth-(Z)-ylidène]-6-(2,5-difluoro-phényl)-imidazo[2,1-b]thiazol-3-one
2-[1-(3-Chloro-4-hydroxy-5-méthoxy-phényl)-méth-(Z)-ylidène]-6-(3-fluorophényl)-imidazo[2,1-b]thiazol-3-one
2-[1-(3-Fluoro-4-hydroxy-5-méthoxy-phényl)-méth-(Z)-ylidène]-6-(3-fluorophényl)-imidazo[2,1-b]thiazol-3-one
2-[1-(4-Hydroxy-3,5-diméthoxy-phényl)-méth-(Z)-ylidène]-6-(3-fluoro-phényl)-imidazo[2,1-b]thiazol-3-one
2-[1-(3-Chloro-4-hydroxy-5-méthoxy-phényl)-méth-(Z)-ylidène]-6-phénylimidazo[2,1-b]thiazol-3-one
2-[1-(3-Chloro-4-hydroxy-5-méthoxy-phényl)-méth-(Z)-ylidène]-6-(3-méthoxyphényl)-imidazo[2,1-b]thiazol-3-one
2-[1-(3-Chloro-4-hydroxy-5-méthoxy-phényl)-méth-(Z)-ylidène]-6-(3-(difluorométhoxy)-phényl)-imidazo[2,1-b]thiazol-3-one
(Z)-3-(2-(3-fluoro-4-hydroxy-5-méthoxybenzylidène)-3-oxo-2,3-dihydroimidazo[2,1-b]thiazol-5-yl)benzonitrile
2-[1-(3-Chloro-4-hydroxy-5-méthoxy-phényl)-méth-(Z)-ylidène]-6-(3-(chlorométhyl)phényl)-imidazo[2,1-b]thiazol-3-one
3-{2-[1-(3-Fluoro-4-hydroxy-5-méthoxy-phényl)-méth-(Z)-ylidène]-3-oxo-2,3-dihydroimidazo[2,1-b]thiazol-6-yl}-benzonitrile
2-[1-(3-Chloro-4-hydroxy-5-méthoxy-phényl)-méth-(Z)-ylidène]-6-(3-(2-fluoroéthoxy)phényl)-imidazo[2,1-b]thiazol-3-one
2-[1-(3-Chloro-4-hydroxy-5-méthoxy-phényl)-méth-(Z)-ylidène]-6-(4-(2-fluoroéthoxy)phényl)-imidazo[2,1-b]thiazol-3-one
2-[1-(3-Chloro-4-hydroxy-5-méthoxy-phényl)-méth-(Z)-ylidène]-6-(2-(2-fluoroéthoxy)phényl)-imidazo[2,1-b]thiazol-3-one
3-{2-[1-(3-Chloro-4-hydroxy-5-méthoxy-phényl)-méth-(Z)-ylidène]-3-oxo-2,3-dihydroimidazo[2,1-b]thiazol-6-yl}-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-benzonitrile
2-[1-(3-Chloro-4-hydroxy-5-méthoxy-phényl)-méth-(Z)-ylidène]-6-(4-(difluorométhoxy)phényl)-imidazo[2,1-b]thiazol-3-one
2-[1-(3-Chloro-4-hydroxy-5-méthoxy-phényl)-méth-(Z)-ylidène]-6-(3-morpholinophényl)-imidazo[2,1-b]thiazol-3-one
2-[1-(3-Chloro-4-hydroxy-5-méthoxy-phényl)-méth-(Z)-ylidène]-6-(4-(pyrrolidin-1-yl)phényl)-imidazo[2,1-b]thiazol-3-one
2-[1-(3-Chloro-4-hydroxy-5-méthoxy-phényl)-méth-(Z)-ylidène]-6-(4-(diéthylamino)phényl)-imidazo[2,1-b]thiazol-3-one
2-[1-(3-Chloro-4-hydroxy-5-méthoxy-phényl)-méth-(Z)-ylidène]-6-(4-morpholinophényl)-imidazo[2,1-b]thiazol-3-one
2-[1-(3-Chloro-4-hydroxy-5-méthoxy-phényl)-méth-(Z)-ylidène]-6-(4-(diméthylamino)phényl)-imidazo[2,1-b]thiazol-3-one
2-[1-(3-Chloro-4-hydroxy-5-méthoxy-phényl)-méth-(Z)-ylidène]-6-[3-(2-oxo-pyrrolidin-1-yl)-phényl]-imidazo[2,1-b]thiazol-3-one
Acide 4-{2-[1-(3-chloro-4-hydroxy-5-méthoxy-phényl)-méth-(Z)-ylidène]-3-oxo-2,3-dihydroimidazo[2,1-b] thiazol-6-yl}-benzoïque
2-[1-(3-Chloro-4-hydroxy-5-méthoxy-phényl)-méth-(Z)-ylidène]-6-(3,4-diméthoxyphényl)-imidazo[2,1-b]thiazol-3-one
4-{2-[1-(3-Chloro-4-hydroxy-5-méthoxy-phényl)-méth-(Z)-ylidène]-3-oxo-2,3-dihydroimidazo[2,1-b]thiazol-6-yl}-benzamide
N-(4-{2-[1-(3-Chloro-4-hydroxy-5-méthoxy-phényl)-méth-(Z)-ylidène]-3-oxo-2,3-dihydro-imidazo[2,1-b]thiazol-6-yl}-phényl)-acétamide
Acide 3-{2-[1-(3-chloro-4-hydroxy-5-méthoxy-phényl)-méth-(Z)-ylidène]-3-oxo-2,3-dihydroimidazo[2,1-b] thiazol-6-yl}-benzoïque
2-[1-(3-Chloro-4-hydroxy-5-méthoxy-phényl)-méth-(Z)-ylidène]-6-(2,6-difluorophényl)-imidazo[2,1-b]thiazol-3-one
3-{2-[1-(3-Chloro-4-hydroxy-5-méthoxy-phényl)-méth-(Z)-ylidène]-3-oxo-2,3-dihydroimidazo [2,1-b] thiazol-6-yl}-N-(2-fluoro-éthyl)-benzamide
3-{2-[1-(3-Chloro-4-hydroxy-5-méthoxy-phényl)-méth-(Z)-ylidène]-3-oxo-2,3-dihydroimidazo[2,l-b]thiazol-6-yl}-N-propyl-benzamide
3-{2-[1-(3-Chloro-4-hydroxy-5-méthoxy-phényl)-méth-(Z)-ylidène]-3-oxo-2,3-dihydroimidazo[2,1-b] thiazol-6-yl}-N-méthyl-benzamide
N-Allyl-3-{2-[1-(3-chloro-4-hydroxy-5-méthoxy-phényl)-méth-(Z)-ylidène]-3-oxo-2,3-dihydroimidazo[2,1-b]thiazol-6-yl}-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-N-méthyl-benzamide
4-{2-[1-(3-Chloro-4-hydroxy-5-méthoxy-phényl)-méth-(Z)-ylidène]-3-oxo-2,3-dihydro-imidazo[2,1-b]thiazol-6-yl}-N-propyl-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-N-(2-fluoro-éthyl)-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-N-(3-fluoro-propyl)-benzamide
3-{2-[1-(3-Chloro-4-hydroxy-5-méthoxy-phényl)-méth-(Z)-ylidène]-3-oxo-2,3-dihydroimidazo[2,1-b]thiazol-6-yl}-N-[2-(3-fluoro-phényl)-éthyl]-N-méthylbenzamide
3-{2-[1-(3-Chloro-4-hydroxy-5-méthoxy-phényl)-méth-(Z)-ylidène]-3-oxo-2,3-dihydroimidazo[2,1-b]thiazol-6-yl}-N-(2-fluoro-éthyl)-N-méthyl-benzamide
3-{2-[1-(3-Fluoro-4-hydroxy-5-méthoxy-phényl)-méth-(Z)-ylidène]-3-oxo-2,3-dihydroimidazo[2,1-b]thiazol-6-yl}-benzamide
4-{2-[1-(3-Chloro-4-hydroxy-5-méthoxy-phényl)-méth-(Z)-ylidène]-3-oxo-2,3-dihydroimidazo[2,1-b] thiazol-6-yl}-N-(2-fluoro-éthyl)-N-méthyl-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-N,N-diméthyl-benzamide
2-[1-(3-Chloro-4-hydroxy-5-méthoxy-phényl)-méth-(Z)-ylidène]-6-[4-(4-fluoropipéridine-1-carbonyl)-phényl]-imidazo[2,1-b]thiazol-3-one
4-{2-[1-(3-Chloro-4-hydroxy-5-méthoxy-phényl)-méth-(Z)-ylidène]-3-oxo-2,3-dihydroimidazo[2,1-b]thiazol-6-yl}-N-méthyl-N-propyl-benzamide
4-{2-[1-(3-Chloro-4-hydroxy-5-méthoxy-phényl)-méth-(Z)-ylidène]-3-oxo-2,3-dihydroimidazo[2,1-b]thiazol-6-yl}-N-(3-fluoropropoxy)-benzamide
2-[1-(3-Chloro-4-hydroxy-5-méthoxy-phényl)-méth-(Z)-ylidène]-6-[4-(3-fluoroazétidine-1-carbonyl)-phényl]-imidazo[2,1-b]thiazol-3-one
4-{2-[1-(3-Chloro-4-hydroxy-5-méthoxy-phényl)-méth-(Z)-ylidène]-3-oxo-2,3-dihydroimidazo[2,1-b] thiazol-6-yl}-N-(3-fluoropropoxy)-N-méthyl-benzamide
Acide 4-{2-[1-(3-fluoro-4-hydroxy-5-méthoxy-phényl)-méth-(Z)-ylidène]-3-oxo-2,3-dihydroimidazo[2,1-b]thiazol-6-yl}-benzoïque
6-[4-(3-Fluoro-azétidine-1-carbonyl)-phényl]-2-[1-(3-fluoro-4-hydroxy-5-méthoxyphényl)-méth-(Z)-ylidène]-imidazo[2,1-b]thiazol-3-one
4-{2-[1-(3-Fluoro-4-hydroxy-5-méthoxy-phényl)-méth-(Z)-ylidène]-3-oxo-2,3-dihydro-imidazo[2,1-b]thiazol-6-yl}-N-(2-fluoroéthoxy)-benzamide
3-{2-[1-(3-Fluoro-4-hydroxy-5-méthoxy-phényl)-méth-(Z)-ylidène]-3-oxo-2,3-dihydroimidazo[2,1-b]thiazol-6-yl}-N-(3-fluoropropoxy)-N-méthyl-benzamide
2-[1-(3-Chloro-4-hydroxy-5-méthoxy-phényl)-méth-(Z)-ylidène]-6-[3-(3-fluoroazétidine-1-carbonyl)-phényl]-imidazo[2,1-b]thiazol-3-one
2-[1-(3-Chloro-4-hydroxy-5-méthoxy-phényl)-méth-(Z)-ylidène]-6-[3-(4-fluoropipéridine-1-carbon-phényl]-imidazo[2,1-b]thiazol-3-one
3-{2-[1-(3-Chloro-4-hydroxy-5-méthoxy-phényl)-méth-(Z)-ylidène]-3-oxo-2,3-dihydroimidazo[2,1-b]thiazol-6-yl}-N-[2-(2-fluoro-éthoxy)-éthyl]-N-méthyl-benzamide
4-{2-[1-(3-Fluoro-4-hydroxy-5-méthoxy-phényl)-méth-(Z)-ylidène]-3-oxo-2,3-dihydroimidazo[2,1-b]thiazol-6-yl}-N-(2-fluoroéthoxy)-N-méthyl-benzamide
4-{2-[1-(3-Fluoro-4-hydroxy-5-méthoxy-phényl)-méth-(Z)-ylidène]-3-oxo-2,3-dihydroimidazo[2,1-b] thiazol-6-yl}-N-(2-fluoropropoxy)-N-méthyl-benzamide Ester 2-fluoro-éthylique d'acide 4-{2-[1-(3-chloro-4-hydroxy-5-méthoxy-phényl)-méth-(Z)-ylidène]-3-oxo-2,3-dihydro-imidazo[2,1-b]thiazol-6-yl}-benzoïque Ester 3-fluoro-propylique d'acide 4-{2-[1-(3-chloro-4-hydroxy-5-méthoxy-phényl)-méth-(Z)-ylidène]-3-oxo-2,3-dihydro-imidazo[2,1-b]thiazol-6-yl}-benzoïque Ester tert-butylique d'acide [2-(4-{2-[1-(3-chloro-4-hydroxy-5-méthoxy-phényl)-méth-(Z)-ylidène]-3-oxo-2,3-dihydro-imidazo[2,1-b]thiazol-6-yl}-benzoylamino)-éthyl]-carbamique
N-(2-Amino-éthyl)-4-{2-[1-(3-chloro-4-hydroxy-5-méthoxy-phényl)-méth-(Z)-ylidène]-3-oxo-2,3-dihydro-imidazo[2,1-b]thiazol-6-yl}-benzamide
Fluorure de 3-{2-[1-(3-Chloro-4-hydroxy-5-méthoxy-phényl)-méth-(Z)-ylidène]-3-oxo-2,3-dihydro-imidazo[2,1-b] thiazol-6-yl}-benzènesulfonyle
3-{2-[1-[3-Chloro-5-(3-fluoro-propoxy)-4-hydroxy-phényl]-méth-(Z)-ylidène]-3-oxo-2,3-dihydro-imidazo[2,1-b]thiazol-6-yl}-benzonitrile
3-Bromo-5-{2-[1-(3-chloro-4-hydroxy-5-méthoxy-phényl)-méth-(Z)-ylidène]-3-oxo-2,3-dihydro-imidazo[2,1-b]thiazol-6-yl}-benzonitrile
2-[1-(3-Chloro-4-hydroxy-5-méthoxy-phényl)-méth-(Z)-ylidène]-6-(3-{2-[2-(2-fluoro-éthoxy)-éthoxy]-éthoxy}-phényl)-imidazo[2,1-b]thiazol-3-one ou
2-[1-(3-Chloro-4-hydroxy-5-méthoxy-phényl)-méth-(Z)-ylidène]-6-(2-{2-[2-(2-fluoro-éthoxy)-éthoxy]-éthoxy}-phényl)-imidazo[2,1-b]thiazol-3-one.

4. Composé de formule IB selon la revendication 1 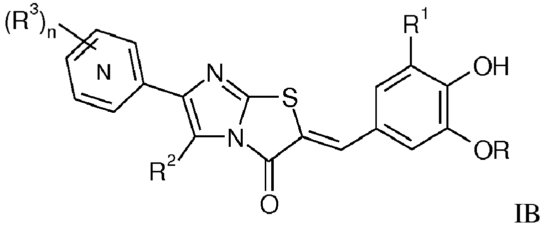 dans laquelle l'atome de N du groupe pyridinyle peut être en différentes positions, et dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
R³ représente un atome d'hydrogène, un atome d'halogène, ou un groupe alkyle en C₁ à C₇, alkyle en C₁ à C₇ substitué par un atome d'halogène, alcoxy en C₁ à C₇, alcoxy en C₁ à C₇ substitué par un atome d'halogène, O(CH₂)ₘO(CH₂)ₘO-alkyle en C₁ à C₇ substitué par un atome d'halogène, cyano, alcoxy en C₁ à C₇ substitué par un groupe hydroxy, alcényloxy en C₁ à C₇, C(O)OH, hétérocycloalkyle choisi parmi un groupe morpholinyle, pyrrolidinyle ou pyrrolidin-2-one, ou représente un groupe hétéroaryle choisi parmi un groupe imidazolyle substitué par un groupe alkyle en C₁ à C₇, ou représente
un groupe NR'R" et R'/R" représentent indépendamment l'un de l'autre un atome d'hydrogène ou un groupe alkyle en C₁ à C₇ ou -C(O)- alkyle en C₁ à C₇ ; ou représente
un groupe -C(O)NR⁴R⁵ et
R⁴ représente un atome d'hydrogène ou un groupe alkyle en C₁ à C₇ et
R⁵ représente un atome d'hydrogène, ou un groupe alkyle en C₁ à C₇, alcényle en C₁ à C₇, -(CH₂)ₘO-alkyle en C₁ à C₇ substitué par un atome d'halogène, alkyle en C₁ à C₇ substitué par un atome d'halogène, -(CH₂)ₙ-phényle facultativement substitué par un atome d'halogène, -CH₂)ₘNHC(O)-alkyle en C₁ à C₇ ou -(CH₂)ₘNH₂, ou
R⁴ et R⁵ peuvent former conjointement avec l'atome de N auquel ils sont fixés un noyau pipéridine ou azétidine, qui peut être substitué par un atome d'halogène ; ou représente
un groupe -C(O)O-alkyle en C₁ à C₇ substitué par un atome d'halogène ;
n vaut 1 ou 2 ;
m vaut 1, 2 ou 3 ;
ou un sel d'addition d'acide pharmaceutiquement acceptable, un mélange racémique ou l'énantiomère et/ou les isomères correspondants de celui-ci.

5. Composé de formule IB selon l'une quelconque des revendications 1 ou 4, lesquels composés sont les suivants
2-[1-(4-Hydroxy-3,5-diméthoxy-phényl)-méth-(Z)-ylidène]-6-pyridin-4-yl-imidazo[2,1-b]thiazol-3-one
2-[1-(4-Hydroxy-3-méthoxy-phényl)-méth-(Z)-ylidène]-6-(2-méthyl-pyridin-4-yl)-imidazo[2,1-b]thiazol-3-one
2-[1-(4-Hydroxy-3-méthoxy-phényl)-méth-(Z)-ylidène]-6-(2,6-diméthyl-pyridin-4-yl)-imidazo[2,1-b]thiazol-3-one
2-[1-(4-Hydroxy-3,5-diméthoxy-phényl)-méth-(Z)-ylidène]-6-(2-méthyl-pyridin-4-yl)-imidazo[2,1-b]thiazol-3-one
2-[1-(4-Hydroxy-3,5-diméthoxy-phényl)-méth-(Z)-ylidène]-6-(2,6-diméthyl-pyridin-4-yl)-imidazo[2,1-b]thiazol-3-one
2-[1-(3-Fluoro-4-hydroxy-5-méthoxy-phényl)-méth-(Z)-ylidène]-6-(6-méthoxypyridin-2-yl)-imidazo[2,1-b]thiazol-3-one
2-[1-(3-Chloro-4-hydroxy-5-méthoxy-phényl)-méth-(Z)-ylidène]-6-(2,6-diméthylpyridin-4-yl)-imidazo[2,1-b]thiazol-3-one
2-[1-(3-Fluoro-4-hydroxy-5-méthoxy-phényl)-méth-(Z)-ylidène]-6-(2,6-diméthylpyridin-4-yl)-imidazo[2,1-b]thiazol-3-one
2-[1-(3-Bromo-4-hydroxy-5-méthoxy-phényl)-méth-(Z)-ylidène]-6-(2,6-diméthylpyridin-4-yl)-imidazo[2,1-b]thiazol-3-one
2-[1-(3-Chloro-4-hydroxy-5-méthoxy-phényl)-méth-(Z)-ylidène]-6-(pyridin-4-yl)-imidazo[2,1-b]thiazol-3-one
2-[1-(3-Fluoro-4-hydroxy-5-méthoxy-phényl)-méth-(Z)-ylidène]-6-(pyridin-4-yl)-imidazo[2,1-b]thiazol-3-one
2-[1-(3-Bromo-4-hydroxy-5-méthoxy-phényl)-méth-(Z)-ylidène]-6-(pyridin-4-yl)-imidazo[2,1-b]thiazol-3-one
2-[1-(3-Fluoro-4-hydroxy-5-méthoxy-phényl)-méth-(Z)-ylidène]-6-(pyridin-3-yl)-imidazo[2,1-b]thiazol-3-one
2-[1-(3-Bromo-4-hydroxy-5-méthoxy-phényl)-méth-(Z)-ylidène]-6-(pyridin-3-yl)-imidazo[2,1-b]thiazol-3-one
2-[1-(3-Chloro-4-hydroxy-5-méthoxy-phényl)-méth-(Z)-ylidène]-6-(2-méthylpyridin-4-yl)-imidazo[2,1-b]thiazol-3-one
2-[1-(3-Fluoro-4-hydroxy-5-méthoxy-phényl)-méth-(Z)-ylidène]-6-(2-méthylpyridin-4-yl)-imidazo[2,1-b]thiazol-3-one
2-[1-(3-Bromo-4-hydroxy-5-méthoxy-phényl)-méth-(Z)-ylidène]-6-(2-méthylpyridin-4-yl)-imidazo[2,1-b]thiazol-3-one
2-[1-(3-Chloro-4-hydroxy-5-méthoxy-phényl)-méth-(Z)-ylidène]-6-(pyridin-3-yl)-imidazo[2,1-b]thiazol-3-one
2-[1-(3-Chloro-4-hydroxy-5-méthoxy-phényl)-méth-(Z)-ylidène]-6-[6-(4-méthyl-3H-imidazol-l-yl)-pyridin-3-yl]-imidazo[2,l-b]thiazol-3-one
2-[1-(3-Chloro-4-hydroxy-5-propoxy-phényl)-méth-(Z)-ylidène]-6-(4-méthylpyridin-3-yl)-imidazo[2,1-b]thiazol-3-one
2-[1-(3-Chloro-4-hydroxy-5-propoxy-phényl)-méth-(Z)-ylidène]-6-pyridin-3-yl-imidazo[2,1-b]thiazol-3-one
2-[1-(3-Chloro-4-hydroxy-5-propoxy-phényl)-méth-(Z)-ylidène]-6-pyridin-4-yl-imidazo[2,1-b]thiazol-3-one
2-[1-(3-Chloro-4-hydroxy-5-propoxy-phényl)-méth-(Z)-ylidène]-6-[6-(4-méthylimidazol-1-yl)-pyridin-3-yl]-imidazo[2,1-b]thiazol-3-one
2-[1-(3-Fluoro-4-hydroxy-5-propoxy-phényl)-méth-(Z)-ylidène]-6-pyridin-4-yl-imidazo[2,1-b]thiazol-3-one
2-[1-(3-Chloro-5-(3-fluoropropoxy)-4-hydroxy-phényl)-méth-(Z)-ylidène]-6-pyridin-4-ylimidazo[2,1-b]thiazol-3-one
2-[1-(3-Fluoro-5-(3-fluoropropoxy)-4-hydroxy-phényl)-méth-(Z)-ylidène]-6-pyridin-4-yl-imidazo[2,1-b]thiazol-3-one
2-[1-(3-Chloro-4-hydroxy-5-propoxy-phényl)-méth-(Z)-ylidène]-6-pyridin-2-yl-imidazo[2,1-b]thiazol-3-one
2-[1-(3-Chloro-4-hydroxy-5-méthoxy-phényl)-méth-(Z)-ylidène]-6-(2-chloropyridin-4-yl)-imidazo[2,1-b]thiazol-3-one ou
2-[1-[3-Chloro-5-(3-fluoro-propoxy)-4-hydroxy-phényl]-méth-(Z)-ylidène]-6-(2,6-diméthylpyridin-4-yl)-imidazo[2,1-b]thiazol-3-one.

6. Composé de formule IC selon la revendication 1, 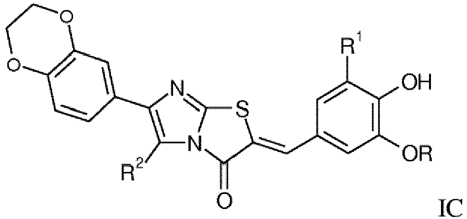 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

7. Composé selon l'une quelconque des revendications 1 ou 6, lequel composé est de la 2-[1-(3-chloro-4-hydroxy-5-méthoxy-phényl)-méth-(Z)-ylidène]-6-(2,3-dihydrobenzo[1,4]dioxin-6-yl)-imidazo[2,1-b]thiazol-3-one.

8. Composé de formule ID selon la revendication 1, 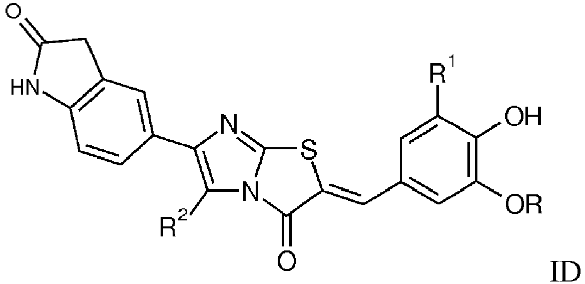 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

9. Composé de formule ID selon l'une quelconque des revendications 1 ou 8, lequel composé est de la 5-{2-[1-(3-chloro-4-hydroxy-5-méthoxy-phényl)-méth-(Z)-ylidène]-3-oxo-2,3-dihydroimidazo[2,1-b]thiazol-6-yl}-1,3-dihydro-indol-2-one.

10. Composé de formule IE selon la revendication 1, 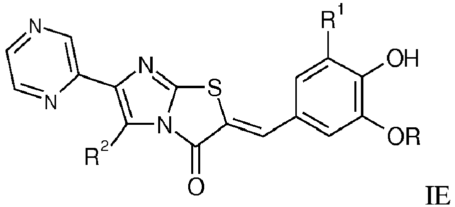 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

11. Composé de formule IE selon l'une quelconque des revendications 1 ou 10 2-[1-(3-Chloro-4-hydroxy-5-propoxy-phényl)-méth-(Z)-ylidène]-6-pyrazin-2-yl-imidazo[2,1-b]thiazol-3-one.

12. Composé de formule IF selon la revendication 1, 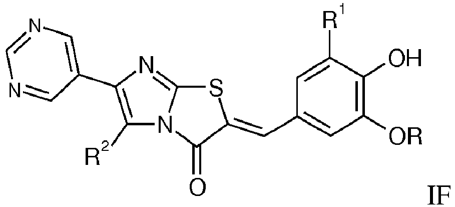 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

13. Composé de formule IF selon l'une quelconque des revendications 1 ou 12, lequel composé est de la 2-[1-(3-chloro-4-hydroxy-5-propoxy-phényl)-méth-(Z)-ylidène]-6-pyrimidin-5-yl-imidazo[2,1-b]thiazol-3-one.

14. Composé de formule IG selon la revendication 1, 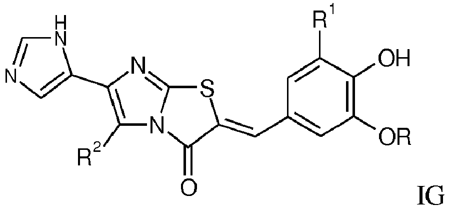 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

15. Composé de formule IG selon l'une quelconque des revendications 1 ou 14, lequel composé est de la 2-[1-(3-chloro-4-hydroxy-5-propoxy-phényl)-méth-(Z)-ylidène]-6-(3H-imidazol-4-yl)-imidazo[2,1-b]thiazol-3-one.

16. Composé de formule IH selon la revendication 1, 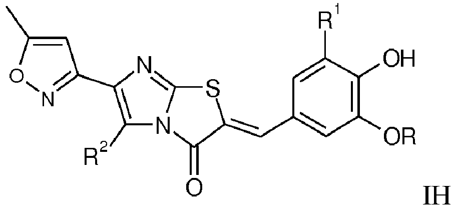 dans laquelle
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

17. Composé de formule IH selon l'une quelconque des revendications 1 ou 16, lequel composé est de la 2-[1-(3-chloro-4-hydroxy-5-méthoxy-phényl)-méth-(Z)-ylidène]-6-(5-méthyl-isoxazol-3-yl)-imidazo[2,1-b]thiazol-3-one.

18. Composé de formule IJ selon la revendication 1, 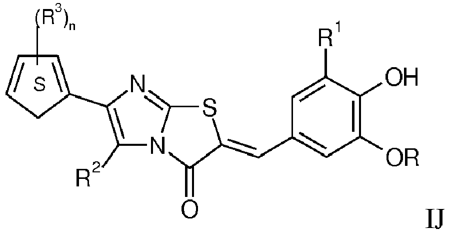 dans laquelle
R³ représente un atome d'hydrogène ou un groupe alkyle en C₁ à C₇ n vaut 1 ou 2
R représente un groupe alkyle en C₁ à C₇ ou alkyle en C₁ à C₇ substitué par un atome d'halogène ;
R¹ représente un atome d'hydrogène, un atome d'halogène, ou un groupe hydroxy, alcoxy en C₁ à C₇, alkyle en C₁ à C₇ ou alcoxy en C₁ à C₇ substitué par un atome d'halogène ;
R² représente un atome d'hydrogène ou un groupe alkyle en C₁ à C₇ ;
ou un sel d'addition d'acide pharmaceutiquement acceptable, un mélange racémique ou l'énantiomère et/ou les isomères correspondants de celui-ci.

19. Composé de formule IJ selon l'une quelconque des revendications 1 ou 18, lesquels composés sont les suivants
2-[1-(3-Chloro-4-hydroxy-5-propoxy-phényl)-méth-(Z)-ylidène]-6-thiophén-3-yl-imidazo[2,1-b]thiazol-3-one
2-[1-(3-Chloro-4-hydroxy-5-propoxy-phényl)-méth-(Z)-ylidène]-6-thiophén-2-yl-imidazo[2,1-b]thiazol-3-one ou
2-[1-(3-Chloro-4-hydroxy-5-propoxy-phényl)-méth-(Z)-ylidène]-6-(2,4-diméthylthiophén-3-yl)-imidazo[2,1-b]thiazol-3-one.

20. Composé de formule I selon l'une quelconque des revendications 1 à 19 destiné à l'utilisation dans la liaison et l'imagerie des agrégats tau, des agrégats bêta-amyloïdes ou des agrégats d'alpha-synucléine.

21. Composé de formule I selon l'une quelconque des revendications 1 à 19 destiné à l'utilisation dans la liaison et l'imagerie des agrégats tau chez les patients atteints de la maladie d'Alzheimer.

22. Composé de formule I selon l'une quelconque des revendications 1 à 19 pour utilisation dans une étude de liaison de tau.

23. Composé de formule I selon l'une quelconque des revendications 1 à 19 pour utilisation de l'imagerie diagnostique des agrégats tau dans le cerveau d'un mammifère.

24. Composition pharmaceutique comprenant un composé de formule I selon l'une quelconque des revendications 1 à 19 et un vecteur pharmaceutique acceptable.

25. Procédé d'imagerie de dépôts d'agrégats tau, comprenant
- l'introduction dans un mammifère d'une quantité détectable d'une composition selon la revendication 24 ;
- le passage d'une durée suffisante pour que le composé de formule I soit associé au dépôt d'agrégats tau, et
- la détection du composé associé à un ou plusieurs dépôts d'agrégats tau.

26. Utilisation d'un composé tel que défini dans l'une quelconque des revendications 1 à 19 pour l'imagerie diagnostique de dépôts d'agrégats tau dans le cerveau d'un mammifère.
